# EUROPEAN PATENT SPECIFICATION

(11) **EP 1 828 201 B1**
(45) Date of publication and mention of the grant of the patent: **11.11.2015**
(21) Application number: 05826362.5
(22) Date of filing: 08.12.2005
(51) Int. Cl.: C07D 498/08, A61K 31/529, A61P 35/00, C07D 273/00, C07D 239/00

(54) **MACROCYCLIC QUINAZOLINE DERIVATIVES AND THEIR USE AS MTKI**
MAKROZYKLISCHE CHINAZOLINDERIVATE UND IHRE VERWENDUNG ALS MTKI
DERIVES DE QUINAZOLINE MACROCYCLIQUE ET LEUR UTILISATION COMME MTKI

(30) Priority: 08.12.2004 US 634228 P; 08.12.2004 EP 04106383
(43) Date of publication of application: 05.09.2007
(73) Proprietor: Janssen Pharmaceutica NV, 2340 Beerse (BE)
(72) Inventor: FREYNE, Eddy Jean Edgard, Janssen Pharmaceutica NV, B-2340 Beerse (BE); WILLEMS, Marc, Janssen Pharmaceutica N.V., B-2340 Beerse (BE); TEN HOLTE, Peter, Janssen Pharmaceutica N.V., B-2340 Beerse (BE); PAPANIKOS, Alexandra, Janssen Pharmaceutica N.V., B-2340 Beerse (BE); EMBRECHTS, Werner C. J., Janssen Pharmaceutica NV, B-2340 Beerse (BE); STORCK, Pierre Henri, Janssen-Cilag, F-27106 Val De Reuil Cedex (FR); PONCELET, Virginie Sophie, Janssen-Cilag, F-27106 Val De Reuil Cedex (FR)
(74) Representative: Daelemans, Frank F.R.
(86) International application number: PCT/EP2005/056609
(87) International publication number: WO 2006/061417

(56) References cited:
- WO-A-96/13529
- WO-A-96/33980
- WO-A-2004/105765
- US-B1- 6 344 459
- "4-ANILINOQUINAZOLINE DERIVATIVES" EXPERT OPINION ON THERAPEUTIC PATENTS, ASHLEY PUBLICATIONS, GB, vol. 8, no. 4, 1998, pages 475-478, XP000999463 ISSN: 1354-3776

## Description

The human genome encompasses some 2,000 proteins that utilize adenosine 5'-triphosphate (ATP) in one way or another and some 500 of these encode for protein kinases, i.e the protein-tyrosine and protein-serine/threonine kinases, that share a catalytic domain conserved in sequence and structure but which are notably different in how their catalysis is regulated. Substrate phosphorylation by these enzymes is nature's predominant molecular way of organizing cellular signal transduction and regulating biochemical processes in general. It is not surprising, therefore, that abnormal phosphorylation of cellular proteins is a hallmark of disease and that there is a growing interest in the use of kinase inhibitors as drugs for therapeutic intervention in many disease states such as cancer, diabetes, inflammation and arthritis.

It is an object of the present invention to provide such kinase inhibitors, that are quinazoline derived macrocycles, hereinafter also referred to as multi targeting kinase inhibitors (MTKI), found to possess anti-proliferative activity, such as anti-cancer activity and which are accordingly useful in methods of treatment of the human or animal body, for example in the manufacture of medicaments for use in hyper proliferative disorders such as atherosclerosis, restenosis and cancer. The invention also relates to processes for the manufacture of said quinazoline derivatives, to pharmaceutical compositions containing them and to their use in the manufacture of medicaments of use in the production of anti-proliferative effect.

In particular, the compounds of the present invention were found to inhibit tyrosine kinase enzymes, also called tyrosine kinases. Tyrosine kinases are a class of enzymes, which catalyse the transfer of the terminal phosphate of adenosine triphosphate to the phenolic hydroxy- group of a tyrosine residue present in the target protein. It is known, that several oncogenes, involved in the transformation of a cell into a malignant tumour cell, encode tyrosine kinase enzymes including certain growth factor receptors such as EGF, FGF, IGF-1R, IR, PDGF and VEGF. This family of receptor tyrosine kinases and in particular the EGF family of receptor tyrosine kinases are frequently present in common human cancers such as breast cancer, non-small cell lung cancers including adenocarcinomas and squamous cell cancer of the lung, bladder cancer, oesophageal cancer, gastrointestinal cancer such as colon, rectal or stomach cancer, cancer of the prostate, leukaemia and ovarian, bronchial or pancreatic cancer, which are examples of cell proliferation disorders.

Accordingly, it has been recognised that the selective inhibition of tyrosine kinases will be of value in the treatment of cell proliferation related disorders. Support for this view is provided by the development of Herceptin® (Trastuzumab) and Gleevec™ (imatinib mesylate) the first examples of target based cancer drugs. Herceptin^{®} (Trastuzumab) is targeted against Her2/*neu*, a receptor tyrosine kinase found to be amplified up to 100-fold in about 30% of patients with invasive breast cancer. In clinical trials Herceptin^{®} (Trastuzumab) proved to have anti-tumour activity against breast cancer (Review by L.K. Shawer et al, "Smart Drugs: Tyrosine kinase inhibitors in cancer therapy", 2002, Cancer Cell Viol.1, 117), and accordingly provided the proof of principle for therapy targeted to receptor tyrosine kinases. The second example, Gleevec™ (imatinib mesylate), is targeted against the abelson tyrosine kinase (BcR-Abl), a constitutively active cytoplasmic tyrosine kinase present in virtually all patients with chronic myelogenous leukaemia (CML) and 15% to 30% of adult patients with acute lymphoblastic leukaemia. In clinical trials Gleevec™ (imatinib mesylate) showed a spectacular efficacy with minimal side effects that led to an approval within 3 months of submission. The speed of passage of this agent through clinical trials and regulatory review has become a case study in rapid drug development (Drucker B.J. & Lydon N., "Lessons learned from the development of an Abl tyrosine kinase inhibitor for chronic myelogenous leukaemia.", 2000, J.Clin.Invest. 105, 3).

Further support is given by the demonstration that EGF receptor tyrosine kinase inhibitors, specifically attenuates the growth in athymic nude mice of transplanted carcinomas such as human mammary carcinoma or human squamous cell carcinoma (Review by T.R. Burke Jr., Drugs of the Future, 1992, 17, 119). As a consequence, to treat different cancers there has been considerable interest in the development of drugs that target the EGFR receptor. For example, several antibodies that bind to the extracellular domain of EGFR are undergoing clinical trials, including Erbitux™ (also called C225, Cetuximab), which was developed by Imclone Systems and is in Phase III clinical trials for the treatment of several cancers. Also, several promising orally active drugs that are potent and relatively specific inhibitors of the EGFR tyrosine kinase are now well advanced in clinical trials. The AstraZeneca compound ZD1839, which is now called IRESSA^{®} and approved for the treatment of advanced non-small-cell lung cancer, and the OSI/Genentech/Roche compound OSI-774, which is now called Tarceva™ (erlotinib), have shown marked efficacy against several cancers in human clinical trials (Morin M.J., "From oncogene to drug: development of small molecule tyrosine kinase inhibitors as anti-tumour and anti-angiogenic agents, 2000, Oncogene 19, 6574).

In addition to the above, EGF receptor tyrosine kinases are shown to be implicated in non-malignant proliferative disorders such as psoriasis (Elder et al., Science, 1989, 243; 811). It is therefore expected that inhibitors of EGF type receptor tyrosine kinases will be useful in the treatment of non-malignant diseases of excessive cellular proliferation such as psoriasis, benign prostatic hypertrophy, atherosclerosis and restenosis.
It is disclosed in International Patent Application WO96/33980 and in J. Med. Chem, 2002, 45, 3865 that certain 4 anilino substituted quinazoline derivatives may be useful as inhibitors of tyrosine kinase and in particular of the EGF type receptor tyrosine kinases. Unexpectedly it was found that Quinazoline derivatives of the present formula (I) that are different in structure show to have tyrosine kinase inhibitory activity.

It is accordingly an object of the present invention to provide further tyrosine kinase inhibitors useful in the manufacture of medicaments in the treatment of cell proliferative related disorders.

This invention concerns compounds of formula (I) 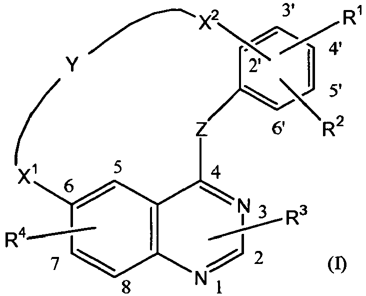 the *N*-oxide forms, the pharmaceutically acceptable addition salts and the stereochemically isomeric forms thereof, wherein
Z represents NH;
Y represents -C₃₋₉alkyl-, -C₂₋₉alkenyl-, -C₁₋₅alkyl-oxy-C₁₋₅alkyl-, -C₁₋₅alkyl-NR¹³-C₁₋₅alkyl-, -C₁₋₅alkyl-NR¹⁴-CO-C₁₋₅alkyl-, -C₁₋₆alkyl-NH-CO-, -NH-CO-C₁₋₆alkyl-, -CO-C₁₋₇alkyl-, -C₁₋₇alkyl-CO-, C₁₋₆alkyl-CO-C₁₋₆alkyl, -C₁₋₂alkyl-NR²³-CO-CR¹⁶R¹⁷-NH-, -C₁₋₂alkyl-CO-NH-CR¹⁸R¹⁹-CO-, -C₁₋₂alkyl-CO-NR²⁰-C₁₋₃alkyl-CO-, -C₁₋₂alkyl-NR²¹-CH₂-CO-NH-C₁₋₃alkyl-, -NR²²-CO-C₁₋₃alkyl-NH-, -C₁₋₃alkyl-NH-CO-Het²⁰-, C₁₋₂alkyl-CO-Het²¹-CO-, or -Het²²-CH₂-CO-NH-C₁₋₃alkyl-;
X¹ represents O, -O-C₁₋₂alkyl-, -O-N=CH-, NR¹¹ or -NR¹¹-C₁₋₂alkyl-; in a particular embodiment X¹ represents O, -O-C₁₋₂alkyl- or NR¹¹-C₁₋₂alkyl;
X² represents a direct bond, C₁₋₂alkyl, O, -O-C₁₋₂alkyl-, CO, -CO-C₁₋₂alkyl-, -O-N=CH-, NR¹² or NR¹²-C₁₋₂alkyl-; in a particular embodiment X² represents a direct bond, -O-, -O-C₁₋₂alkyl, -CO-C₁₋₂alkyl- or NR¹²-C₁₋₂alkyl-;
R¹ represents hydrogen, cyano, halo or hydroxy, preferably halo;
R² represents hydrogen, cyano, halo, hydroxy, hydroxycarbonyl-, C₁₋₄alkyloxycarbonyl-, Het¹⁶-carbonyl-, C₁₋₄alkyl-, C₂₋₆alkynyl-, Ar⁵, Het¹ or dihydroxyborane;
R³ represents hydrogen, cyano, halo, hydroxy, formyl, C₁₋₆alkoxy-, C₁₋₆alkyl-, C₁₋₆alkoxy- substituted with halo, or R³ represents C₁₋₄alkyl substituted with one or where possible two or more substituents selected from hydroxy or halo;
R⁴ represents Ar⁴-C₁₋₄alkyloxy-, C₁₋₄alkyloxy- or R⁴ represents C₁₋₄alkyloxy substituted with one or where possible two or more substituents selected from hydroxy-, halo, C₁₋₄alkyloxy-, C₁₋₄alkyloxy-C₁₋₄alkyloxy-, NR³⁷R³⁸-carbonyloxy-, Het⁵-carbonyloxy-, NR⁷R⁸, NR⁹R¹⁰-carbonyl-, Het³-carbonyl-, Het¹³-oxy- or Het²-;
R⁷ represents hydrogen, hydroxy-C₁₋₄alkyl- or C₁₋₄alkyl;
R⁸ represents C₃₋₆cycloalkyl; Het⁶-carbonyl-; Het⁷-aminocarbonyl-; Het⁸; Het⁹-oxycarbonyl-; Het¹⁰-sulfonyl-; C₁₋₄alkyloxycarbonyl;
mono- or di(C₁₋₄alkyl)aminocarbonyl-; mono- or di(C₁₋₄alkyl)aminocarbonyl substituted with C₁₋₄alkylsulfonyl-; or
C₁₋₄alkylcarbonyl optionally substituted with one or more substituents selected from C₁₋₄alkylsulfonyl, hydroxy- and C₁₋₄alkyloxy-; or
R⁸ represents C₁₋₄alkyl substituted with one or more substituents selected from C₁₋₄alkylsulfonyl- NR²⁵R²⁶, aminocarbonyloxy-, C₁₋₄alkylcarbonyloxyaminocarbonyl-, hydroxy-C₁₋₄alkyloxy-, C₁₋₄alkyloxy-C₁₋₄alkyloxy-, and Het¹¹;
R⁹ represents hydrogen or C₁₋₄alkyl-;
R¹⁰ represents Het⁴ or C₁₋₄alkyl- substituted with C₁₋₄alkylsulfonyl-;
R¹¹ represents hydrogen, C₁₋₄alkyl- or C₁₋₄alkyl-oxy-carbonyl-;
R¹² represents hydrogen, C₁₋₄alkyl-, C₁₋₆alkyloxycarbonyl- or C₁₋₆alkyloxycarbonylsubstituted with phenyl;
R¹³ represents hydrogen, Het¹⁴-C₁₋₄alkyl, C₁₋₆alkyloxycarbonyl optionally substituted with phenyl or R¹³ represents Ar⁶-sulfonyl or Het²⁴-C₁₋₄alkylcarbonyl; in particular morpholinyl-C₁₋₄alkyl;
R¹⁴ and R¹⁵ are each independently selected from hydrogen, C₁₋₄alkyl, Het¹⁵-C₁₋₄alkyl- or C₁₋₄alkyloxyC₁₋₄alkyl-;
R¹⁶ and R¹⁷ each independently represents hydrogen, C₁₋₄alkyl or C₁₋₄alkyl substituted with hydroxy-, C₃₋₆cycloalkyl or phenyl; or R¹⁶ and R¹⁷ taken together with the carbon atom to which they are attached form a C₃₋₆cycloalkyl;
R¹⁸ represents hydrogen or C₁₋₄alkyl optionally substituted with hydroxy or phenyl;
R¹⁹ represents hydrogen or C₁₋₄alkyl, in particular hydrogen or methyl, even more particular hydrogen;
R²⁰ represents hydrogen or C₁₋₄alkyl, in particular hydrogen or methyl;
R²¹ represents hydrogen, C₁₋₄alkyl, Het²³-C₁₋₄alkylcarbonyl- or R²¹ represents mono-or di(C₁₋₄alkyl)amino-C₁₋₄alkyl-carbonyl- optionally substituted with hydroxy, pyrimidinyl, dimethylamine or C₁₋₄alkyloxy;
R²² represents hydrogen or C₁₋₄alkyl optionally substituted with hydroxy or C₁₋₄alkyloxy;
R²³ represents C₁₋₄alkyl optionally substituted with hydroxy-, C₁₋₄alkyloxy- or Het²⁵; R²³ may also represent hydrogen when R¹⁶ and R¹⁷ taken together with the carbon atom to which they are attached form a C₃₋₆cycloalkyl;
R²⁵ and R²⁶ each independently represent hydrogen, C₁₋₄alkyl, C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-, in particular R²⁵ and R²⁶ each independently represent hydrogen, C₁₋₄alkyl, C₁₋₄alkylsulfonyl-, aminocarbonyl-, mono- or di(C₁₋₄alkyl)aminocarbonyl- or C₁₋₄alkylcarbonyl-;
R²⁷ and R²⁸ each independently represent hydrogen, C₁₋₄alkyl, C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-; or for those compounds of formula (I) wherein Het² represents a heterocycle selected from morpholinyl, piperazinyl, piperidinyl, pyrrolidinyl or thiomorpholinyl substituted with NR²⁷R²⁸-C₁₋₄alkyl said R²⁷ and R²⁸ each independently represent C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R²⁹ and R³⁰ each independently represent hydrogen, aminosulfonyl, aminocarbonyl, mono- or di(C₁₋₄alkyl)aminocarbonyl-, mono- or di(C₁₋₄alkyl)aminosulfonyl-, or C₁₋₄alkyl- optionally substituted with one or more substituents selected from NR³¹R³², C₁₋₄alkylsulfonyl, aminocarbonyloxy-, hydroxy-, C₁₋₄alkyloxy , aminocarbonyl- and mono- or di(C₁₋₄alkyl)aminocarbonyl-, or C₁₋₄alkyloxycarbonyl optionally substituted with one or more substituents selected from hydroxy, C₁₋₄alkyloxy- and C₁₋₄alkylsulfonyl-, or C₁₋₄alkylcarbonyl optionally substituted with one or more substituents selected from hydroxy-, C₁₋₄alkyloxy- and C₁₋₄alkylsulfonyl-;
R³¹ and R³² each independently represent hydrogen, C₁₋₄alkyl, C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R³³ represents hydrogen or C₁₋₄alkyl;
R³⁴ represents C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R³⁵ represents hydrogen or C₁₋₄alkyl;
R³⁶ represents C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R³⁷ and R³⁸ each independently represent hydrogen, C₁₋₄alkyl, C₁₋₄alkylsulfonyl-, Het¹² or C₁₋₄alkyl substituted with one or more substituents selected from C₁₋₄alkylsulfonyl-, hydroxy- and C₁₋₄alkyloxy-;
R³⁹ and R⁴⁰ each independently represent aminosulfonyl, aminocarbonyl,
mono- or di(C₁₋₄alkyl)aminocarbonyl-, mono- or di(C₁₋₄alkyl)aminosulfonyl-, or
C₁₋₄alkyl- substituted with one or more substituents selected from NR³¹R³²,
C₁₋₄alkylsulfonyl, aminocarbonyloxy-, hydroxy-, C₁₋₄alkyloxy-, aminocarbonyl-and mono- or di(C₁₋₄alkyl)aminocarbonyl-, or
C₁₋₄alkyloxycarbonyl optionally substituted with one or more substituents selected from hydroxy-, C₁₋₄alkyloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-;
Het¹ represents thiazolyl or 2-bora-1,3-dioxolanyl wherein said Het¹ is optionally substituted with one or where possible two, three, four or more substituents selected from amino, C₁₋₄alkyl, hydroxy-C₁₋₄alkyl-, phenyl, phenyl-C₁₋₄alkyl-, C₁₋₄alkyl-oxy-C₁₋₄alkyl-, mono- or di(C₁₋₄alkyl)amino- or amino-carbonyl-;
Het² represents a heterocycle selected from tetrahydropyranyl, tetrahydrofuranyl, furanyl, 1,1-dioxothiomorpholinyl, piperazininonyl, tetrahydro-1,1-dioxido-2H-thiopyranyl, piperidinonyl, azetidinyl or 2-azetidinonyl wherein said Het² is optionally substituted with one or where possible two or more substituents selected from hydroxy, amino, NR²⁹R³⁰, aminocarbonyl, mono- or
di(C₁₋₄alkyl)aminocarbonyl, C₁₋₄alkylsulfonyl or
C₁₋₄alkyl- optionally substituted with one or more substituents selected from NR²⁷R²⁸, C₁₋₄alkylsulfonyl, aminocarbonyloxy-, aminocarbonyl- and mono- or di(C₁₋₄alkyl)aminocarbonyl-, or
C₁₋₄alkyloxy- optionally substituted with C₁₋₄alkyloxy-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-; or
Het² represents a heterocycle selected from morpholinyl, piperazinyl, piperidinyl, pyrrolidinyl, thiomorpholinyl or 1,1-dioxothiomorpholinyl wherein said Het² is optionally substituted with one or where possible two or more substituents selected from
C₁₋₄alkyl- optionally substituted with one or more substituents selected from NR²⁷R²⁸, C₁₋₄alkylsulfonyl, aminocarbonyloxy-, aminocarbonyl- and mono- or di(C₁₋₄alkyl)aminocarbonyl-, or
C₁₋₄alkyloxy- optionally substituted with C₁₋₄alkyloxy-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-;
Het³ represents a heterocycle selected from tetrahydropyranyl, tetrahydrofuranyl, furanyl, 1,1-dioxothiomorpholinyl, piperazininonyl, tetrahydro-1,1-dioxido-2H-thiopyranyl, piperidinonyl, azetidinyl or 2-azetidinonyl wherein said Het³ is optionally substituted with one or where possible two or more substituents hydroxy-, amino, C₁₋₄alkyl-, C₃₋₆cycloalkyl-C₁₋₄alkyl-, aminosulfonyl-, mono- or di(C₁₋₄alkyl)aminosulfonyl-, amino-C₁₋₄alkyl-, Mono- or di(C₁₋₄alkyl)amino-C₁₋₄alkyl, NR³⁵R³⁶, C₁₋₄alkyl-sulfonyl-C₁₋₄alkyl- or C₁₋₄alkyloxy- optionally substituted with C₁₋₄alkyloxy- or hydroxy; or
Het³ represents a heterocycle selected from morpholinyl, piperazinyl, piperidinyl, furanyl or pyrrolidinyl wherein said Het³ is substituted with one or where possible two or more substituents selected from NR³⁵R³⁶, C₁₋₄alkyl-sulfonyl-C₁₋₄alkyl- or C₁₋₄alkyloxy- optionally substituted with C₁₋₄alkyloxy- or hydroxy;
Het⁴ represents a heterocycle selected from morpholinyl, piperazinyl, piperidinyl, furanyl, pyrazolyl, dioxolanyl, thiazolyl, oxazolyl, imidazolyl, isoxazolyl, oxadiazolyl, pyridinyl or pyrrolidinyl wherein said Het⁴ is substituted with one or where possible two or more substituents selected from C₁₋₄alkyl-sulfonyl-C₁₋₄alkyl-, C₁₋₄alkyloxy- optionally substituted with C₁₋₄alkyloxy- or hydroxy;
Het⁵ represents a heterocycle selected from furanyl, piperazinyl, 1,1-dioxothiomorpholinyl, piperazininonyl, piperidinyl, tetrahydro-1,1-dioxido-2H-thiopyranyl, piperidinonyl, morpholinyl or pyrrolidinyl wherein said Het⁵ is optionally substituted with hydroxy, amino, mono- or di(C₁₋₄alkyl)-amino-, C₁₋₄alkyl,
Het⁶ and Het⁷ each independently represents a heterocycle selected from piperazinyl, piperidinyl or pyrrolidinyl wherein said heterocycles are optionally substituted with one or more substituents selected from hydroxy-, amino, hydroxy-C₁₋₄alkyl-, C₁₋₄alkyloxy-C₁₋₄alkyl- and C₁₋₄alkyl-;
Het⁸ represents a heterocycle selected from tetrahydropyranyl, tetrahydrofuranyl, 1,1 -dioxothiomorpholinyl, piperazininonyl, tetrahydro-1,1-dioxido-2H-thiopyranyl, piperidinonyl, azetidinyl or 2-azetidinonyl wherein said Het⁸ is optionally substituted with aminosulfonyl, aminocarbonyl,
mono- or di(C₁₋₄alkyl)aminocarbonyl-, mono- or di(C₁₋₄alkyl)aminosulfonyl-, or C₁₋₄alkyl- optionally substituted with one or more substituents selected from amino, mono- or di(C₁₋₄alkyl)amino-, NR³³R³⁴, C₁₋₄alkylsulfonyl, aminocarbonyloxy-, hydroxy-, C₁₋₄alkyloxy-, aminocarbonyl- and mono- or di(C₁₋₄alkyl)aminocarbonyl-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy, C₁₋₄alkyloxy- and C₁₋₄alkylsulfonyl-; or
Het⁸ represents a heterocycle selected from furanyl, piperidinyl or piperazinyl wherein said Het⁸ is substituted with aminocarbonyl,
mono- or di(C₁₋₄alkyl)aminocarbonyl-, mono- or di(C₁₋₄alkyl)aminosulfonyl-, or C₁₋₄alkyl- substituted with one or more substituents selected from NR³³R³⁴, C₁₋₄alkylsulfonyl, aminocarbonyloxy-, hydroxy-, C₁₋₄alkyloxy-, aminocarbonyl-and mono- or di(C₁₋₄alkyl)aminocarbonyl-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy, C₁₋₄alkyloxy- and C₁₋₄alkylsulfonyl-;
Het⁹ and Het¹⁰ each independently represents a heterocycle selected from piperazinyl, piperidinyl or pyrrolidinyl wherein said heterocycles are optionally substituted with one or more substituents selected from hydroxy-, amino, hydroxy-C₁₋₄alkyl-, C₁₋₄alkyloxy-C₁₋₄alkyl- and C₁₋₄alkyl-;
Het¹¹ represents 2-imidazolidinonyl- or 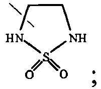 Het¹² represents a heterocycle selected from morpholinyl, piperazinyl, piperidinyl or pyrrolidinyl wherein said Het¹² is optionally substituted with one or where possible two or more substituents selected from hydroxy, amino or C₁₋₄alkyl-;
Het¹³ represents a heterocycle selected from furanyl, piperazinyl, 1,1-dioxothiomorpholinyl, piperazininonyl, piperidinyl, tetrahydro-1,1-dioxido-2H-thiopyranyl, piperidinonyl, morpholinyl, piperazinyl or pyrrolidinyl;
Het¹⁴ and Het¹⁵ each independently represent a heterocycle selected from morpholinyl, piperazinyl, piperidinyl or pyrrolidinyl wherein said Het¹⁴ and Het¹⁵ are optionally substituted with one or where possible two or more substituents selected from hydroxy, amino or C₁₋₄alkyl;
Het¹⁶ represents a heterocycle selected from piperidinyl or pyrrolidinyl;
Het²⁰ represents pyrrolidinyl, 2-pyrrolidinonyl, piperidinyl or hydroxy-pyrrolidinyl, preferably pyrrolidinyl or hydroxy-pyrrolidinyl;
Het²¹ represents pyrrolidinyl or hydroxy-pyrrolidinyl;
Het²² represents pyrrolidinyl, piperazinyl or piperidinyl;
Het²³ and Het²⁵ each independently represents a heterocycle selected from morpholinyl, pyrrolidinyl, piperazinyl or piperidinyl wherein said Het²³ is optionally substituted with one or where possible two or more substituents selected from C₁₋₄alkyl, C₃₋₆cycloalkyl, hydroxy-C₁₋₄alkyl-, C₁₋₄alkyloxyC₁₋₄alkyl or polyhydroxy-C₁₋₄alkyl-;
Het²⁴ represents morpholinyl, pyrrolidinyl, piperazinyl or piperidinyl;
Ar⁴, Ar⁵ or Ar⁶ each independently represent phenyl optionally substituted with nitro, cyano, C₁₋₄alkylsulfonyl-, C₁₋₄alkylsulfonylamino-, aminosulfonylamino-, hydroxy-C₁₋₄alkyl, aminosulfonyl-, hydroxy-, C₁₋₄alkyloxy- or C₁₋₄alkyl, preferably Ar⁴ or Ar⁵ each independently represent phenyl optionally substituted with cyano;
further characterised in that either
Y represents -C₁₋₂alkyl-NR²³-CO-CR¹⁶R¹⁷-NH-;
Het¹ represents 2-bora-1,3-dioxolanyl optionally substituted with one or where possible two, three, four or more substituents selected from amino, C₁₋₄alkyl, hydroxy-C₁₋₄alkyl-, phenyl, phenyl-C₁₋₄alkyl-, C₁₋₄alkyl-oxy-C₁₋₄alkyl-, mono-or di(C₁₋₄alkyl)amino- or amino-carbonyl-;
R¹³ represents C₁₋₆alkyloxycarbonyl optionally substituted with phenyl or R¹³ represents Ar⁶-sulfonyl or Het²⁴-C₁₋₄alkylcarbonyl; or
R⁴ represents C₁₋₄alkyloxy substituted with at least one substituent selected from C₁₋₄alkyloxy-C₁₋₄alkyloxy-, NR³⁷R³⁸-carbonyloxy-, Het⁵-carbonyloxy-, NR⁷R⁸, NR⁹R¹⁰-carbonyl-, Het³-carbonyl-, Het¹³-oxy- or Het²-; wherein
   R⁸ represents Het⁷-aminocarbonyl-; Het⁹-oxycarbonyl-; Het¹⁰-sulfonyl-; C₁₋₄alkyloxycarbonyl; mono- or di(C₁₋₄alkyl)aminocarbonyl-; mono- or di(C₁₋₄alkyl)aminocarbonyl substituted with C₁₋₄alkylsulfonyl-; or C₁₋₄alkylcarbonyl optionally substituted with one or more substituents selected from C₁₋₄alkylsulfonyl, hydroxy- and C₁₋₄alkyloxy-; or R⁸ represents C₁₋₄alkyl substituted with one or more substituents selected from C₁₋₄alkylsulfonyl-, NR²⁵R²⁶, aminocarbonyloxy-, C₁₋₄alkylcarbonyloxy-, aminocarbonyl-, C₁₋₄alkyloxy-C₁₋₄alkyloxy-, and Het¹¹; and
   Het² represents a heterocycle selected from morpholinyl, piperazinyl, piperidinyl, pyrrolidinyl or thiomorpholinyl said Het² substituted with one or where possible two or more substituents selected from C₁₋₄alkyl- substituted with one or more substituents selected from NR²⁷R²⁸, C₁₋₄alkylsulfonyl, aminocarbonyloxy-, aminocarbonyl- and mono- or di(C₁₋₄alkyl)aminocarbonyl-; or
      C₁₋₄alkyloxy- optionally substituted with C₁₋₄alkyloxy-; or C₁₋₄alkyloxycarbonyl optionally substituted with one or more substituents selected from hydroxy, C₁₋₄alkyloxy- and C₁₋₄alkylsulfonyl-; or C₁₋₄alkylcarbonyl optionally substituted with one or more substituents selected from hydroxy-, C₁₋₄alkyloxy- and C₁₋₄alkylsulfonyl-;
      or Het² represents 1,1-dioxothiomorpholinyl optionally substituted with C₁₋₄alkyl- optionally substituted with one or more substituents selected from NR²⁷R²⁸, C₁₋₄alkylsulfonyl, aminocarbonyloxy-, aminocarbonyl-and mono- or di(C₁₋₄alkyl)aminocarbonyl-; or
      C_{I}-4alkyloxy- optionally substituted with C₁₋₄alkyloxy-; or C₁₋₄alkyloxycarbonyl optionally substituted with one or more substituents selected from hydroxy, C₁₋₄alkyloxy- and C₁₋₄alkylsulfonyl-;
      or C₁₋₄alkylcarbonyl optionally substituted with one or more substituents selected from hydroxy-, C₁₋₄alkyloxy- and C₁₋₄alkylsulfonyl-.

As used in the foregoing definitions and hereinafter,
- halo is generic to fluoro, chloro, bromo and iodo;
- C₁₋₂alkyl defines methyl or ethyl;
- C₁₋₃alkyl defines straight and branched chain saturated hydrocarbon radicals having from 1 to 3 carbon atoms such as, for example, methyl, ethyl, propyl and the like;
- C₁₋₄alkyl defines straight and branched chain saturated hydrocarbon radicals having from 1 to 4 carbon atoms such as, for example, methyl, ethyl, propyl, butyl, 1-methylethyl, 2-methylpropyl, 2,2-dimethylethyl and the like;
- C₁₋₅alkyl defines straight and branched chain saturated hydrocarbon radicals having from 1 to 5 carbon atoms such as, for example, methyl, ethyl, propyl, butyl, pentyl, 1-methylbutyl, 2,2-dimethylpropyl, 2,2-dimethylethyl and the like;
- C₁₋₆alkyl is meant to include C₁₋₅alkyl and the higher homologues thereof having 6 carbon atoms such as, for example hexyl, 1,2-dimethylbutyl, 2-methylpentyl and the like;
- C₁₋₇alkyl is meant to include C₁₋₆alkyl and the higher homologues thereof having 7 carbon atoms such as, for example 1,2,3-dimethylbutyl, 1,2-methylpentyl and the like;
- C₃₋₉alkyl defines straight and branched chain saturated hydrocarbon radicals having from 3 to 9 carbon atoms such as propyl, butyl, pentyl, hexyl, heptyl, octyl, nonyl and the like;
- C₂₋₄alkenyl defines straight and branched chain hydrocarbon radicals containing one double bond and having from 2 to 4 carbon atoms such as, for example vinyl, 2-propenyl, 3-butenyl, 2-butenyl and the like;
- C₃₋₉alkenyl defines straight and branched chain hydrocarbon radicals containing one double bond and having from 3 to 9 carbon atoms such as, for example 2-propenyl, 3-butenyl, 2-butenyl, 2-pentenyl, 3-pentenyl, 3-methyl-2-butenyl, 3-hexenyl and the like;
- C₂₋₆alkynyl defines straight and branched chain hydrocarbon radicals containing one triple bond and having from 2 to 6 carbon atoms such as, for example, 2-propynyl, 3-butynyl, 2-butynyl, 2-pentynyl, 3-pentynyl, 3-methyl-2-butynyl, 3-hexynyl and the like;
- C₃₋₆cycloalkyl is generic to cyclopropyl, cyclobutyl, cyclopentyl and cyclohexyl;
- C₁₋₄alkyloxy defines straight or branched saturated hydrocarbon radicals such as methoxy, ethoxy, propyloxy, butyloxy, 1-methylethyloxy, 2-methylpropyloxy and the like;
- C₁₋₆alkyloxy is meant to include C₁₋₄alkyloxy and the higher homologues such as methoxy, ethoxy, propyloxy, butyloxy, 1-methylethyloxy, 2-methylpropyloxy and the like;
- polyhydroxy-C₁₋₄alkyl is generic to a C₁₋₄alkyl as defined hereinbefore, having two, three or where possible more hydroxy substituents, such as for example trifluoromethyl.

As used in the foregoing definitions and hereinafter, the term formyl refers to a radical of formula -CH(=O). When X¹ represents the divalent radical -O-N=CH-, said radical is attached with the carbon atom to the R³, R⁴ bearing cyclic moiety of the compounds of formula (I) and when X² represents the divalent radical -O-N=CH-, said radical is attached with the carbon atom to the R¹, R² bearing phenyl moiety of the compounds of formula (I).

The heterocycles as mentioned in the above definitions and hereinafter, are meant to include all possible isomeric forms thereof, for instance pyrrolyl also includes 2*H*-pyrrolyl; triazolyl includes 1,2,4-triazolyl and 1,3,4-triazolyl; oxadiazolyl includes 1,2,3-oxadiazolyl, 1,2,4-oxadiazolyl, 1,2,5-oxadiazolyl and 1,3,4-oxadiazolyl; thiadiazolyl includes 1,2,3-thiadiazolyl, 1,2,4-thiadiazolyl, 1,2,5-thiadiazolyl and 1,3,4-thiadiazolyl; pyranyl includes 2*H*-pyranyl and 4*H-*pyranyl.

Further, the heterocycles as mentioned in the above definitions and hereinafter may be attached to the remainder of the molecule of formula (I) through any ring carbon or heteroatom as appropriate. Thus, for example, when the heterocycle is imidazolyl, it maybe a 1-imidazolyl, 2-imidazolyl, 3-imidazolyl, 4-imidazolyl and 5-imidazolyl; when it is thiazolyl, it may be 2-thiazolyl, 4-thiazolyl and 5-thiazolyl; when it is triazolyl, it may be 1,2,4-triazol-1-yl, 1,2,4-triazol-3-yl, 1,2,4-triazol-5-yl, 1,3,4-triazol-1-yl and 1,3,4-triazol-2-yl; when it is benzothiazolyl, it may be 2-benzothiazolyl, 4-benzothiazolyl, 5-benzothiazolyl, 6-benzothiazolyl and 7-benzothiazolyl.

The pharmaceutically acceptable addition salts as mentioned hereinabove are meant to comprise the therapeutically active non-toxic acid addition salt forms which the compounds of formula (I) are able to form. The latter can conveniently be obtained by treating the base form with such appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic (i.e. butane-dioic acid), maleic, fumaric, malic, tartaric, citric, methanesulfonic, ethanesulfonic, benzenesulfonic, *p*-toluenesulfonic, cyclamic, salicylic, *p*-aminosalicylic, pamoic and the like acids.
The pharmaceutically acceptable addition salts as mentioned hereinabove are meant to comprise the therapeutically active non-toxic base addition salt forms which the compounds of formula (I) are able to form. Examples of such base addition salt forms are, for example, the sodium, potassium, calcium salts, and also the salts with pharmaceutically acceptable amines such as, for example, ammonia, alkylamines, benzathine, *N*-methyl-D-glucamine, hydrabamine, amino acids, e.g. arginine, lysine.

Conversely said salt forms can be converted by treatment with an appropriate base or acid into the free acid or base form.

The term addition salt as used hereinabove also comprises the solvates which the compounds of formula (I) as well as the salts thereof, are able to form. Such solvates are for example hydrates, alcoholates and the like.

The term stereochemically isomeric forms as used hereinbefore defines the possible different isomeric as well as conformational forms which the compounds of formula (I) may possess. Unless otherwise mentioned or indicated, the chemical designation of compounds denotes the mixture of all possible stereochemically and conformationally isomeric forms, said mixtures containing all diastereomers, enantiomers and/or conformers of the basic molecular structure. All stereochemically isomeric forms of the compounds of formula (I) both in pure form or in admixture with each other are intended to be embraced within the scope of the present invention.

Some of the compounds of formula (I) may also exist in their tautomeric forms. Such forms although not explicitly indicated in the above formula are intended to be included within the scope of the present invention.

The *N*-oxide forms of the compounds of formula (I) are meant to comprise those compounds of formula (I) wherein one or several nitrogen atoms are oxidized to the so-called *N*-oxide.

A first group of compounds according to the present invention consists of those compounds of formula (I) wherein one or more of the following restrictions apply;
Z represents NH;
Y represents -C₃₋₉alkyl-, -C₂₋₉alkenyl-, -C₁₋₅alkyl-oxy-C₁₋₅alkyl-, -C₁₋₅alkyl-NR¹³-C₁₋₅alkyl-, -C₁₋₅alkyl-NR¹⁴-CO-C₁₋₅alkyl-, -C₁₋₆alkyl-NH-CO-, -NH-CO-C₁₋₆alkyl-, -CO-C₁₋₇alkyl-, -C₁₋₇alkyl-CO-, C₁₋₆alkyl-CO-C₁₋₆alkyl, -C₁₋₂alkyl-NR²³-CO-CR¹⁶R¹⁷-NH-, -C₁₋₂alkyl-CO-NH-CR¹⁸R¹⁹-CO-, -C₁₋₂alkyl-CO-NR²⁰-C₁₋₃alkyl-CO-, -C₁₋₂alkyl-NR²¹-CH₂-CO-NH-C₁₋₃alkyl-, -NR²²-CO-C₁₋₃alkyl-NH-, -C₁₋₃alkyl-NH-CO-Het²⁰-, C₁₋₂alkyl-CO-Het²¹-CO-, or -Het²²-CH₂-CO-NH-C₁₋₃alkyl-;
X¹ represents O, -O-C₁₋₂alkyl-, -O-N=CH-, NR¹¹ or -NR¹¹-C₁₋₂alkyl-; in a particular embodiment X¹ represents O, -O-C₁₋₂alkyl- or NR¹¹-C₁₋₂alkyl;
X² represents a direct bond, C₁₋₂alkyl, O, -O-C₁₋₂alkyl-, CO, -CO-C₁₋₂alkyl-, -O-N=CH-, NR¹² or NR¹²-C₁₋₂alkyl-; in a particular embodiment X² represents a direct bond, -O-, -O-C₁₋₂alkyl, -CO-C₁₋₂alkyl- or NR¹²-C₁₋₂alkyl-;
R¹ represents hydrogen, cyano, halo or hydroxy, preferably halo;
R² represents hydrogen, cyano, halo, hydroxy, hydroxycarbonyl-, C₁₋₄alkyloxycarbonyl-, Het¹⁶-carbonyl-, C₁₋₄alkyl-, C₂₋₆alkynyl-, Ar⁵, Het¹ or dihydroxyborane;
R³ represents hydrogen, cyano, halo, hydroxy, formyl, C₁₋₆alkoxy-, C₁₋₆alkyl-, C₁₋₆alkoxy- substituted with halo, or R³ represents C₁₋₄alkyl substituted with one or where possible two or more substituents selected from hydroxy or halo;
R⁴ represents Ar⁴-C₁₋₄alkyloxy-, C₁₋₄alkyloxy- or R⁴ represents C₁₋₄alkyloxy substituted with one or where possible two or more substituents selected from hydroxy-, halo, C₁₋₄alkyloxy-, C₁₋₄alkyloxy-C₁₋₄alkyloxy-, NR³⁷R³⁸-carbonyloxy-, Het⁵-carbonyloxy-, NR⁷R⁸, NR⁹R¹⁰-carbonyl-, Het³-carbonyl-, Het¹³-oxy- or Het²-;
R⁷ represents hydrogen or C₁₋₄alkyl;
R⁸ represents C₃₋₆cycloalkyl, Het⁶-carbonyl-, Het⁷-aminocarbonyl-, Het⁸, Het⁹-oxycarbonyl-, Het¹⁰-sulfonyl-, mono- or di(C₁₋₄alkyl)aminocarbonyl-, mono-or di(C₁₋₄alkyl)aminocarbonyl substituted with C₁₋₄alkylsulfonyl-, or C₁₋₄alkylcarbonyl optionally substituted with one or more substituents selected from C₁₋₄alkylsulfonyl, hydroxy- and C₁₋₄alkyloxy-, or R⁸ represents C₁₋₄alkyl substituted with one or more substituents selected from C₁₋₄alkylsulfonyl-, NR²⁵R²⁶, aminocarbonyloxy-, aminocarbonyl-, C₁₋₄alyloxy-C₁₋₄alkyloxy-, and Het¹¹;
R⁹ represents hydrogen or C₁₋₄alkyl-;
R¹⁰ represents Het⁴ or C₁₋₄alkyl- substituted with C₁₋₄alkylsulfonyl-;
R¹¹ represents hydrogen, C₁₋₄alkyl- or C₁₋₄alkyl-oxy-carbonyl-;
R¹² represents hydrogen, C₁₋₄alkyl-, C₁₋₆alkyloxycarbonyl- or C₁₋₆alkyloxycarbonyl-substituted with phenyl;
R¹³ represents hydrogen, Het¹⁴-C₁₋₄alkyl, C₁₋₆alkyloxycarbonyl optionally substituted with phenyl or R¹³ represents Ar⁶-sulfonyl or Het²⁴-C₁₋₄alkylcarbonyl; in particular morpholinyl-C₁₋₄alkyl;
R¹⁴ and R¹⁵ are each independently selected from hydrogen, C₁₋₄alkyl, Het¹⁵-C₁₋₄alkyl- or C₁₋₄alkyloxyC₁₋₄alkyl-;;
R¹⁶ and R¹⁷ each independently represents hydrogen, C₁₋₄alkyl or C₁₋₄alkyl substituted with hydroxy- or phenyl; or R¹⁶ and R¹⁷ taken together with the carbon atom to which they are attached form a C₃₋₆cycloalkyl;
R¹⁸ represents hydrogen or C₁₋₄alkyl optionally substituted with hydroxy or phenyl;
R¹⁹ represents hydrogen or C₁₋₄alkyl, in particular hydrogen or methyl, even more particular hydrogen;
R²⁰ represents hydrogen or C₁₋₄alkyl, in particular hydrogen or methyl;
R²¹ represents hydrogen, C₁₋₄alkyl, Het²³-C₁₋₄alkylcarbonyl- or R²¹ represents mono-or di(C₁₋₄alkyl)amino-C₁₋₄alkyl-carbonyl- optionally substituted with hydroxy, pyrimidinyl, dimethylamine or C₁₋₄alkyloxy;
R²² represents hydrogen or C₁₋₄alkyl optionally substituted with hydroxy or C₁₋₄alkyloxy;
R²³ represents C₁₋₄alkyl optionally substituted with hydroxy-, C₁₋₄alkyloxy- or Het²³; R²³ may also represent hydrogen when R¹⁶ and R¹⁷ taken together with the carbon atom to which they are attached form a C₃₋₆cycloalkyl;
R²⁵ and R²⁶ each independently represent hydrogen, C₁₋₄alkyl, C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-, in particular R²⁵ and R²⁶ each independently represent hydrogen, C₁₋₄alkyl, C₁₋₄alkylsulfonyl-, aminocarbonyl-, mono- or di(C₁₋₄alkyl)aminocarbonyl- or C₁₋₄alkylcarbonyl-;
R²⁷ and R²⁸ each independently represent hydrogen, C₁₋₄alkyl, C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R²⁹ and R³⁰ each independently represent hydrogen, aminosulfonyl, aminocarbonyl, mono- or di(C₁₋₄alkyl)aminocarbonyl-, mono- or di(C₁₋₄alkyl)aminosulfonyl-, or C₁₋₄alkyl- optionally substituted with one or more substituents selected from NR³¹R³², C₁₋₄alkylsulfonyl, aminocarbonyloxy-, hydroxy-, C₁₋₄alkyloxy-, aminocarbonyl- and mono- or di(C₁₋₄alkyl)aminocarbonyl-, or C₁₋₄alkyloxycarbonyl optionally substituted with one or more substituents selected from hydroxy, C₁₋₄alkyloxy- and C₁₋₄alkylsulfonyl-, or C₁₋₄alkylcarbonyl optionally substituted with one or more substituents selected from hydroxy-, C₁₋₄alkyloxy- and C₁₋₄alkylsulfonyl-;
R³¹ and R³² each independently represent hydrogen, C₁₋₄alkyl, C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R³³ represents hydrogen or C₁₋₄alkyl;
R³⁴ represents C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R³⁵ represents hydrogen or C₁₋₄alkyl;
R³⁶ represents C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R³⁷ and R³⁸ each independently represent hydrogen, C₁₋₄alkyl, C₁₋₄alkylsulfonyl-, Het¹² or C₁₋₄alkyl substituted with one or more substituents selected from C₁₋₄alkylsulfonyl-, hydroxy- and C₁₋₄alkyloxy-;
R³⁹ and R⁴⁰ each independently represent aminosulfonyl, aminocarbonyl, mono- or di(C₁₋₄alkyl)aminocarbonyl-, mono- or di(C₁₋₄alkyl)aminosulfonyl-, or C₁₋₄alkyl- substituted with one or more substituents selected from NR³¹R³², C₁₋₄alkylsulfonyl, aminocarbonyloxy-, hydroxy-, C₁₋₄alkyloxy-, aminocarbonyl-and mono- or di(C₁₋₄alkyl)aminocarbonyl-, or C₁₋₄alkyloxycarbonyl optionally substituted with one or more substituents selected from hydroxy, C₁₋₄alkyloxy- and C₁₋₄alkylsulfonyl-, or C₁₋₄alkylcarbonyl optionally substituted with one or more substituents selected from hydroxy-, C₁₋₄alkyloxy- and C₁₋₄alkylsulfonyl-;
Het¹ represents thiazolyl or 2-bora-1,3-dioxolanyl wherein said Het¹ is optionally substituted with one or where possible two, three, four or more substituents selected from amino, C₁₋₄alkyl, hydroxy-C₁₋₄alkyl-, phenyl, phenyl-C₁₋₄alkyl-, C₁₋₄alkyl-oxy-C₁₋₄alkyl-, mono- or di(C₁₋₄alkyl)amino- or amino-carbonyl-;
Het² represents a heterocycle selected from tetrahydropyranyl, tetrahydrofuranyl, furanyl, 1,1-dioxothiomorpholinyl, piperazininonyl, tetrahydro-1,1-dioxido-2H-thiopyranyl, piperidinonyl, azetidinyl or 2-azetidinonyl wherein said Het² is optionally substituted with one or where possible two or more substituents selected from hydroxy, amino, NR²⁹R³⁰, aminocarbonyl, mono- or
di(C₁₋₄alkyl)aminocarbonyl, C₁₋₄alkylsulfonyl or
C₁₋₄alkyl- optionally substituted with one or more substituents selected from NR²⁷R²⁸, C₁₋₄alkylsulfonyl, aminocarbonyloxy-, aminocarbonyl- and mono- or di(C₁₋₄alkyl)aminocarbonyl-, or
C₁₋₄alkyloxy- optionally substituted with C₁₋₄alkyloxy-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-; or
Het² represents a heterocycle selected from morpholinyl, piperazinyl, piperidinyl or pyrrolidinyl wherein said morpholinyl, piperazinyl, piperidinyl or pyrrolidinyl are optionally substituted with one or where possible two or more substituents selected from
C₁₋₄alkyl- optionally substituted with one or more substituents selected from NR²⁷R²⁸, C₁₋₄alkylsulfonyl, aminocarbonyloxy-, aminocarbonyl- and mono- or di(C₁₋₄alkyl)aminocarbonyl-, or
C₁₋₄alkyloxy- optionally substituted with C₁₋₄alkyloxy-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy-, C₁₋4alkyloxy- and C₁₋₄alkylsulfonyl-;
Het³ represents a heterocycle selected from tetrahydropyranyl, tetrahydrofuranyl, furanyl, 1,1-dioxothiomorpholinyl, piperazininonyl, tetrahydro-1,1-dioxido-2H-thiopyranyl, piperidinonyl, azetidinyl or 2-azetidinonyl wherein said Het³ is optionally substituted with one or where possible two or more substituents hydroxy-, amino, C₁₋₄alkyl-, C₃₋₆cycloalkyl-C₁₋₄alkyl-, aminosulfonyl-, mono- or di(C₁₋₄alkyl)aminosulfonyl-, amino-C₁₋₄alkyl-, Mono- or di(C₁₋₄alkyl)amino-C₁₋₄alkyl, NR³⁵R³⁶, C₁₋₄alkyl-sulfonyl-C₁₋₄alkyl- or C₁₋₄alkyloxy- optionally substituted with C₁₋₄alkyloxy- or hydroxy; or
Het³ represents a heterocycle selected from morpholinyl, piperazinyl, piperidinyl or pyrrolidinyl wherein said Het³ is optionally substituted with one or where possible two or more substituents selected from NR³⁵R³⁶, C₁₋₄alkyl-sulfonyl-C₁₋₄alkyl- or C₁₋₄alkyloxy- optionally substituted with C₁₋₄alkyloxy- or hydroxy;
Het⁴ represents a heterocycle selected from morpholinyl, piperazinyl, piperidinyl, furanyl, pyrazolyl, dioxolanyl, thiazolyl, oxazolyl, imidazolyl, isoxazolyl, oxadiazolyl, pyridinyl or pyrrolidinyl wherein said Het⁴ is substituted with one or where possible two or more substituents selected from C₁₋₄alkyl-sulfonyl-C₁₋₄allcyl-, C₁₋₄alkyloxy- optionally substituted with C₁₋₄alkyloxy- or hydroxy;
Het⁵ represents a heterocycle selected from furanyl, piperazinyl, 1,1-dioxothiomorpholinyl, piperazininonyl, piperidinyl, tetrahydro-1,1-dioxido-2H-thiopyranyl, piperidinonyl, morpholinyl or pyrrolidinyl wherein said Het⁵ is optionally substituted with hydroxy, amino, mono- or di(C₁₋₄alkyl)-amino-, C₁₋₄alkyl,
Het⁶ and Het⁷ each independently represents a heterocycle selected from piperazinyl, piperidinyl or pyrrolidinyl wherein said heterocycles are optionally substituted with one or more substituents selected from hydroxy-, amino-, hydroxy-C₁₋₄alkyl-, C₁₋₄alkyloxy-C₁₋₄alkyl- and C₁₋₄alkyl-;
Het⁸ represents a heterocycle selected from tetrahydropyranyl, tetrahydrofuranyl, 1,1 -dioxothiomorpholinyl, piperazininonyl, tetrahydro-1,1-dioxido-2H-thiopyranyl, piperidinonyl, azetidinyl or 2-azetidinonyl wherein said Het⁸ is optionally substituted with aminosulfonyl, aminocarbonyl,
mono- or di(C₁₋₄alkyl)aminocarbonyl-, mono- or di(C₁₋₄alkyl)aminosulfonyl-, or C₁₋₄alkyl- optionally substituted with one or more substituents selected from amino, mono- or di(C₁₋₄alkyl)amino-, NR³³R³⁴, C₁₋₄alkylsulfonyl, aminocarbonyloxy-, hydroxy-, C₁₋₄alkyloxy-, aminocarbonyl- and mono- or di(C₁₋₄alkyl)aminocarbonyl-, or
C₁₋₄alkyloxycarbonyl optionally substituted with one or more substituents selected from hydroxy-, C₁₋₄alkyloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-; or
Het⁸ represents a heterocycle selected from furanyl, piperidinyl or piperazinyl wherein said Het⁸ is substituted with aminocarbonyl,
mono- or di(C₁₋₄alkyl)aminocarbonyl-, mono- or di(C₁₋₄alkyl)aminosulfonyl-, or C₁₋₄alkyl- substituted with one or more substituents selected from NR³³R³⁴,
C₁₋₄alkylsulfonyl, aminocarbonyloxy-, hydroxy-, C₁₋₄alkyloxy-, aminocarbonyl-and mono- or di(C₁₋₄alkyl)aminocarbonyl-, or
C₁₋₄alkyloxycarbonyl optionally substituted with one or more substituents selected from hydroxy-, C₁₋₄alkyloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-;
Het⁹ and Het¹⁰ each independently represents a heterocycle selected from piperazinyl, piperidinyl or pyrrolidinyl wherein said heterocycles are optionally substituted with one or more substituents selected from hydroxy-, amino, hydroxy-C₁₋₄alkyl-, C₁₋₄alkyloxy-C₁₋₄alkyl- and C₁₋₄alkyl-;
Het¹¹ represents 2-imidazolidinonyl- or 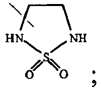 Het¹² represents a heterocycle selected from morpholinyl, piperazinyl, piperidinyl or pyrrolidinyl wherein said Het¹² is optionally substituted with one or where possible two or more substituents selected from hydroxy-, amino or C₁₋₄alkyl-;
Het¹³ represents a heterocycle selected from furanyl, piperazinyl, 1,1-dioxothiomorpholinyl, piperazininonyl, piperidinyl, tetrahydro-1,1-dioxido-2H-thiopyranyl, piperidinonyl, morpholinyl, piperazinyl or pyrrolidinyl
Het¹⁶ represents a heterocycle selected from piperidinyl or pyrrolidinyl;
Het²⁰ represents pyrrolidinyl, 2-pyrrolidinonyl, piperidinyl or hydroxy-pyrrolidinyl, preferably pyrrolidinyl or hydroxy-pyrrolidinyl;
Het²¹ represents pyrrolidinyl or hydroxy-pyrrolidinyl;
Het²² represents pyrrolidinyl, piperazinyl or piperidinyl;
Het²³ represents a heterocycle selected from morpholinyl, pyrrolidinyl, piperazinyl or piperidinyl wherein said Het²³ is optionally substituted with one or where possible two or more substituents selected from C₁₋₄alkyl, C₃₋₆cycloalkyl, hydroxy-C₁₋₄alkyl-, C₁₋₄alkyloxyC₁₋₄alkyl or polyhydroxy-C₁₋₄alkyl-;
Ar⁴, Ar⁵ or Ar⁶ each independently represent phenyl optionally substituted with nitro, cyano, C₁₋₄alkylsulfonyl-, C₁₋₄alkylsulfonylamino-, aminosulfonylamino-, hydroxy-C₁₋₄alkyl, aminosulfonyl-, hydroxy-, C₁₋₄alkyloxy- or C₁₋₄alkyl, preferably Ar⁴ or Ar⁵ each independently represent phenyl optionally substituted with cyano;
further characterised in that either
Y represents -C₁₋₂alkyl-NR²³-CO-CR¹⁶R¹⁷-NH-;
Het¹ represents 2-bora-1,3-dioxolanyl optionally substituted with one or where possible two, three, four or more substituents selected from amino, C₁₋₄alkyl, hydroxy-C₁₋₄alkyl-, phenyl, phenyl-C₁₋₄alkyl-, C₁₋₄alkyl-oxy-C₁₋₄alkyl-, mono-or di(C₁₋₄alkyl)amino- or amino-carbonyl-;
R¹³ represents C₁₋₆alkyloxycarbonyl optionally substituted with phenyl or R¹³ represents Ar⁶-sulfonyl or Het²⁴-C₁₋₄alkylcarbonyl; or
R⁴ represents C₁₋₄alkyloxy substituted with at least one substituent selected from C₁₋₄alkyloxy-C₁₋₄alkyloxy-, NR³⁷R³⁸-carbonyloxy-, Het⁵-carbonytoxy-, NR⁷R⁸, NR⁹R¹⁰-carbonyl-, Het³-carbonyl-, Het¹³-oxy- or Het²-.

Another group of compounds according to the present invention consists of those compounds of formula (I) wherein one or more of the following restrictions apply;
Z represents NH;
Y represents -C₃₋₉alkyl-, -C₁₋₅alkyl-NR¹³-C₁₋₅alkyl-, -C₁₋₅alkyl-NR¹⁴-CO-C₁₋₅alkyl-, -C₁₋₆alkyl-CO-NH-, -C₁₋₆alkyl-NH-CO-, -C₁₋₂alkyl-NR²³-CO-CR¹⁶R¹⁷-NH-, -C₁₋₂alkyl-NR²¹-CH₂-CO-NH-C₁₋₃alkyl or C₁₋₃alkyl-NH-CO-Het²⁰-; in particular Y represents -C₃₋₉alkyl-, -C₁₋₅alkyl-NR¹³-C₁₋₅alkyl-, -C₁₋₅alkyl-NR¹⁴-CO-C₁₋₅alkyl-, -C₁₋₆alkyl-CO-NH-, -C₁₋₆alkyl-NH-CO-, -C₁₋₂alkyl-NR²³-CO-CR¹⁶R¹⁷-NH- or C₁₋₃alkyl-NH-CO-Het²⁰-
X¹ represents O, -o-C₁₋₂alkyl-, NR¹¹, or -NR¹¹-C₁₋₂alkyl-;
X² represents a direct bond, -C₁₋₂alkyl-, CO-C₁₋₂alkyl or NR¹²-C₁₋₂alkyl-; in particular X² represents a direct bond, -C₁₋₂alkyl- or NR¹²-C₁₋₂alkyl-;
R¹ represents hydrogen, cyano, halo or hydroxy;
R² represents hydrogen, halo, cyano, C₂₋₆alkynyl, hydroxy, hydroxycarbonyl, C₁₋₄alkyloxycarbonyl- or Het¹; in particular R² represents hydrogen, halo, cyano, acetylene (-C≡CH), hydroxy, hydroxycarbonyl, C₁₋₄alkyloxycarbonyl- or Het¹; more in particular R² represents hydrogen, halo, cyano, hydroxy, hydroxycarbonyl, C₁₋₄alkyloxycarbonyl- or Het¹
R³ represents hydrogen, cyano, halo, hydroxy, formyl, C₁₋₆alkyloxy or C₁₋₆alkyloxysubstituted with halo;
R⁴ represents Ar⁴-C₁₋₄alkyloxy, C₁₋₄alkyloxy-, or C₁₋₄alkyloxy- substituted with one or where possible two or more substituents selected from hydroxy, C₁₋₄alkyloxy-, C₁₋₄alkyloxy-C₁₋₄alkyloxy, NR⁷R⁸ or Het²; in particular R⁴ represents Ar⁴-C₁₋₄alkyloxy, C₁₋₄alkyloxy-, or C₁₋₄alkyloxy- substituted with one or where possible two or more substituents selected from C₁₋₄alkyloxy-, C₁₋₄alkyloxy-C₁₋₄alkyloxy or NR⁷R⁸
R⁷ represents hydrogen, hydroxyC₁₋₄alkyl- or C₁₋₄alkyl;
R⁸ represents C₁₋₄alkyloxycarbonyl or C₁₋₄alkyl- substituted with one or more substituents selected from C₁₋₄alkylsulfonyl-, C₁₋₄alkylcarbonyloxy or NR²⁵R²⁶ in particular R⁸ represents C₁₋₄alkyl- substituted with one or more substituents selected from C₁₋₄alkylsulfonyl- or NR²⁵R²⁶;
R¹¹ represents hydrogen, C₁₋₄alkyloxycarbonyl or C₁₋₄alkyl; in particular R¹¹ represents hydrogen or C₁₋₄alkyl;
R¹² represents hydrogen or C₁₋₄alkyl;
R¹³ represents C₁₋₆alkyloxycarbonyl optionally substituted with phenyl or R¹³ represents Ar⁶-sulfonyl or Het²⁴-C₁₋₄alkylcarbonyl;
R¹⁴ and R¹⁵ each independently represent hydrogen or C₁₋₄alkyl; in particular R¹⁴ and R¹⁵ each independently represent hydrogen;
R¹⁶ and R¹⁷ each independently represent hydrogen or C₁₋₄alkyl optionally substituted with C₃₋₆cycloalkyl or R¹⁶ and R¹⁷ taken together with the carbon atom to which they are attached form a C₃₋₆cycloalkyl; in a particular embodiment R¹⁶ and R¹⁷ taken together with the carbon atom to which they are attached form a C₃₋₆cycloalkyl;
R²¹ represents hydrogen;
R²³ represents C₁₋₄alkyl optionally substituted with hydroxy-, C₁₋₄alkyloxy- or Het²⁵; R²³ may also represent hydrogen when R¹⁶ and R¹⁷ taken together with the carbon atom to which they are attached form a C₃₋₆cycloalkyl;
R²⁵ and R²⁶ each independently represent hydrogen, C₁₋₄alkyl, C₁₋₄alkylsulfonyl, C₁₋₄alkyloxycarbonyl or C₁₋₄alkylcarbonyl; in particular R²⁵ and R²⁶ each independently represents hydrogen or C₁₋₄alkylcarbonyl;
R²⁷ and R²⁸ each independently represent hydrogen, C₁₋₄alkyl, C₁₋₄alkylsulfonyl, C₁₋₄alkyloxycarbonyl or C₁₋₄alkylcarbonyl; in particular R²⁷ and R²⁸ each independently represent hydrogen or C₁₋₄alkylcarbonyl;
Het¹ represents 2-bora-1,3-dioxolanyl- optionally substituted with one or where possible two, three, four or more substituents selected from amino, C₁₋₄alkyl, hydroxy-C₁₋₄alkyl-, phenyl, phenyl-C₁₋₄alkyl, C₁₋₄alkyloxyC₁₋₄alkyl-, mono- or di(C₁₋₄alkyl)amino- or aminocarbonyl-;
Het² represents 1,1-dioxothiomorpholinyl optionally substituted with C₁₋₄alkyloxycarbonyl or C₁₋₄alkyl-NR²⁷R²⁸; or Het² represents piperidinyl or piperazinyl substituted with C₁₋₄alkyloxycarbonyl or -C₁₋₄alkyl-NR²⁷R²⁸;
Het ²⁰ represents pyrrolidinyl, 2-pyrrolidinonyl, piperidinyl or hydroxy-pyrrolidinyl; in particular Het²⁰ represents pyrrolidinyl, piperidinyl or hydroxy-pyrrolidinyl; more in particular Het²⁰ represents pyrrolidinyl;
Het²⁵ represents a heterocycle selected from morpholinyl or piperazinyl wherein said heterocycle is optionally substituted with C₁₋₄alkyl, hydroxy-C₁₋₄alkyl, C₁₋₄alkyloxy-C₁₋₄alkyl or polyhydroxy-C₁₋₄alkyl; or
Ar⁴, Ar⁵ or Ar⁶ each independently represents phenyl optionally substituted with nitro, cyano, hydroxy, hydroxyC₁₋₄alkyl, C₁₋₄alkyl or C₁₋₄alkyloxy;
further characterised in that either
Y represents -C₁₋₂alkyl-NR²³-CO-CR¹⁶R¹⁷-NH-; or
R⁴ represents C₁₋₄alkyloxy substituted with at least one substituent selected from C₁₋₄alkyloxy-C₁₋₄alkyloxy-, NR⁷R⁸ or Het².

Another group of compounds according to the present invention consists of those compounds of formula (I) wherein one or more of the following restrictions apply;
Z represents NH;
Y represents -C₃₋₉alkyl-, -C₁₋₅alkyl-NR¹³-C₁₋₅alkyl-, -C₁₋₅alkyl-NR¹⁴-CO-C₁₋₅alkyl-, -C₁₋₆alkyl-CO-NH-, -C₁₋₆alkyl-NH-CO-, -C₁₋₂alkyl-NR²³-CO-CR¹⁶R¹⁷-NH-, -C₁₋₂alkyl-NR²¹-CH₂-CO-NH-C₁₋₃alkyl or C₁₋₃alkyl-NH-CO-Het²⁰-; in particular Y represents -C₃₋₉alkyl-, -C₁₋₅alkyl-NR¹³-C₁₋₅alkyl-, -C₁₋₅alkyl-NR¹⁴-CO-C₁₋₅alkyl-, -C₁₋₆alkyl-CO-NH-, -C₁₋₆alkyl-NH-CO-, -C₁₋₂alkyl-NR²³-CO-CR¹⁶R¹⁷-NH- or C₁₋₃alkyl-NH-CO-Het²⁰-
X¹ represents O, -O-C₁₋₂alkyl-, NR¹¹, or -NR¹¹-C₁₋₂alkyl-;
X² represents a direct bond, -C₁₋₂alkyl-, CO-C₁₋₂alkyl or NR¹²-C₁₋₂alkyl-; in particular X² represents a direct bond, -C₁₋₂alkyl- or NR¹²-C₁₋₂alkyl-;
R¹ represents hydrogen or halo;
R² represents hydrogen, halo, C₂₋₆alkynyl, cyano or Het¹; in particular R² represents hydrogen, halo, C₂₋₆alkynyl or Het¹; more in particular R² represents hydrogen, halo, acetylene or Het¹; or R² represents hydrogen, halo, cyano or Het¹;
R³ represents hydrogen;
R⁴ represents Ar⁴-C₁₋₄alkyloxy, C₁₋₄alkyloxy-, or C₁₋₄alkyloxy- substituted with one or where possible two or more substituents selected from hydroxy, C₁₋₄alkyloxy-, C₁₋₄alkyloxy-C₁₋₄alkyloxy, NR⁷R⁸ or Het²; in particular R⁴ represents Ar⁴-C₁₋₄alkyloxy, C₁₋₄alkyloxy-, or C₁₋₄alkyloxy- substituted with one or where possible two or more substituents selected from C₁₋₄alkyloxy-, C₁₋₄alkyloxy-C₁₋₄alkyloxy or NR⁷R⁸
R⁷ represents hydrogen or C₁₋₄alkyl;
R⁸ represents C₁₋₄alkyloxycarbonyl or C₁₋₄alkyl- substituted with one or more substituents selected from C₁₋₄alkylsulfonyl-, C₁₋₄alkylcarbonyloxy or NR²⁵R²⁶; in particular R⁸ represents C₁₋₄alkyl- substituted with one or more substituents selected from C_{I}-4alkylsulfonyl- or NR²⁵R²⁶;
R¹¹ represents hydrogen or C₁₋₄alkyl;
R¹² represents hydrogen or C₁₋₄alkyl;
R¹³ represents Ar⁶-sulfonyl or C₁₋₆alkyloxycarbonyl optionally substituted with phenyl;
R¹⁴ and R¹⁵ represent hydrogen;
R¹⁶ and R¹⁷ each independently represent hydrogen or C₁₋₄alkyl optionally substituted with C₃₋₆cycloalkyl or R¹⁶ and R¹⁷ taken together with the carbon atom to which they are attached form a C₃₋₆cycloalkyl; in a particular embodiment R¹⁶ and R¹⁷ taken together with the carbon atom to which they are attached form a C₃₋₆cycloalkyl;
R²¹ represents hydrogen;
R²³ represents C₁₋₄alkyl optionally substituted with hydroxy-, C₁₋₄alkyloxy- or Het²⁵; R²³ may also represent hydrogen when R¹⁶ and R¹⁷ taken together with the carbon atom to which they are attached form a C₃₋₆cycloalkyl;
R²⁵ and R²⁶ each independently represent hydrogen or C₁₋₄alkylcarbonyl;
R²⁷ and R²⁸ each independently represent hydrogen or C₁₋₄alkylcarbonyl;
Het¹ represents 2-bora-1,3-dioxolanyl-;
Het² represents 1,1-dioxothiomorpholinyl, piperidinyl or piperazinyl wherein said Het² is optionally substituted with C₁₋₄alkyloxycarbonyl or -C₁₋₄alkyl-NR²⁵R²⁸;
Het²⁰ represents pyrrolidinyl;
Het²⁵ represents a heterocycle selected from morpholinyl or piperazinyl wherein said heterocycle is optionally substituted with C₁₋₄alkyl, hydroxy-C₁₋₄alkyl, C₁₋₄alkyloxy-C₁₋₄alkyl or polyhydroxy-C₁₋₄alkyl;
Ar⁴ represents phenyl;
Ar⁵ represents phenyl; or
Ar⁶ represents phenyl optionally substituted with nitro;
further characterised in that either
Y represents -C₁₋₂alkyl-NR²³-CO-CR¹⁶R¹⁷-NH-; or
R⁴ represents C₁₋₄alkyloxy substituted with at least one substituent selected from C₁₋₄alkyloxy-C₁₋₄alkyloxy-, NR⁷R⁸ or Het²; in particular C₁₋₄alkyloxy substituted with C₁₋₄alkyloxy-C₁₋₄alkyloxy- or NR⁷R⁸.

An interesting group of compounds consists of those compounds of formula (I) wherein one or more of the following restrictions apply :
Z represents NH;
Y represents -C₃₋₉alkyl-, -C₁₋₅alkyl-NR¹³-C₁₋₅alkyl-, -C₁₋₅alkyl-NR¹⁴-CO-C₁₋₅alkyl-, -C₁₋₂alkyl-NR²¹-CH₂-CO-NH-C₁₋₃alkyl- or -C₁₋₂alkyl-NR²³-CO-CR¹⁶R¹⁷-NH-; in particular Y represents -C₃₋₉alkyl-, -C₁₋₅alkyl-NR¹³-C₁₋₅alkyl- or -C₁₋₂alkyl-NR²³-CO-CR¹⁶R¹⁷-NH-
X¹ represents O or -O-C₁₋₂alkyl-; in particular X¹ represents O;
X² represents a direct bond, C₁₋₂alkyl, -CO-C₁₋₂alkyl or NR¹²-C₁₋₂alkyl; in particular X² represents a direct bond or NR¹²-C₁₋₂alkyl-;
R¹ represents hydrogen or halo; in particular R¹ represents hydrogen;
R² represents halo, C₂₋₆alkynyl, cyano or Het¹; in particular R² represents halo, acetylene or Het¹; more in particular R² represents halo or Het¹;
R³ represents hydrogen;
R⁴ represents Ar⁴-C₁₋₄alkyloxy-, C₁₋₄alkyloxy- or C₁₋₄alkyloxy substituted with one or where possible two or more substituents selected from Het², NR⁷R⁸, hydroxy and C₁₋₄alkyloxy-C₁₋₄alkyloxy-; in particular R⁴ represents Ar⁴-C₁₋₄alkyloxy-, C₁₋₄alkyloxy- or C₁₋₄alkyloxy substituted with C₁₋₄alkyloxy-C₁₋₄alkyloxy-;
R⁷ represents hydrogen or C₁₋₄alkyl;
R⁸ represents C₁₋₄alkyl substituted with NR²⁵R²⁶ or C₁₋₄alkylsulfonyl;
R¹² represents hydrogen or C₁₋₄alkyl-;
R¹³ represents Ar⁶-sulfonyl or C₁₋₆alkyloxycarbonyl optionally substituted with phenyl;
R¹⁶ and R¹⁷ represents hydrogen, C₁₋₄alkyl or R¹⁶ and R¹⁷ taken together with the carbon atom to which they are attached from a C₃₋₆cycloalkyl;
R²³ represents hydrogen or C₁₋₄alkyl; in particular R²³ represents C₁₋₄alkyl and R²³ represents hydrogen when R¹⁶ and R¹⁷ taken together with the carbon atom to which they are attached from a C₃₋₆cycloalkyl;
R²⁵ and R²⁶ each independently represent hydrogen or C₁₋₄alkylcarbonyl;
R²⁷ and R²⁸ each independently represent hydrogen or C₁₋₄alkylcarbonyl;
Het¹ represents 2-bora-1,3-dioxolanyl;
Het² represents piperidinyl, piperazinyl, morpholinyl, thiomorpholinyl or 1,1-dioxothiomorpholinyl wherein said Het² is optionally substituted with C₁₋₄alkyloxycarbonyl or NR²⁷R²⁸-C₁₋₄alkyl; in particular Het² represents 1,1-dioxothiomorpholinyl; piperidinyl substituted with C₁₋₄alkyloxycarbonyl; or piperazinyl substituted with C₁₋₄alkyloxycarbonyl or NR²⁷R²⁸-C₁₋₄alkyl-;
Ar⁴ represents phenyl;
Ar⁵ represents phenyl; or
Ar⁶ represents phenyl optionally substituted with nitro.

An interesting group of compounds consists of those compounds of formula (I) wherein one or more of the following restrictions apply :
Z represents NH;
Y represents -C₃₋₉alkyl-, -C₁₋₅alkyl-NR¹³-C₁₋₅alkyl-, -C₁₋₅alkyl-NR¹⁴-CO-C₁₋₅alkyl-, -C₁₋₂alkyl-NR²¹-CH₂-CO-NH-C₁₋₃alkyl-, C₁₋₆alkyl-NH-CO- or -C₁₋₂alkyl-NR²³-CO-CR¹⁶R¹⁷-NH-; in particular Y represents -C₃₋₉alkyl-, C₁₋₆alkyl-NH-CO--C₁₋₅alkyl-NR¹³-C₁₋₅alkyl-, -C₁₋₅alkyl-NR¹⁴-CO-C₁₋₅alkyl-, or -C₁₋₂alkyl-NR²³-CO-CR¹⁶R¹⁷-NH-
X¹ represents O or -O-C₁₋₂alkyl-; in particular X¹ represents O;
X² represents a direct bond, C₁₋₂alkyl, -CO-C₁₋₂alkyl or NR¹²-C₁₋₂alkyl; in particular X² represents -CO-C₁₋₂alkyl or NR¹²-C₁₋₂alkyl-;
R¹ represents hydrogen, cyano or halo; in particular R¹ represents hydrogen or halo, more in particular R¹ represents hydrogen, fluoro or bromo;
R² represents halo, C₂₋₆alkynyl, cyano or Het¹; in particular R² represents halo, acetylene or Het¹; more in particular R² represents halo or Het¹;
R³ represents hydrogen;
R⁴ represents Ar⁴-C₁₋₄alkyloxy-, C₁₋₄alkyloxy- or C₁₋₄alkyloxy substituted with one or where possible two or more substituents selected from Het², NR⁷R⁸, hydroxy and C₁₋₄alkyloxy-C₁₋₄alkyloxy-; in particular R⁴ represents Ar⁴-C₁₋₄alkyloxy-, C₁₋₄alkyloxy- or C₁₋₄alkyloxy substituted with one or where possible two or more substituents selected from Het², NR⁷R⁸ or hydroxy;
R⁷ represents hydrogen, hydroxy-C₁₋₄alkyl- or C₁₋₄alkyl;
R⁸ represents C₁₋₄alkylcarbonyl, C₁₋₄alkyloxycarbonyl or C₁₋₄alkyl substituted with hydroxy-C₁₋₄alkyloxy-, NR²⁵R²⁶, C₁₋₄alkylcarbonyloxy- or C₁₋₄alkylsulfonyl;
R¹² represents hydrogen or C₁₋₄alkyl-;
R¹³ represents Ar⁶-sulfonyl or C₁₋₆alkyloxycarbonyl optionally substituted with phenyl;
R¹⁶ and R¹⁷ each independently represents hydrogen, C₁₋₄alkyl or R¹⁶ and R¹⁷ taken together with the carbon atom to which they are attached from a C₃₋₆cycloalkyl;
R²³ represents C₁₋₄alkyl optionally substituted with Het²⁵;
   R²³ may also represent hydrogen when R¹⁶ and R¹⁷ taken together with the carbon atom to which they are attached form a C₃₋₆cycloalkyl;
R²⁵ and R²⁶ each independently represent hydrogen or C₁₋₄alkylcarbonyl;
R²⁷ and R²⁸ each independently represent hydrogen or C₁₋₄alkylcarbonyl;
Het¹ represents 2-bora-1,3-dioxolanyl;
Het² represents piperidinyl, piperazinyl, morpholinyl, thiomorpholinyl or 1,1-dioxothiomorpholinyl wherein said Het² is optionally substituted with C₁₋₄alkyloxycarbonyl or NR²⁷R²⁸-C₁₋₄alkyl; in particular Het² represents 1,1-dioxothiomorpholinyl; piperidinyl substituted with C₁₋₄alkyloxycarbonyl; or piperazinyl substituted with C₁₋₄alkyloxycarbonyl or NR²⁷R²⁸-C₁₋₄alkyl-;
Het²⁵ represents morpholinyl;
Ar⁴ represents phenyl;
Ar⁵ represents phenyl; or
Ar⁶ represents phenyl optionally substituted with nitro.

A further interesting group of compounds consists of those compounds of formula (I) wherein one or more of the following restrictions apply :
Z represents NH; Y represents -C₃₋₉alkyl-,-C₁₋₅alkyl-NR¹³-C₁₋₅alkyl-, -C₁₋₅alkyl-NR¹⁴-CO-C₁₋₅alkyl-, -C₁₋₂alkyl-NR²¹-CH₂-CO-NH-C₁₋₃alkyl- or -C₁₋₂alkyl-NR²³-CO-CR¹⁶R¹⁷₋NH-;
X¹ represents O or -O-C₁₋₂alkyl-; X² represents a direct bond, C₁₋₂alkyl, -CO-C₁₋₂alkyl or NR¹²-C₁₋₂alkyl;
R¹ represents hydrogen or halo; R² represents halo, acetylene or Het¹
R³ represents hydrogen or cyano; R⁴ represents Ar⁴-C₁₋₄alkyloxy-, C₁₋₄alkyloxy- or C₁₋₄alkyloxy substituted with one or where possible two or more substituents selected from Het², NR⁷R⁸, hydroxy and C₁₋₄alkyloxy-C₁₋₄alkyloxy-;
R⁷ represents hydrogen or C₁₋₄alkyl; R⁸ represents C₁₋₄alkyl substituted with NR²⁵R²⁶ or C₁₋₄alkylsulfonyl;
R¹² represents hydrogen or C₁₋₄alkyl-; R¹³ represents Ar⁶-sulfonyl or C₁₋₆alkyloxycarbonyl optionally substituted with phenyl;
R¹⁶ and R¹⁷ represents hydrogen, C₁₋₄alkyl or R¹⁶ and R¹⁷ taken together with the carbon atom to which they are attached from a C₃₋₆cycloalkyl;
R²³ represents C₁₋₄alkyl and R²³ represents hydrogen when R¹⁶ and R¹⁷ taken together with the carbon atom to which they are attached from a C₃₋₆cycloalkyl;
R²⁵, R²⁶, R²⁷ and R²⁸ each independently represent hydrogen or C₁₋₄alkylcarbonyl;
Het¹ represents 2-bora-1,3-dioxolanyl; Het² represents piperidinyl, piperazinyl, morpholinyl, thiomorpholinyl or 1,1-dioxothiomorpholinyl wherein said Het² is optionally substituted with C₁₋₄alkyloxycarbonyl or NR²⁷R²⁸-C₁₋₄alkyl;
Ar⁴ and Ar⁵ represents phenyl; Ar⁶ represents phenyl optionally substituted with nitro.

Other special group of compounds are:
- those compounds of formula (I) wherein -X¹- represents -O-;
- those compounds of formula (I) wherein -X¹- represents C₁₋₂alkyl;
- those compounds of formula (I) wherein -X¹- represents -NH¹¹-, in particular NH-;
- those compounds of formula (I) wherein -X²- represents -NR¹²-C₁₋₂alkyl, in particular -N(CH₃)-C₁₋₂alkyl-;
- those compounds of formula (I) wherein R¹ is fluoro, chloro or bromo;
- those compounds of formula (I) wherein R² is fluoro, chloro or bromo;
- those compounds of formula (I) wherein R² is Het¹, in particular 2-bora-1,3-dioxolanyl;
- those compounds of formula (I) wherein R⁴ is at position 7 of the structure of formula (I).
- those compounds of formula (I) wherein R⁴ represents C₁₋₄alkyloxy substituted with hydroxy and one substituent selected from NR⁷R⁸ or Het²-;
- those compounds of formula (I) wherein R⁷ is hydrogen or methyl and R⁸ represents aminocarbonyl-C₁₋₄alkyl-, NR²⁵R²⁶, C₁₋₄alkylsulfonyl-C₁₋₄alkyl-, C₁₋₄alkylcarbonyloxy-C₁₋₄alkyl or Het¹¹-C₁₋₄alkyl-; in particular those compounds of formula (I) wherein R⁷ is hydrogen or methyl and R⁸ represents aminocarbonyl-C₁₋₄alkyl-, NR²⁵R²⁶, C₁₋₄alkylsulfonyl-C₁₋₄atkyl- or Het¹¹-C₁₋₄alkyl-
- those compounds of formula (I) wherein Het² represent piperidinyl, 1,1-dioxothiomorpholinyl or piperazinyl and said Het² is optionally substituted with one or where possible two or more substituents selected from NR³⁹R⁴⁰, aminocarbonyl, mono- or di(C₁₋₄alkyl)aminocarbonyl or C₁₋₄alkylsulfonyl; in particular those compounds of formula (I) wherein Het² represent piperidinyl or piperazinyl and said Het² is optionally substituted with one or where possible two or more substituents selected from NR³⁹R⁴⁰, aminocarbonyl, mono- or di(C₁₋₄alkyl)aminocarbonyl or C₁₋₄alkylsulfonyl.

In a further embodiment of the present invention the X² substituent is at position 2', the R¹ substituent represents hydrogen or halo and is at position 4', the R² substituent represents halo and is at position 5', the R³ substituent is at position 2 and the R⁴ substituent at position 7 of the structure of formula (I). Alternatively, the X² substituent is at position 3', the R¹ substituent represents hydrogen or halo and is at position 4', the R² substituent represents halo and is at position 5', the R³ substituent is at position 2 and the R⁴ substituent at position 7 of the structure of formula (I).

The compounds of this invention can be prepared by any of several standard synthetic processes commonly used by those skilled in the art of organic chemistry and described for instance in the following references; "Heterocyclic Compounds" - Vol.24 (part4) p 261-304 Fused pyrimidines, Wiley - Interscience ; Chem. Pharm. Bull., Vol 41(2) 362-368 (1993); J.Chem.Soc., Perkin Trans. 1, 2001, 130-137.

As further exemplified in the experimental part of the description, a particular group of compounds are those compounds of formula (I) were -X¹- represents -O- hereinafter referred to as the compounds of formula (3). Said compounds are generally prepared starting from the known 6-acetoxy-4-chloro-7-methoxy quinazoline (II') which can be prepared from commercially available veratric acid and 4-hydroxy-3-methoxy benzoic acid, respectively.

Coupling of the latter with suitable substituted anilines (III') under standard conditions, for example stirred in 2-propanol at an elevated temperature ranging form 40-100°C during 3-12 h, furnish the intermediate compounds (IV') (Scheme 1). 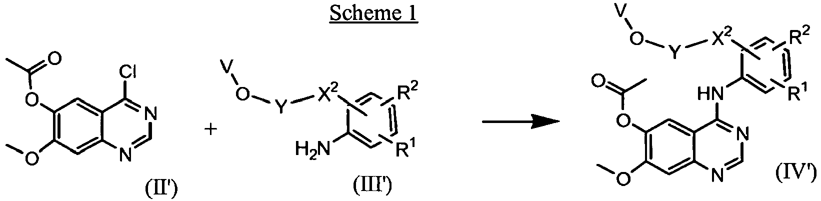 V = hydrogen or a protective group such as for example, methylcarbonyl, t-butyl, methyl, ethyl, benzyl or trialkylsilyl groups
X², R¹ and R² are defined as for the compounds of formula (I)

Deprotection of the intermediates of formula (IV') as described in Protective Groups in Organic Synthesis by T. W. Greene and P. G.M. Wuts, 3rd edition, 1998 followed by ring closure under Mitsunobu conditions give the macrocyclic compounds (1) that are used as starting compounds in the synthesis of the final compounds of the present invention. (Scheme 2 - wherein V is defined as hereinbefore). 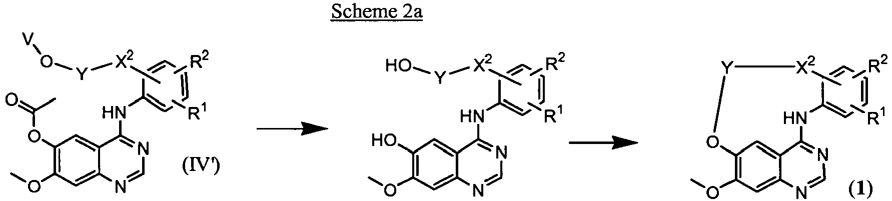 V = hydrogen or a protective group such as for example, methylcarbonyl, t-butyl, methyl, ethyl, benzyl or trialkylsilyl groups
X², R¹ and R² are defined as for the compounds of formula (I)

In brief, said macrocyclic compounds of formula (**1**) are demethylated using art known conditions such as for example provided in Schemes 3&4 hereinbelow, followed by an alkylation with an appropriate alcohol, such as for example described in Scheme 5 hereinafter.

### Quinazoline demethylation. Scheme 3:

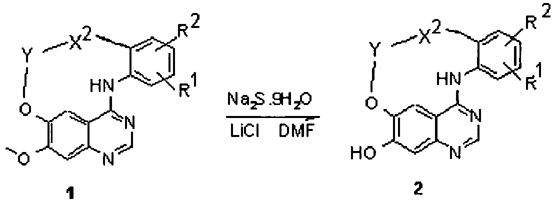

A stirred suspension of **1** (1 equiv), LiCl (7 equiv.) and Na₂S.9H₂O (7 equiv) in DMF, was heated under microwave conditions to 140° C until completion (30 minutes). The reaction mixture was allowed to cool to ambient temperature and was then poured onto ice water. The mixture was filtered and the yellow precipitation was re-dissolved in DCM/MeOH (9:1) with some HCOOH and purified over silica gel filter (eluens: DCM/MeOH 9.5/0.5). The pure fractions were collected, evaporated and co-evaporated with toluene to give pure 2 (yield: 70 %).

### Quinazoline demethylation. Scheme 4:

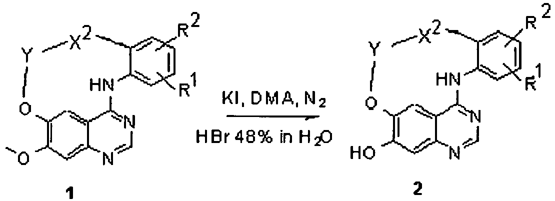

To a stirred suspension of **1** (1 equiv) and KI (10 equiv) in DMA, was added HBr (48 % in H₂O) while bubbling N₂ through the reaction mixture. The mixture was rapidly heated to 130° C and stirred at this temperature until completion (± 2h). The reaction mixture was allowed to cool to 70° C and poured onto ice/H₂O/NH₃. The mixture was filtered and the yellow precipitation was re-dissolved in THF/MeOH (2:1), concentrated and co-evaporated with toluene. Crystalization from 2-propanol afford pure **2** (yield : 42 - 78 %).

### Quinazoline alkylation. Scheme 5

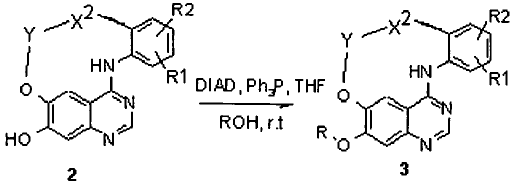

wherein R represents Ar⁴-C₁₋₄alkyl-, C₁₋₄alkyl- or R represents C₁₋₄alkyl substituted with one or where possible two or more substituents selected from hydroxy, halo, C₁₋₄alkyloxy-, C₁₋₄alkyloxy-C₁₋₄alkyloxy-, NR⁷R⁸ or Het²-. Ar⁴, Het², R⁷ and R⁸ are defined as for the compounds of formula (I) hereinbefore.

To a stirred suspension of **2** (1 equiv), alcohol (8 equiv) and triphenylphosphine (2 equiv) in THF, DIAD (2 equiv) was added dropwise and the mixture was stirred at room temperature for 60 min. The reaction mixture was concentrated under reduced pressure, and the crude product was triturated from acetonitrile to afford pure **3**.

For those compounds of formula (**3**) wherein R¹ or R² represent acetylene the following synthesis scheme (Scheme 6) is generally applied. In brief, the halogenated form of the compounds of formula (**3**) is acetylated using trimethylsilylacetylene followed by deprotection of the acetylene group to yield the compounds of general formula (**5**).

### Incorporation of acetylene moiety. Scheme 6

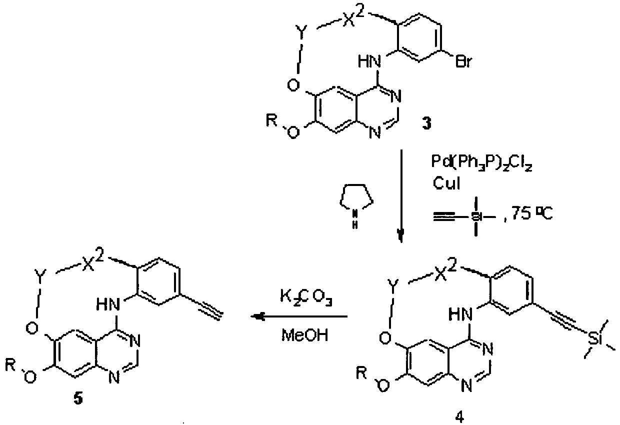

wherein R is defined as in Scheme 5 hereinbefore

To a stirred solution of **3** (1 equiv) in pyrrolidine was added bis(triphenylphosphine)palladium(II)chloride (20 mol%) followed by CuI (cat). The reaction mixture was heated to 75°C and trimethylsilylacetylene (2.5 equiv) was added. The mixture was stirred at this temperature until the reaction was essentially complete and was then filtered through a short pad of celite and concentrated to dryness. The residue was re-dissolved in EtOAc and was partitioned between EtOAc and water. The combined organic layers were concentrated under reduced pressure and the residue was treated with MP-TMT in acetonitrile overnight. It was then filtered, the resin was washed with acetonitrile followed by DCM and the filtrate was concentrated to afford **4**.

Compound **4** and aqueous K₂CO₃ (sat.) in MeOH (1:1) were stirred at room temperature for 1 hour. The reaction mixture was concentrated under reduced pressure, the residue was re-dissolved in DCM and washed with water. The organic phase was separated, dried (MgSO₄) and concentrated under vaccuo. The residue was purified either by column chromatography or reverse phase HPLC to afford pure **5**.

A particular group of compounds are those compounds of formula (**3**) wherein R represents C₁₋₄alkyl substituted with NR⁷R⁸ or Het² wherein said Het² is attached to the remainder of the molecule through the nitrogen atom. Said compounds of general formula (**7**) are generally made according to synthesis scheme 7 departing from the intermediate compounds of general formula (**2**). 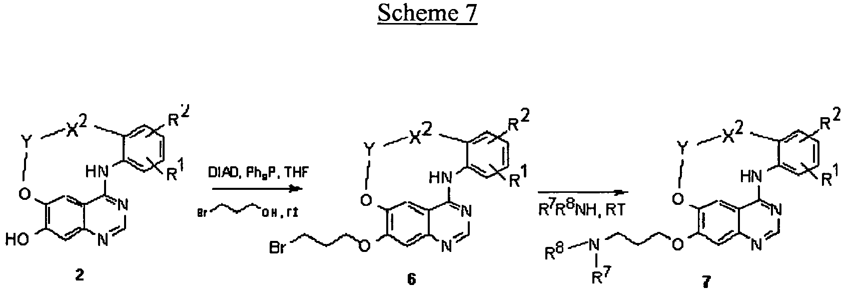 wherein R⁷ and R⁸ are defined as for the compounds of formula (I), or R⁷ and R⁸ taken together with the nitrogen atom to which they are attached from a heterocycle wherein said heterocycle is defined as Het² for the compounds of formula (I) hereinbefore.

To a stirred suspension of **2** (1 equiv), bromopropyl alcohol (2 equiv) and triphenylphosphine (2 equiv) in THF, DIAD (2 equiv) was added dropwise and the mixture was stirred at room temperature for 60 min. The reaction mixture was concentrated under vaccuo, and the crude product was triturated from acetonitrile to afford pure **6**.

To a stirred suspension of **6** (1 equiv), in acetonitrile was added the amine (20 equiv) and the mixture was stirred at room temperature overnight. The reaction mixture was concentrated under vaccuo, and the crude product was triturated from acetonitrile to afford pure **7**.

Alternatively to the above, and in particular for those compounds of formula (**7**) wherein the C₁₋₄alkyl moiety is further substituted with hydroxy-, said compounds are made using a nucleophilic addition reaction departing from the oxirane analog 3' (Scheme 8)

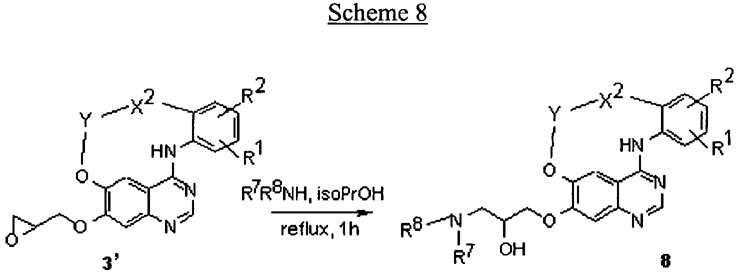

Wherein R⁷ and R⁸ are defined as for the compounds of formula (I), or R⁷ and R⁸ taken together with the nitrogen atom to which they are attached from a heterocycle wherein said heterocycle is defined as Het² for the compounds of formula (I) hereinbefore.

To a stirred suspension of 3' (1 equiv), in 2-propanol was added the amine (20 equiv) and the mixture was stirred at 70° C for 2 hours. The reaction mixture was cooled, and the product crystallized from 2-propanol to afford pure **8**.

Where necessary or desired, any one or more of the following further steps in any order may be performed :
(i) removing any remaining protecting group(s);
(ii) converting a compound of formula (I) or a protected form thereof into a further compound of formula (I) or a protected form thereof;
(iii) converting a compound of formula (I) or a protected form thereof into a *N*-oxide, a salt, a quaternary amine or a solvate of a compound of formula (I) or a protected form thereof;
(iv) converting a *N*-oxide, a salt, a quaternary amine or a solvate of a compound of formula (I) or a protected form thereof into a compound of formula (I) or a protected form thereof;
(v) converting a *N*-oxide, a salt, a quaternary amine or a solvate of a compound of formula (I) or a protected form thereof into another *N*-oxide, a pharmaceutically acceptable addition salt a quaternary amine or a solvate of a compound of formula (I) or a protected form thereof;
(vi) where the compound of formula (I) is obtained as a mixture of (R) and (S) enantiomers resolving the mixture to obtain the desired enantiomer.

Compounds of formula (I), *N*-oxides, addition salts, quaternary amines and stereochemical isomeric forms thereof can be converted into further compounds according to the invention using procedures known in the art.

It will be appreciated by those skilled in the art that in the processes described above the functional groups of intermediate compounds may need to be blocked by protecting groups.

Functional groups, which are desirable to protect, include hydroxy, amino and carboxylic acid. Suitable protecting groups for hydroxy include trialkylsilyl groups (e.g. tert-butyldimethylsilyl, tert-butyldiphenylsilyl or trimethylsilyl), benzyl and tetrahydropyranyl. Suitable protecting groups for amino include tert-butyloxycarbonyl or benzyloxycarbonyl. Suitable protecting groups for carboxylic acid include C₍₁₋₆₎alkyl or benzyl esters.

The protection and deprotection of functional groups may take place before or after a reaction step.

Additionally, the N-atoms in compounds of formula (I) can be methylated by art-known methods using CH₃-I in a suitable solvent such as, for example 2-propanone, tetrahydrofuran or dimethylformamide.

The compounds of formula (I) can also be converted into each other following art-known procedures of functional group transformation of which some examples are mentioned hereinafter.

The compounds of formula (I) may also be converted to the corresponding *N*-oxide forms following art-known procedures for converting a trivalent nitrogen into its *N*-oxide form. Said *N*-oxidation reaction may generally be carried out by reacting the starting material of formula (I) with 3-phenyl-2-(phenylsulfonyl)oxaziridine or with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. t-butyl hydroperoxide. Suitable solvents are, for example, water, lower alkanols, e.g. ethanol and the like, hydrocarbons, e.g. toluene, ketones, e.g. 2-butanone, halogenated hydrocarbons, e.g. dichloromethane, and mixtures of such solvents.

Pure stereochemically isomeric forms of the compounds of formula (I) may be obtained by the application of art-known procedures. Diastereomers may be separated by physical methods such as fractional crystallization and chromatographic techniques, e.g. counter-current distribution, liquid chromatography and the like.

Some of the compounds of formula (I) and some of the intermediates in the present invention may contain an asymmetric carbon atom. Pure stereochemically isomeric forms of said compounds and said intermediates can be obtained by the application of art-known procedures. For example, diastereoisomers can be separated by physical methods such as fractional crystallization or chromatographic techniques, e.g. counter current distribution, liquid chromatography and the like methods. Enantiomers can be obtained from racemic mixtures by first converting said racemic mixtures with suitable resolving agents such as, for example, chiral acids, to mixtures of diastereomeric salts or compounds; then physically separating said mixtures of diastereomeric salts or compounds by, for example, fractional rystallization or chromatographic techniques, e.g. liquid chromatography and the like methods; and finally converting said separated diastereomeric salts or compounds into the corresponding enantiomers. Pure stereochemically isomeric forms may also be obtained from the pure stereochemically isomeric forms of the appropriate intermediates and starting materials, provided that the intervening reactions occur stereospecifically.

An alternative manner of separating the enantiomeric forms of the compounds of formula (I) and intermediates involves liquid chromatography, in particular liquid chromatography using a chiral stationary phase.

Some of the intermediates and starting materials as used in the reaction procedures mentioned hereinabove are known compounds and may be commercially available or may be prepared according to art-known procedures.

As described in the experimental part hereinafter, the growth inhibitory effect and antitumour activity of the present compounds has been demonstrated in vitro, in enzymatic assays on the receptor tyrosine kinases such as for example EGFR, Abl, Fyn, FlT1, HcK or the Sar kinase family such as for example Lyn, Yes and cSRC. In an alternative assay, the growth inhibitory effect of the compounds was tested on a number of carcinamo cell lines, in particular in the ovarian carcinoma cell line SKOV3 and the squamous carcinoma cell line A431 using art known cytotoxicity assays such as MTT.

Accordingly, the present invention provides the compounds of formula (I) and their pharmaceutically acceptable *N*-oxides, addition salts, quaternary amines and stereochemically isomeric forms for use in therapy. More particular in the treatment or prevention of cell proliferation mediated diseases. The compounds of formula (I) and their pharmaceutically acceptable *N*-oxides, addition salts, quaternary amines and the stereochemically isomeric forms may hereinafter be referred to as compounds according to the invention.

Disorders for which the compounds according to the invention are particularly useful are atherosclerosis, restenosis, cancer and diabetic complications e.g. retinopathy.

Compounds according to the invention, for use in a method of treating a cell proliferative disorder such as atherosclerosis, restenosis and cancer are provided.

Said method comprising the systemic or topical administration of an effective amount of a compound according to the invention, to animals, including humans. One skilled in the art will recognize that a therapeutically effective amount of the EGFR inhibitors of the present invention is the amount sufficient to induce the growth inhibitory effect and that this amount varies *inter alia*, depending on the size, the type of the neoplasia, the concentration of the compound in the therapeutic formulation, and the condition of the patient. Generally, an amount of EGFR inhibitor to be administered as a therapeutic agent for treating cell proliferative disorder such as atherosclerosis, restenosis and cancer, will be determined on a case by case by an attending physician.

Generally, a suitable dose is one that results in a concentration of the EGFR inhibitor at the treatment site in the range of 0.5 nM to 200 µM, and more usually 5 nM to 10 µM. To obtain these treatment concentrations, a patient in need of treatment likely will be administered between 0.01 mg/kg to 300 mg/kg body weight, in particular from 10 mg/kg to 100 mg/kg body weight. As noted above, the above amounts may vary on a case-by-case basis. In these methods of treatment the compounds according to the invention are preferably formulated prior to admission. As described herein below, suitable pharmaceutical formulations are prepared by known procedures using well known and readily available ingredients.

Due to their high degree of selectivity as EGFR inhibitors, the compounds of formula (I) as defined above, are also useful to mark or identify the kinase domain within the receptor tyrosine kinase receptors. To this purpose, the compounds of the present invention can be labelled, in particular by replacing, partially or completely, one or more atoms in the molecule by their radioactive isotopes. Examples of interesting labelled compounds are those compounds having at least one halo which is a radioactive isotope of iodine, bromine or fluorine; or those compounds having at least one ¹¹C-atom or tritium atom.

One particular group consists of those compounds of formula (I) wherein R¹ is a radioactive halogen atom. In principle, any compound of formula (I) containing a halogen atom is prone for radiolabelling by replacing the halogen atom by a suitable isotope. Suitable halogen radioisotopes to this purpose are radioactive iodides, e.g. ¹²²I, ¹²³I, ¹²⁵I, ¹³¹I; radioactive bromides, e.g. ⁷⁵Br, ⁷⁶Br, ⁷⁷Br and ⁸²Br, and radioactive fluorides, e.g. ¹⁸F. The introduction of a radioactive halogen atom can be performed by a suitable exchange reaction or by using any one of the procedures as described hereinabove to prepare halogen derivatives of formula (I).

Another interesting form of radiolabelling is by substituting a carbon atom by a ¹¹C-atom or the substitution of a hydrogen atom by a tritium atom.

Hence, said radiolabelled compounds of formula (I) can be used in a process of specifically marking receptor sites in biological material. Said process comprises the steps of (a) radiolabelling a compound of formula (I), (b) administering this radiolabelled compound to biological material and subsequently (c) detecting the emissions from the radiolabelled compound.

Alternatively the compounds are labeled with stable isotopes. In this form of labeling the naturally abundant isotopes of hydrogen, carbon and nitrogen (¹H, ¹²C and ¹⁴N) are replaced with stable isotopes of these elements (²H [deuterium], ¹³C and ¹⁵N, respectively). Labeling with stable isotopes is used for two principal purposes:
- Incorporation of stable isotopes into proteins, carbohydrates and nucleic acids facilitates their structural determination at the atomic level.
- Metabolic studies exploiting the increased mass of compounds labeled with stable isotopes

The term biological material is meant to comprise every kind of material which has a biological origin. More in particular this term refers to tissue samples, plasma or body fluids but also to animals, specially warm-blooded animals, or parts of animals such as organs.

When used *in in vivo* assays, the radiolabelled compounds are administered in an appropriate composition to an animal and the location of said radiolabelled compounds is detected using imaging techniques, such as, for instance, Single Photon Emission Computerized Tomography (SPECT) or Positron Emission Tomography (PET) and the like. In this manner the distribution to the particular receptor sites throughout the body can be detected and organs containing said receptor sites can be visualized by the imaging techniques mentioned hereinabove.

In yet a further aspect, the present invention provides the use of the compounds according to the invention in the manufacture of a medicament for treating any of the aforementioned cell proliferative disorders or indications.

The amount of a compound according to the present invention, also referred to here as the active ingredient, which is required to achieve a therapeutical effect will, of course, vary with the particular compound, the route of administration, the age and condition of the recipient, and the particular disorder or disease being treated. A suitable daily dose would be from 0.01 mg/kg to 300 mg/kg body weight, in particular from 10 mg/kg to 100 mg/kg body weight. A method of treatment may also include administering the active ingredient on a regimen of between one and four intakes per day.

While it is possible for the active ingredient to be administered alone, it is preferable to present it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of this invention may be prepared by any methods well known in the art of pharmacy, for example, using methods such as those described in Gennaro et al. Remington's Pharmaceutical Sciences (18th ed., Mack Publishing Company, 1990, see especially Part 8 : Pharmaceutical preparations and their Manufacture). A therapeutically effective amount of the particular compound, in base form or addition salt form, as the active ingredient is combined in intimate admixture with a pharmaceutically acceptable carrier, which may take a wide variety of forms depending on the form of preparation desired for administration. These pharmaceutical compositions are desirably in unitary dosage form suitable, preferably, for systemic administration such as oral, percutaneous or parenteral administration; or topical administration such as via inhalation, a nose spray, eye drops or via a cream, gel, shampoo or the like. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions: or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The present invention is directed only to compounds of formula (I) and the specific examples encompassed by the current set of claims. All the other structures are reference embodiments.

### Experimental part

Hereinafter, the term 'THF' means tetrahydrofuran, 'DIPE' means diisopropyl ether, 'DMF' means *N*,*N*-dimethylformamide, 'NaBH(OAc)₃' means sodium triacetoxyborohydride, 'EtOAc' means ethyl acetate, 'EDCI' means *N*'-(ethylcarbonimidoyl)-*N*,*N*-dimethyl-1,3-propanediamine monohydrochloride, 'HOBT' means 1-hydroxy-1H-benzotriazole, 'CDI' means 1,1'-carbonylbis-1H-imidazole, 'DIPEA' means *N*-ethyl-*N*-(1-methylethyl)- 2-propanamine, 'NaBH₄' means sodium tetrahydroborate(-1), 'DMA' means dimethylacetamide, 'DIAD' means bis(1-methylethyl) ester diazenedicarboxylic acid, 'HBTU' means 1-[bis(dimethylamino)methylene]-1H-Benzotriazoliumhexafluorophosphate(1-)3-oxide, 'HATU' means 1-[bis(dimethylamino)methylene]-1H-1,2,3-triazolo[4,5-b]pyridinium 3-oxide, hexafluorophosphate(1-), 'HOAT' means 3-hydroxy-3H-1,2,3-triazolo[4,5-b]pyridine

A. Preparation of the intermediates

### Example A1

### a) Preparation of intermediate (1)

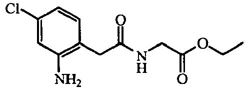

A mixture of *N*-[(4-chloro-2-nitrophenyl)acetyl]glycine ethyl ester (0.023 mol) in THF (250ml) was hydrogenated with Pt/C (2.0 g) as a catalyst in the presence of a 4% thiophene solution in DIPE (1ml). After uptake of H₂ (3 equiv.), the catalyst was filtered off and the filtrate was evaporated. The obtained residue was suspended in DIPE, then the suspension was stirred at boiling temperature, cooled and the desired product was collected by filtration, yielding 6.2g (100%) of intermediate (1).

### b) Preparation of intermediate (2)

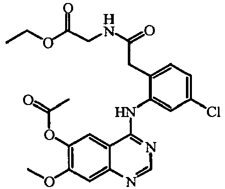

A mixture of 4-chloro-7-methoxy-6-quinazolinol acetate ester (0.00050 mol) and intermediate (1) (0.00050 mol) in 2-propanol (5ml) was stirred for 16 hours in a pressure tube at 80°C (oil bath temperature), then the reaction mixture was filtered and the filter residue was air-dried, yielding 0.165g (67.7%) of intermediate (2).

### c) Preparation of intermediate (3)

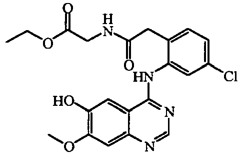

A mixture of intermediate (2) (0.0244 mol) in NH₃/CH₃OH (7N) (50ml) and CH₃OH (100ml) was stirred overnight at room temperature and then the solvent was evaporated (Genevac.) under reduced pressure and at room temperature. Finally, the obtained residue was dried (vac.) overnight at 60°C, yielding 8.2g (75%) of intermediate (3).

### d) Preparation of intermediate (4)

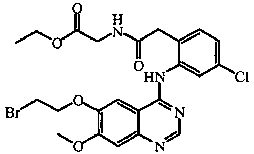

A mixture of intermediate (3) (0.0138 mol) and Cs₂CO₃ (0.0690 mol) in DMF (120ml) was stirred for 30 minutes at room temperature, then 1,2-dibromoethane (0.117 mol) was added and the reaction mixture was stirred overnight at room temperature. The solvent was evaporated under reduced pressure and the residue was co-evaporated with toluene. The obtained residue was stirred in DIPE and the desired product was filtered off, yielding 6.93g (91%) of intermediate (4).

### e) Preparation of intermediate (5)

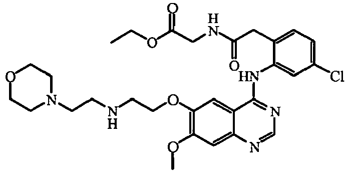

A mixture of intermediate (4) (0.00181 mol) and 4-morpholineethanamine (0.00907 mol) in ethanol (20ml) was heated in a microwave oven for 90 minutes at 100°C and then the reaction mixture was purified by reversed-phase high-performance liquid chromatography. The product fractions were collected and the solvent was evaporated, yielding 0.39g (36%) of intermediate (5).

### f) Preparation of intermediate (6)

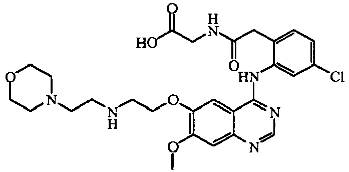

A mixture of intermediate (5) (0.00065 mol) and lithium hydroxide (0.0032 mol) in ethanol (20ml) and H₂O (2ml) was stirred for 2 hours at room temperature and then the solvent was evaporated under reduced pressure, yielding intermediate (6) (quantitative yield).

### Example A2

### a) Preparation of intermediate (7)

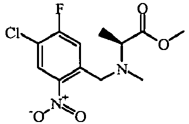

A mixture of 4-chloro-5-fluoro-2-nitrobenzaldehyde (0.0491 mol), *N*-methyl-L-alanine methyl ester hydrochloride (0.0589 mol) and titanium(4+) 2-propanol salt (0.0737 mol) in 1,2-dichloroethane (100ml) was stirred at room temperature for 30 minutes. NaBH(OAc)₃ (0.0589 mol) was added. The mixture was stirred overnight, then diluted in CH₂Cl₂, quenched with aqueous (10%) K₂CO₃ and filtered. The organic layer was separated, dried (MgSO₄), filtered, and the solvent was evaporated to dryness, yielding 16.5g (quantitative yield) of intermediate (7) (S-configuration).

### b) Preparation of intermediate (8)

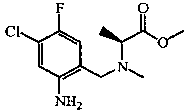

A mixture of intermediate (7) (0.0491 mol), Fe (0.246 mol) and NH₄Cl (0.491 mol) in THF/CH₃OH/H₂O (4/4/2; 500ml) was stirred and refluxed overnight, then cooled to room temperature and filtered. The filtrate was diluted in CH₂Cl₂. The organic layer was separated, dried (MgSO₄), filtered and the solvent was evaporated to dryness, yielding 13g (96%) of intermediate (8) (S-configuration).

### c) Preparation of intermediate (9)

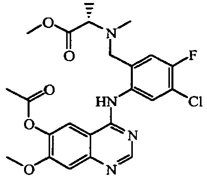

A mixture of 4-chloro-7-methoxy-6-quinazolinol acetate ester (0.0162 mol) and intermediate (8) (0.0162 mol) in CH₃CN (150ml) was stirred and refluxed for 4 hours, then cooled back to room temperature, the solvent was evaporated in vacuo and the residue was taken up in K₂CO₃ (aq.) (10%) and CH₂Cl₂. The organic layer was separated, dried (MgSO₄), filtered, and the solvent was evaporated to dryness. The residue (6.4g) was purified by column chromatography over silica gel (eluent: CH₂Cl₂/CH₃OH 100/0 to 99/1; 15-40µm). The desired fractions were collected and the solvent was evaporated, yielding 3.09g (37%) of intermediate (9) (S-configuration).

### d) Preparation of intermediate (10)

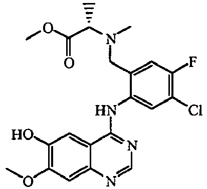

A mixture of intermediate (9) (0.0061 mol) in NH₃/CH₃OH (7N) (20ml) and CH₃OH (100ml) was stirred at room temperature for 40 hours, then evaporated to dryness. The residue was taken up in CH₃CN/DIPE. The precipitate was filtered off and dried, yielding 1.93g (70%) of intermediate (10) (M.P.: 234°C; S-configuration).

### e) Preparation of intermediate (11)

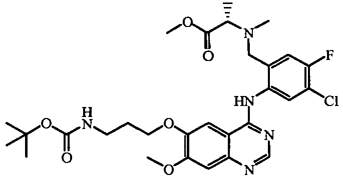

Cs₂CO₃ (0.0063 mol) was added to a solution of intermediate (10) (0.0042 mol) in dry DMF (20ml). The mixture was stirred at room temperature for 1 hour. A solution of (3-bromopropyl)-1,1-dimethylethyl ester carbamic acid (0.0046 mol) in dry DMF (5ml) was added. The mixture was stirred at room temperature for 3 hours, poured into H₂O and extracted with EtOAc. The organic layer was separated, dried (MgSO₄), filtered, and the solvent was evaporated to dryness, yielding: 2.8g (quantitative yield) of intermediate (11) (S-configuration).

### f) Preparation of intermediate (12)

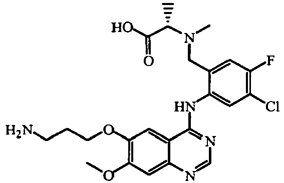

A mixture of intermediate (11) (0.0042 mol) in HCl (aq.) (6N) (20ml) and dioxane (100ml) was stirred at 60°C for 3 hours, then cooled to room temperature and evaporated to dryness. The residue was taken up in ethanol/diethyl ether. The precipitate was filtered under N₂ flow and dried in vacuo, yielding 2.24g (100%) of intermediate (12) as a hydrochloric acid salt(.3.02HCl.1.88H₂O; S-configuration; M.P.: 175°C).

### g) Preparation of intermediate (13)

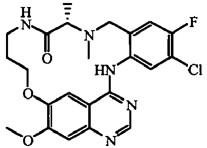

Intermediate (12) (0.0018 mol) was added portionwise to a warm solution (50°C) of EDCI (0.0037 mol), HOBT (0.0037 mol) and triethylamine (0.008 mol) in CH₂Cl₂/THF (50/50; 1000ml) over a 3 hour period, under vigorous stirring at 50°C. After evaporation of the solvent, the residue was taken up in K₂CO₃ (aq.) (10%). The mixture was extracted with CH₂Cl₂. The organic layer was separated, dried (MgSO₄), filtered and the solvent was evaporated to dryness. The residue (1g) was crystallized from ethanol/DIPE. The precipitate was filtered off and dried. This fraction was crystallized again from CH₃CN. The precipitate was filtered off and dried, yielding 0.21g (24%) of intermediate (13) (M.P.: 270°C; S-configuration).

### h) Preparation of intermediate (14)

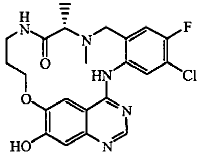

A mixture of intermediate (13) (0.0001 mol), sodium sulfide (0.001 mol) and lithium chloride (0.0011 mol) in DMF (1ml) was stirred at room temperature for 5 minutes, then heated in a microwave oven at 90°C for 15 minutes, poured into saturated NaHCO₃ and extracted with diethyl ether three times. The organic layer was washed with saturated NaCl, dried (MgSO₄), filtered and the solvent was evaporated to dryness. The residue (1.3g) was purified by column chromatography over silica gel (eluent: CH₂Cl₂/CH₃OH 100/0 to 90/10; 15-40µm). The desired fractions were collected and the solvent was evaporated. The residue was crystallized from CH₃CN. The precipitate was filtered off and dried, yielding 0.406g (84%) of intermediate (14) (M.P.: 196°C; S-configuration).

### i) Preparation of intermediate (15)

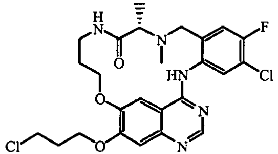

1-Bromo-3-chloropropane (0.0012 mol) was added to a suspension of intermediate (14) (0.0008 mol) and K₂CO₃ (aq.) (0.0016 mol) in CH₃CN/DMF (8ml). The mixture was stirred and refluxed for 18 hours, then cooled to room temperature, poured into H₂O and extracted with EtOAc. The organic layer was separated, dried (MgSO₄), filtered and the solvent was evaporated to dryness. The residue (0.85g) was purified by column chromatography over silica gel (eluent: CH₂Cl₂/CH₃OH 100/0 to 97/3; 15-40µm). The pure fractions were collected and the solvent was evaporated, yielding 0.24g (58%) of intermediate (15) (S-configuration).

### Example A3

### a) Preparation of intermediate (16)

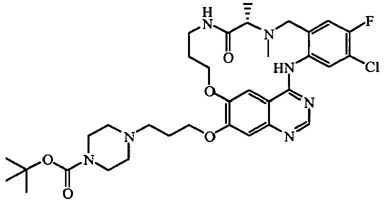

A mixture of intermediate (15) (0.0005 mol), 1,1-dimethylethyl ester 1-piperazinecarboxylic acid (0.001 mol) and K₂CO₃ (aq.) (0.0005 mol) in CH₃CN (3ml) was stirred and refluxed overnight. 1,1-Dimethylethyl ester 1-piperazinecarboxylic acid (0.001 mol) and K₂CO₃ (aq.) (0.0005 mol) were added again. The mixture was stirred and refluxed for 18 hours, cooled to room temperature, poured into H₂O and extracted with CH₂Cl₂. The organic layer was separated, dried (MgSO₄), filtered and the solvent was evaporated to dryness. The residue (0.487g) was purified by column chromatography over kromasil (eluent: CH₂Cl₂/CH₃OH/NH₄OH 99/1/0.05 to 90/10/0.5; 5µm). The pure fractions were collected and the solvent was evaporated, yielding 0.165g (46%) of intermediate (16) (S-configuration; M.P.: 140°C).

### b) Preparation of intermediate (17)

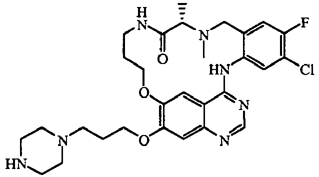

HCl/2-propanol (0.3ml) was added to a mixture of intermediate (16) (0.0001 mol) in CH₃OH (3ml). The mixture was stirred at room temperature overnight, then stirred at room temperature for 18 extra hours and evaporated to dryness. This hydrochloric acid salt was taken up in K₂CO₃ (aq.) (10%). The mixture was extracted with CH₂Cl₂. The organic layer was separated, dried (MgSO₄), filtered and the solvent was evaporated to dryness, yielding: 0.095g (100%) of intermediate (17) (S-configuration).

### Example A4

### a) Preparation of intermediate (18)

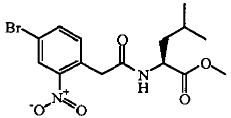

A mixture of 4-bromo-2-nitrobenzeneacetic acid (0.077 mol) and HOBT (0.077 mol) in CH₂Cl₂ (550ml) was stirred at room temperature. CDI (0.077 mol) was added and stirring was continued for 10 minutes. Then DIPEA (0.077 mol) was added and the reaction mixture was stirred at room temperature for 30 minutes. L-Leucine methyl ester hydrochloride (0.077 mol) was added at once and the mixture was stirred overnight at room temperature. An extra amount of HOBT (0.077 mol), CDI (0.077 mol) and DIPEA (0.077 mol) was added and the reaction mixture was stirred at room temperature over the weekend. The mixture was quenched with H₂O and the layers were separated. The organic layer was washed with saturated K₂CO₃ (aq.) (1x) and HCl (1N) (1x), then dried (MgSO₄), filtered and the solvent was evaporated. The red gum-like product was triturated from 2-propanol. The off-white solid was filtered off and dried, yielding 7.87g of intermediate (18) (S-configuration).

### b) Preparation of intermediate (19)

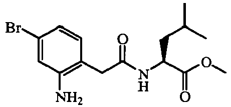

A mixture of intermediate (18) (0.056 mol) in toluene (219ml) was stirred (mixture (1)). A mixture of NH₄Cl (0.283 mol) in H₂O (151ml) was added to mixture (1) and in a next step Fe (0.283 mol) was added. The reaction mixture was refluxed overnight. Then another portion of NH₄Cl (0.283 mol) and Fe (0.283 mol) was added and the reaction mixture was refluxed for 1 hour. The mixture was cooled to room temperature and then filtered through dicalite. The layers were separated and the aqueous layer was washed with toluene. The combined organic layers were dried (MgSO₄), filtered and the solvent was evaporated, yielding 20.1g of intermediate (19) (S-configuration).

### c) Preparation of intermediate (20)

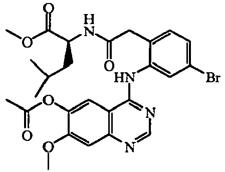

A solution of intermediate (19) (0.055 mol) in 2-propanol (200ml) was heated to 70°C (solution (1)). A solution of 4-chloro-7-methoxy-6-quinazolinol acetate ester (0.066 mol) in 2-propanol (200ml) was also heated to 70°C and this solution was added to solution (1). Stirring at 70°C was continued for 75 minutes. An extra amount of 4-chloro-7-methoxy-6-quinazolinol acetate ester (0.027 mol) in 2-propanol (100ml) was added and the mixture was reacted further for 2 hours. The solvent was evaporated and the residue was purified by column chromatography over silica gel (eluent: CH₂Cl₂/CH₃OH 99.5/0.5 till 90/10). The product fractions were collected and the solvent was evaporated, yielding 13.82g of intermediate (20) (S-configuration).

### d) Preparation of intermediate (21)

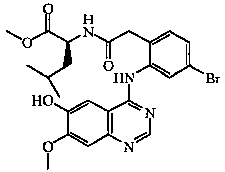

A mixture of intermediate (20) (0.081 mol) in CH₃OH (400ml) was stirred at room temperature. NH₃/CH₃OH (7N) (200ml) was added and the reaction mixture was stirred at room temperature for 95 minutes. The solvent was evaporated and the residue was triturated from 2-propanol. The pale yellow solid was filtered off and dried, yielding 43g (99.9%) of intermediate (21) (S-configuration).

### e) Preparation of intermediate (22)

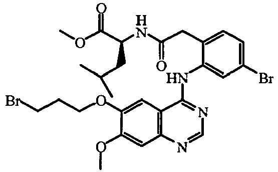

A mixture of intermediate (21) (0.0113 mol) in DMF (300ml) was stirred. K₂CO₃ (aq.) (0.056 mol) was added and the reaction mixture was stirred at room temperature for 35 minutes. Then 1,3-dibromopropane (0.113 mol) was added and the reaction mixture was stirred for 40 hours at room temperature. The reaction mixture was filtered and concentrated under reduced pressure till ~20ml. The concentrate was poured into H₂O and the precipitation was filtered off and dried, yielding 7.16g (97.1 %) of intermediate (22) (S-configuration).

### f) Preparation of intermediate (23)

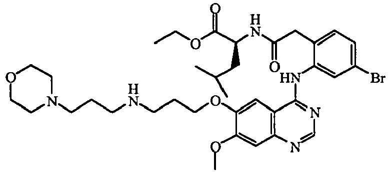

A mixture of intermediate (22) (0.003832 mol) and 4-morpholinepropanamine (0.0383 mol) in ethanol (40ml) was heated to 100°C for 1 hour and then purified by high performance liquid chromatography. The organic solvent was evaporated and the water layer was concentrated to ~20ml. The concentrate was made alkaline with aqueous NaOH (1N) to a pH of ~10 and extracted with EtOAc. The separated organic layer was dried (MgSO₄), filtered and the mixture was concentrated, yielding 9.14g of intermediate (23) (S-configuration).

### g) Preparation of intermediate (24)

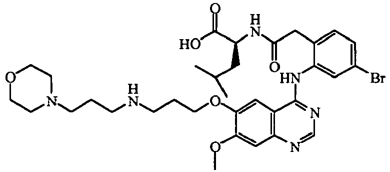

A mixture of intermediate (23) (0.007909 mol) in CH₃OH (40ml) and H₂O (4ml) was stirred at room temperature until dissolution. Lithium hydroxide (0.0395 mol) was added and the reaction mixture was stirred for 85 minutes. The reaction mixture was concentrated and the residue was dried, yielding 5.53 g (99.6%) of intermediate (24) (S-configuration).

### h) Preparation of intermediate (25)

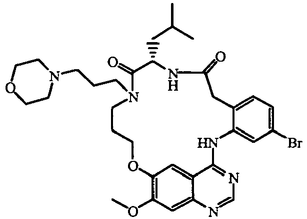

A mixture of HATU (0.002052 mol) and HOAT (0.00008551 mol) in DMA (50ml) was added dropwise to a mixture of intermediate (24) (0.0007126 mol) and DIPEA (0.002138 mol) in DMA (50ml). The reaction mixture was stirred overnight. H₂O was added and the mixture was concentrated to ~10ml. EtOAc was added to the mixture to become a solution. H₂O was added and the two layers were separated. The organic layer was dried (MgSO₄), filtered and the solvent was evaporated. The obtained residue was purified by high performance liquid chromatography (NH₄HCO₃ buffer). The product fractions were collected, the solvent was evaporated and the residue was dried, yielding intermediate (25) (S-configuration; quantitative yield).

### i) Preparation of intermediate (26)

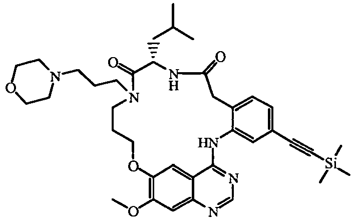

A mixture of intermediate (25) (0.0007314 mol) in pyrrolidine (10ml) was stirred dichlorobis(triphenylphosphine)palladium (0.00003657 mol) and copper iodide (catalytic amount) were added and the reaction mixture was heated to 75°C. Ethynyltrimethylsilane (0.001828 mol) was added and heating was continued for 30 minutes. Then an extra portion of dichlorobis(triphenylphosphine)palladium (0.00003657 mol) and ethynyltrimethylsilane (0.001828 mol) was added and the mixture was reacted for 270 minutes. The reaction mixture was filtered through celite and washed with CH₃OH. The solvent was evaporated and the residue was redissolved in EtOAc and washed 2x with H₂O. The organic layer was dried (MgSO₄), filtered and the solvent was evaporated. The crude residue was redissolved in CH₃CN and MP-TMT resin (0.0003657 mol) was added to scavenge any residual Pd. This mixture was stirred for 36 hours at room temperature and was then filtered. The resin was washed with CH₃OH and the filtrate was evaporated, yielding 0.48 g of intermediate (26) (S-configuration).

### Example A5

### a) Preparation of intermediate (27)

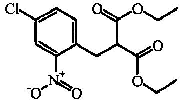

4-Chloro-1-(chloromethyl)-2-nitrobenzene (0.81 mol) and propanedioic acid diethyl ester (0.794 mol) were suspended in hexane (300ml). K₂CO₃ (aq.) (0.81 mol) was added. Then, 18-crown-6 (0.008 mol) was added. The resultant reaction mixture was stirred and refluxed for 30 hours under N₂ atmosphere. The reaction mixture was cooled to 20°C. This mixture was extracted with water (750ml). The layers were separated. The aqueous phase was washed with toluene. The combined organic layers were dried (Na₂SO₄), filtered and the solvent was evaporated, yielding 255.8g of intermediate (27).

### b) Preparation of intermediate (28)

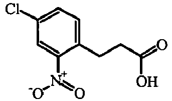

Intermediate (27) (255.8g, 0.466 mol) was dissolved in acetic acid (1000ml). A 20% aqueous HCl solution (1000ml) was added and the resulting reaction mixture was stirred and refluxed for 16 hours. The reaction mixture was cooled to 20°C and the solvent was evaporated. The residue was suspended in water (500ml) and treated with a 10% aqueous NaOH solution (500ml). This mixture was stirred for one hour. This mixture was extracted with diethyl ether (3 x 500ml) and then acidified with concentrated HCl resulting in precipitation from the cooled aqueous layer. The precipitate was filtered off and dried, yielding 109g of intermediate (28) (M.P.: 109-111°C).

### c) Preparation of intermediate (29)

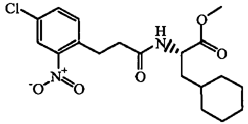

A mixture of intermediate (28) (0.015 mol) and HOBT (0.015 mol) in CH₂Cl₂ (10ml) was stirred for 30 minutes at room temperature. CDI (0.015 mol) was added and the reaction mixture was stirred for 30 minutes at room temperature. The resultant solution was added to a mixture of α-aminocyclohexanepropanoic acid methyl ester hydrochloride (0.01875 mol) and diisopropylmethylamine/resin (1 0.05 mol) in CH₂Cl₂ (70ml) and the reaction mixture was shaken overnight at room temperature. An excess of scavenger resins (polystyrylmethyl)trimethylammonium bicarbonate and sulfonic acid resin MP (70-90 mesh) were added and the mixture was shaken for 18 hours. The mixture was filtered. The filtrate was concentrated at room temperature, yielding intermediate (29) (S-configuration; quantitative yield).

### d) Preparation of intermediate (30)

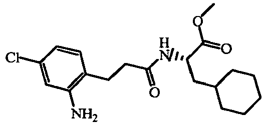

A mixture of intermediate (29) (0.001 mol) in 2-propanol (20ml) was hydrogenated with 5% Pt/C (catalytic quantity) as a catalyst in the presence of vanadium oxide (q.s.) and a 4% thiophene solution in DIPE (q.s.). After uptake of H₂ (3 equiv), the catalyst was filtered off and the filtrate was evaporated, yielding intermediate (30) (S-configuration; quantitative yield).

### e) Preparation of intermediate (31)

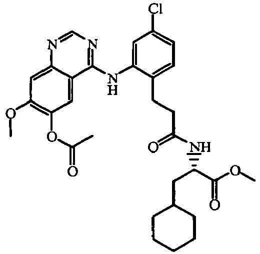

A mixture of 4-chloro-7-methoxy-6-quinazolinol acetate ester (0.001 mol) and intermediate (30) (1 equiv; 0.001 mol) in 2-propanol (25ml) was stirred for 6 hours at 80°C. The reaction mixture was cooled to room temperature and used as such in next reaction step, yielding intermediate (31) (S-configuration; quantitative yield).

### f) Preparation of intermediate (32)

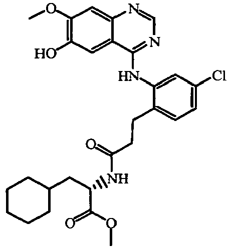

A mixture of intermediate (31) (0.0010 mol) in 2-propanol (25ml) and NH₃/CH₃OH (5ml) was stirred for 18 hours at room temperature. The solvent was evaporated under reduced pressure. The residue was purified by high-performance liquid chromatography. The product fractions were collected and the solvent was evaporated, yielding intermediate (32) (S-configuration; quantitative yield).

### g) Preparation of intermediate (33)

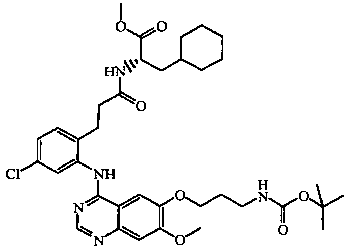

A mixture of intermediate (32) (crude) and Cs₂CO₃ (5 equiv.) in DMF (5ml) was stirred for 30 minutes at room temperature. (3-bromopropyl)-1,1-dimethylethyl ester carbamic acid (1.1 equiv.) was added and the reaction mixture was stirred for 18 hours at room temperature. The solvent was evaporated under reduced pressure, yielding intermediate (33) (S-configuration; quantitative yield).

### h) Preparation of intermediate (34)

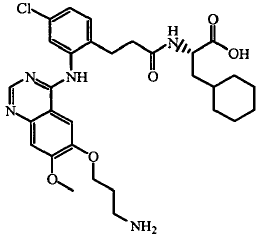

A solution of intermediate (33) (crude) in HCl (6N) (2ml) and dioxane (2ml) was stirred for 16 hours at 60°C. The solvent was evaporated under reduced pressure, yielding of intermediate (34) (S-configuration; quantitative yield).

### Example A6

### a) Preparation of intermediate (35)

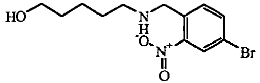

A solution of 4-bromo-2-nitrobenzaldehyde (0.013 mol), 5-amino-1-pentanol (0.013 mol) and titanium(4+) 2-propanol salt (0.014 mol) in ethanol (15ml) was stirred at room temperature for 1 hour, then the reaction mixture was heated to 50°C and stirred for 30 minutes. The mixture was cooled to room temperature and NaBH₄ (0.013 mol) was added portionwise. The reaction mixture was stirred overnight and then poured onto ice water (50ml). The resulting mixture was stirred for 20 minutes, the formed precipitate was filtered off (giving Filtrate (I)), washed with H₂O and stirred in CH₂Cl₂ (to dissolve the product and to remove it from the Ti-salt). The mixture was filtered and then the filtrate was dried (MgSO₄) and filtered, finally the solvent was evaporated to dryness. Filtrate (I) was evaporated until ethanol was removed and the aqueous concentrate was extracted 2 times with CH₂Cl₂. The organic layer was separated, dried (MgSO₄), filtered off and the solvent was evaporated dry, yielding 3.8 g (93%) of intermediate (35).

### b) Preparation of intermediate (36)

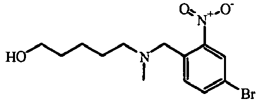

A solution of intermediate (35) (0.0047 mol), formaldehyde (0.025 mol) and titanium(4+) 2-propanol salt (0.0051 mol) in ethanol (150ml) was heated to 50°C and stirred for 1 hour, then NaBH₄ (0.026 mol) was added portionwise at room temperature. The reaction mixture was stirred overnight and then quenched with water (100ml). The resulting mixture was stirred for 1 hour; the formed precipitate was filtered off and washed. The organic filtrate was concentrated, then the aqueous concentrate was extracted with CH₂Cl₂ and dried. The solvent was evaporated dry and the residue was filtered over silica gel (eluent: CH₂Cl₂/CH₃OH from 98/2 to 95/5). The product fractions were collected and the solvent was evaporated dry, yielding 0.5g of intermediate (36).

### c) Preparation of intermediate (37)

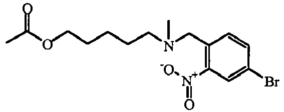

A solution of intermediate (36) (0.0015 mol) and pyridine (0.015 mol) in acetic acid anhydride (8ml) was stirred overnight at room temperature, then the solvent was evaporated and co-evaporated with toluene, yielding intermediate (37) (quantitative yield).

### d) Preparation of intermediate (38)

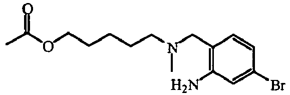

A mixture of intermediate (37) (0.0015 mol) in THF (50ml) was hydrogenated with 5% Pt/C (0.5 g) as a catalyst in the presence of a 4% thiophene solution in DIPE (0.5ml). After uptake of H₂ (3 equiv.), the catalyst was filtered off and the filtrate was evaporated, yielding 0.5g of intermediate (38).

### e) Preparation of intermediate (39)

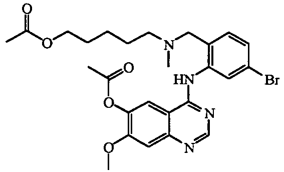

A mixture of intermediate (38) (0.0015 mol) and 4-chloro-7-methoxy-6-quinazolinol acetate ester (0.0015 mol) in 2-propanol (30ml) was heated to 80°C and the reaction mixture was stirred for 1 day. The solvent was evaporated under reduced pressure, yielding 0.83g of intermediate (39).

### f) Preparation of intermediate (40)

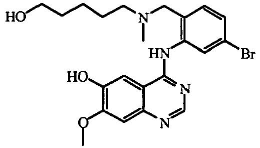

A solution of intermediate (39) (0.0015 mol) in CH₃OH (25ml) was stirred at room temperature and a solution of K₂CO₃ (0.003 mol) in H₂O (2.5ml) was added, then the reaction mixture was heated to 60°C and stirred for 18 hours. The solvent was evaporated and H₂O (20ml) was added, then the mixture was neutralised with acetic acid and the formed precipitate was filtered off. The filtrate was concentrated under reduced pressure and the concentrate was extracted with CH₂Cl₂, filtered, then dried (MgSO₄) and the mixture was concentrated under reduced pressure, yielding 0.5g (70%) of intermediate (40).

### g) Preparation of intermediate (41)

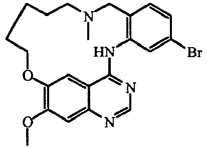

A solution of intermediate (40) (0.0011 mol) in THF (50ml) was stirred at room temperature and tributylphosphine (0.0016 mol) was added, then 1,1'-(azodicarbonyl)bispiperidine (0.0016 mol) was added and the reaction mixture was stirred for 2 hours. The solvent was evaporated until 1/3 of the initial volume. The resulting precipitate was filtered off and washed. The filtrate was evaporated and the residue was purified by high-performance liquid chromatography. The product fractions were collected and the organic solvent was evaporated. The aqueous concentrate was extracted 2 times with CH₂Cl₂ and the organic layer was dried (MgSO₄). The solvent was evaporated dry and the residue was dried (vacuum) at 50°C, yielding 0.004g (0.8 %) of intermediate (41).

### h) Preparation of intermediate (42)

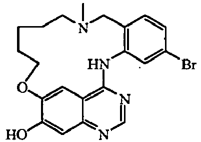

A 48% solution of hydrobromide in water (5.5ml) was added to a suspension of intermediate (41) (0.0058 mol) and potassium iodide (0.044 mol) in DMA (55ml), stirred at room temperature under N₂ flow. The reaction mixture was stirred for 2.5 hours at 130°C. The reaction mixture was poured onto ice water. The layers were separated. The aqueous layer was neutralised with NaOH (1N) and the resulting precipitate was filtered off, then dissolved in CH₂Cl₂, washed with water, separated and the organic phase was dried, filtered and the solvent evaporated under reduced pressure. The residue was stirred in water, filtered off, dissolved in THF and the solvent was evaporated (toluene was added and azeotroped on the rotary evaporator), yielding 1.58g (61%) of intermediate (42).

### i) Preparation of intermediate (43)

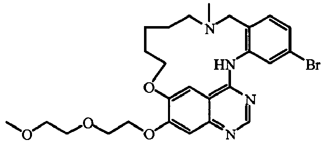

Bis(1-methylethyl) ester diazenedicarboxylic acid (0.0158 mol) was added dropwise to a suspension of intermediate (42) (0.007895 mol), 2-(2-methoxyethoxy)ethanol (0.0631 mol) and triphenylphosphine (0.0158 mol) in THF (120ml), stirred at room temperature. The reaction mixture was stirred at room temperature for 20 minutes. The solvent was evaporated in vacuo. The residue was stirred for 10 minutes in CH₃CN at room temperature. The precipitate was filtered off, washed with CH₃CN and dried, yielding 3.37g (78%) of intermediate (43).

### j) Preparation of intermediate (44)

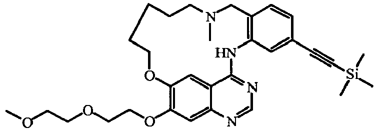

Intermediate (43) (0.0009166 mol) was stirred in pyrrolidine (10ml). Dichlorobis(triphenylphosphine)palladium (0.00004583 mol) was added, followed by addition of copper iodide (catalytic quantity). The mixture was heated to 70°C. Ethynyltrimethylsilane (0.002292 mol) was added and the reaction mixture was stirred at 70°C for 4.75 hours. The reaction mixture was cooled to room temperature, filtered through dicalite and the filter residue was washed with CH₃OH. The filtrate was concentrated. The concentrate was redissolved in EtOAc, then partitioned between water and EtOAc. The organic layer was separated, dried (MgSO₄), filtered and the solvent was evaporated. The residue was redissolved in CH₃CN and treated with MP-TMT resin (0.002292 mol) to scavenge any residual Pd. The mixture was stirred slowly over the weekend. The mixture was filtered. The resin was washed with CH₃OH and the filtrate's solvent was evaporated, yielding 0.400g of intermediate (44).

### Example A7

### a) Preparation of intermediate (45)

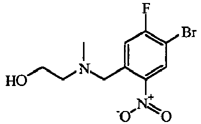

A solution of 2-(methylamino)ethanol (0.077 mol) in CH₂Cl₂ (180ml) was stirred at room temperature. Tetrakis(2-methyl-2-propanolato)titanate(1-) (0.077 mol) was added, followed by triethylamine (0.077 mol). 4-Bromo-5-fluoro-2-nitrobenzaldehyde (0.077 mol) was added and the mixture was stirred for 90 minutes. NaBH(OAc)₃ (0.0847 mol) was added and the reaction mixture was stirred for 18 hours at room temperature. The mixture was poured into an aqueous NaHCO₃ solution. The precipitate was filtered off. The layers were separated. The organic phase was washed with water (2 x), dried (MgSO₄), filtered and the solvent was evaporated. The residue was purified by column chromatography over silica gel (eluent: CH₂Cl₂/CH₃OH 99/1 to 99/2). The desired fractions were collected and the solvent was evaporated, yielding 18g of intermediate (45).

### b) Preparation of intermediate (46)

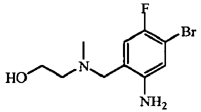

A mixture of intermediate (45) (0.059 mol) in EtOAc (250ml) was hydrogenated at room temperature and atmospheric pressure with 5% Pt/C (2 g) as a catalyst in the presence of vanadium oxide (0.5 g) and a 4% thiophene solution in DIPE (2ml). After uptake of H₂ (3 equiv), the catalyst was filtered off and the filtrate was evaporated, yielding intermediate (46) (quantitative yield).

### c) Preparation of intermediate (47)

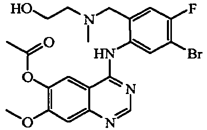

A mixture of intermediate (46) (0.0396 mol) and 4-chloro-7-methoxy-6-quinazolinol acetate ester (0.0396 mol) in 2-propanol (300ml) was stirred for 1 day at 75°C. More 4-chloro-7-methoxy-6-quinazolinol acetate ester (5 g) was added and the reaction mixture was stirred again for 1 day at 75°C. The solvent was evaporated under reduced pressure, yielding intermediate (47) (quantitative yield).

### d) Preparation of intermediate (48)

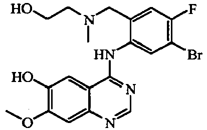

A mixture of intermediate (47) (0.0396 mol) in NH₃/CH₃OH (200ml) and CH₃OH (100ml) was stirred overnight at room temperature. The resulting precipitate was filtered off, washed and dried (vacuum, 60°C), yielding 15.7g of intermediate (48).

### e) Preparation of intermediate (49)

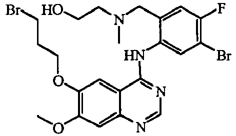

A solution of intermediate (48) (0.0347 mol) in DMF (150ml) was stirred at room temperature and treated with K₂CO₃ (aq.) (0.16 mol). The reaction mixture was stirred for 45 minutes at room temperature. 1,3-Dibromopropane (0.31 mol) was added and the reaction mixture was stirred for 2 hours at room temperature. The mixture was poured onto ice/water, stirred for 10 minutes, and the resulting precipitate was filtered off, washed and dried (vacuum, 60°C). The solid was stirred in DIPE, filtered off, washed, then dried again in vacuo at 60°C, yielding 19.2g (97%) of intermediate (49).

### f) Preparation of intermediate (50)

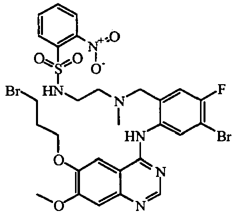

A solution of intermediate (49) (0.033 mol), 2-nitrobenzenesulfonamide (0.10 mol) and triphenylphosphine (0.0495 mol) in THF (700ml) was stirred at room temperature. A solution of bis(1-methylethyl) ester diazenedicarboxylic acid (0.0495 mol) in THF (50ml) was added dropwise and the reaction mixture was stirred overnight. The solvent was evaporated under reduced pressure. The residue was purified by column chromatography over silica gel. The product fractions were collected and the solvent was evaporated, yielding intermediate (50) (quantitative yield).

### Example A8

### a) Preparation of intermediate (51)

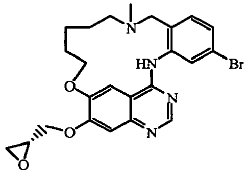

DIAD (0.005 mol) was added dropwise to a mixture of intermediate (42) (0.0017 mol), (2R)-xiranemethanol (0.0105 mol) and triphenylphosphine (0.005 mol) in THF (30ml), stirred at room temperature for 5 hours. The precipitate was filtered off, washed with THF, and dried, yielding 0.545 g (64%) of intermediate (51) (R-configuration).

### Example A9

### a) Preparation of intermediate (52)

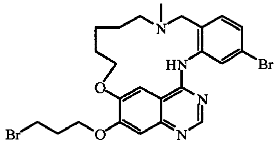

DIAD (0.0003 mol) was added dropwise to a solution of intermediate (42) (0.000138 mol), 3-bromo-1-propanol (0.00055 mol) and triphenylphosphine (0.0003 mol) in THF (2ml), stirred at room temperature. The reaction mixture was stirred for 1 hour at room temperature. The solvent was evaporated under a gentle flow of N₂, yielding intermediate (52) (quantitative yield).

### B. Preparation of the compounds

### Example B1

### Preparation of compound (1)

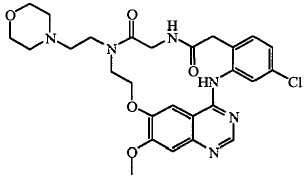

HBTU (0.00195 mol) was added to a stirred solution of intermediate (6) (0.00069 mol) and DIPEA (0.00324 mol) in *N,N-*dimethylacetamide (250ml) at room temperature, then the reaction mixture was stirred for 3 hours and the solvent was co-evaporated with toluene under reduced pressure. The obtained residue was purified by reversed-phase high-performance liquid chromatography (eluent 1: NH₄OAc; eluent 2: NH₄HCO₃). The pure product fractions were collected and the solvent was evaporated under reduced pressure. The obtained residue (0.030 g) was crystallised from 2-propanol, then the resulting precipitate was filtered off and dried (vacuum), yielding 0.0165g of compound (1).

### Example B2

### Preparation of compound (2) and compound (3)

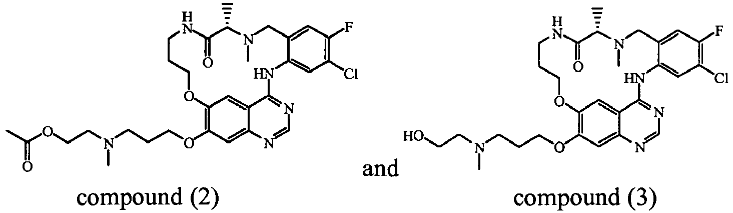

A mixture of intermediate (15) (0.0002 mol), 2-(methylamino)ethanol (0.0005 mol) and K₂CO₃ (aq.) (0.0002 mol) in CH₃CN (1.5ml) was stirred and refluxed overnight, then cooled to room temperature, poured into H₂O and extracted with CH₂Cl₂. The organic layer was separated, dried (MgSO₄), filtered and the solvent was evaporated to dryness. The residue (0.16g) was purified by column chromatography over kromasil (eluent: CH₂Cl₂/CH₃OH/NH₄OH 99/1/0.05 to 88/12/1.2; 5µm). Two fractions were collected and the solvent was evaporated, yielding 0.009g (6%) of compound (3) (S-configuration) and 0.05g (31%) of compound (2) (S-configuration).

### Example B3

### Preparation of compound (4)

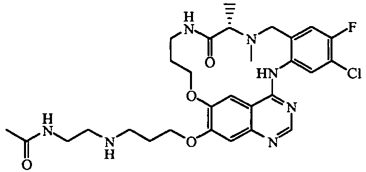

A mixture of intermediate (15) (0.0002 mol), *N*-(2-aminoethyl)acetamide (0.0005 mol) and K₂CO₃ (aq.) (0.0002 mol) in CH₃CN (1.5ml) was stirred and refluxed overnight. *N-*(2-aminoethyl)acetamide and K₂CO₃ (aq.) were added again. The mixture was stirred and refluxed for 5 hours, then cooled to room temperature, poured into H₂O and extracted with CH₂Cl₂. The organic layer was separated, dried (MgSO₄), filtered and the solvent was evaporated to dryness. The residue (0.146g) was purified by column chromatography over kromasil (eluent: CH₂Cl₂/CH₃OH/NH₄OH 99/1/0.05 to 75/25/1; 5µm). The pure fractions were collected and the solvent was evaporated. The residue (0.042g, 27%) was crystallized from diethyl ether. The precipitate was filtered off and dried, yielding 0.034g (22%) of compound (4) (S-configuration; M.P.: 112°C).

### Example B4

### Preparation of compound (5)

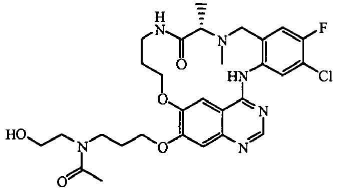

A mixture of intermediate (15) (0.0002 mol), ethanolamine (0.0005 mol) and K₂CO₃ (aq.) (0.0002 mol) in CH₃CN (1.5ml) was stirred and refluxed overnight. CH₃OH was added. The mixture was stirred at room temperature for 18 hours, poured into H₂O and extracted with EtOAc. The organic layer was separated, dried (MgSO₄), filtered and the solvent was evaporated to dryness. The residue (0.12g) was purified by column chromatography over kromasil (eluent: CH₂Cl₂/CH₃OH/NH₄OH 96/4/0.4 to 86/4/1.4; 5µm). The pure fractions were collected and the solvent was evaporated, yielding 0.048g (33%) of compound (5) (S-configuration).

### Example B5

### Preparation of compound (6)

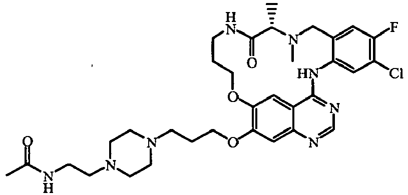

A mixture of intermediate (17) (0.0001 mol), *N-*(2-chloroethyl)acetamide (0.0001 mol), K₂CO₃ (aq.) (0.0003 mol) and potassium iodide (0.004g) in ethanol (3ml) was stirred and refluxed for 3 days, then cooled to room temperature, poured into H₂O and extracted with CH₂Cl₂. The organic layer was separated, dried (MgSO₄), filtered and the solvent was evaporated to dryness. The residue (0.097g) was purified by column chromatography over kromasil (eluent: CH₂Cl₂/CH₃OH/NH₄OH 96/4/0.5 to 90/10/0.5; 5µm). The pure fractions were collected and the solvent was evaporated. The residue (0.042g, 42%) was crystallized from CH₃CN. The precipitate was filtered off and dried, yielding 0.032g (32%) of compound (6) (S-configuration; M.P.: 136°C).

### Example B6

### Preparation of compound (7)

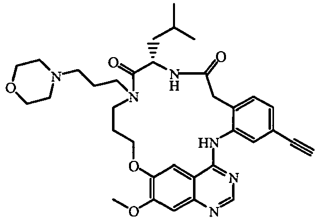

A mixture of intermediate (26) (0.0006848 mol) in a saturated aqueous K₂CO₃ solution (60ml) and CH₃OH (60ml) was stirred for 30 minutes at room temperature. The solvent was evaporated and the residue was dissolved in CH₂Cl₂/H₂O. The layers were separated and the organic layer was dried (MgSO₄), filtered and the solvent was evaporated. The crude residue was purified by flash column chromatography over silica gel (eluent: CH₂Cl₂/CH₃OH 99/1 till 95/5; column was stripped with CH₂Cl₂/(CH₃OH/NH₃) 95/5). The desired fractions were purified again by column chromatography over silica gel (eluent: CH₂Cl₂/(CH₃OH/NH₃) 100/0 to 97/3). The product fractions were collected and the solvent was evaporated. The residue was purified by high-performance liquid chromatography (ammonium acetate buffer). The product fractions were collected, the CH₃CN was evaporated and the aqueous layer was made alkaline (pH=10). The product was extracted with CH₂Cl₂. The separated organic layer was dried and the solvent was evaporated, yielding 0.419 g of compound (7) (S-configuration).

### Example B7

### Preparation of compound (8)

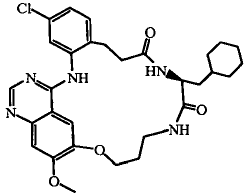

A solution of HBTU (excess) and DIPEA (3 equiv) in DMF (3ml) was stirred at room temperature. A solution of intermediate (34) (crude) in DMF (2ml) was added dropwise (Zymark). The resultant reaction mixture was stirred overnight at room temperature. The solvent was evaporated. The residue was purified by high-performance liquid chromatography. The product fractions were collected and the solvent was evaporated, yielding 0.016g of compound (8) (S-configuration).

### Example B8

### Preparation of compound (9)

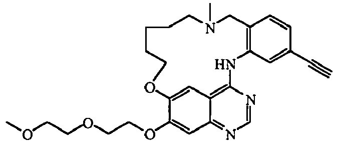

Lithium hydroxide (0.340 g, 0.0081 mol) was added to a mixture of intermediate (44) (0.0006 mol) in CH₃OH (25ml) and H₂O (5ml), stirred at room temperature. The reaction mixture was stirred for one hour at 40°C. The mixture was concentrated under reduced pressure to one fifth of the initial volume. The concentrate was poured into water. The mixture was stirred for 30 minutes at room temperature. The precipitate was filtered off, stirred in THF (20ml) for one hour, then the precipitate was filtered off again. The solid was dissolved in THF/CH₃OH 1/1 (200ml). The whole was filtered and the filtrate was evaporated under reduced pressure. The residue was dried, then stirred for one hour in CH₃CN. The precipitate was filtered off and dried, yielding 0.142g (48%) of compound (9).

### Example B9

### Preparation of compound (10)

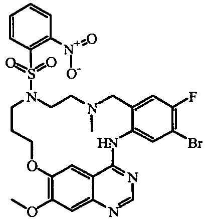

To a stirred mixture of Cs₂CO₃ (0.018 mol), CH₃CN (100ml) and *N,N,N-*tributyl-1-butanaminium iodide (0.0072 mol) was added a solution of intermediate (50) (0.0036 mol) in CH₃CN (300ml) at 60°C. The reaction mixture was stirred for 4 hours at 60°C. The solvent was evaporated under reduced pressure. The residue was purified by high-performance liquid chromatography. The product fractions were collected and the solvent was evaporated, yielding 1.4g of compound (10).

### Example B10

### Preparation of compound (11)

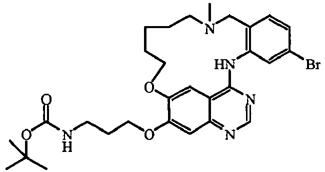

A mixture of intermediate (42) (0.0017 mol), (3-hydroxypropyl)-1,1-dimethylethyl ester carbamic acid (0.0041 mol) and triphenylphosphine (0.0038 mol) in THF (20ml) was stirred at room temperature. DIAD (0.004 mol) was added dropwise and the reaction mixture was stirred for 1 hour at room temperature. The solvent was evaporated and the residue was stirred up in CH₃CN (50ml). The precipitate was filtered off, washed with CH₃CN and dried, yielding 0.815g (80%) of compound (11).

### Example B11

### Preparation of compound (12)

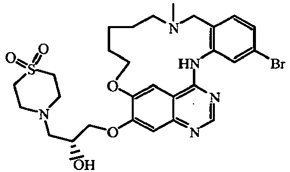

A mixture of intermediate (51) (0.00032 mol) and 1,1-dioxidethiomorpholine (0.00185 mol) in 2-propanol (2ml) was stirred for 2 hours at 70°C. DMF (2ml) was added and the resultant reaction mixture was stirred for 16 hours at 70°C. The reaction mixture was cooled at room temperature slowly. The precipitate was filtered off and dried, yielding 0.108g (53%) of compound (12) (R-configuration).

### Example B12

### Preparation of compound (13)

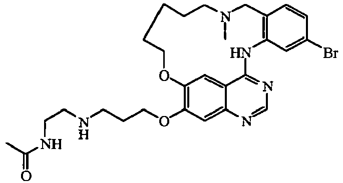

Intermediate (52) (0.003190 mol) was stirred in CH₃CN (20ml).
*N-*(2-aminoethyl)acetamide (2ml) was added and the resultant reaction mixture was stirred overnight at room temperature. K₂CO₃ (aq.) (0.009569 mol) was added and the reaction mixture was stirred and refluxed for 2 hours, then cooled to room temperature and the solvent was evaporated in vacuo. Water was added to the residue and this mixture was stirred for 30 minutes at room temperature. The yellow precipitate was filtered off and dried. This fraction was purified by flash column chromatography over a Biotage cartridge (eluent: CH₂Cl₂/(CH₃OH/NH₃) 95/5 up to 80/20). The product fractions were collected and the solvent was evaporated, yielding 0.94g of compound (13).

### Example B 13

### Preparation of compound (14)

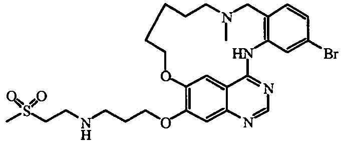

Intermediate (52) (0.003544 mol) was stirred in CH₃CN (20ml).
2-(Methylsulfonyl)ethanamine hydrochloride (0.007088 mol) was added. K₂CO₃ (aq.) (0.0106 mol) was added and the reaction mixture was stirred and refluxed overnight, then cooled to room temperature and the solvent was evaporated in vacuo. Water was added to the residue and this mixture was stirred for 10 minutes at room temperature. The yellow precipitate was filtered off and dried. This fraction was purified by flash column chromatography over a Biotage cartridge (eluent: CH₂Cl₂/(CH₃OH/NH₃) from 100/0 to 94/6). The product fractions were collected and the solvent was evaporated, yielding 1.24g (58%) of compound (14).

Table F-1 lists the compounds that were prepared according to one of the above Examples. The following abbreviations were used in the tables : M.P. stands for the melting point.

**Table F-1**

| | |
|---|---|
| 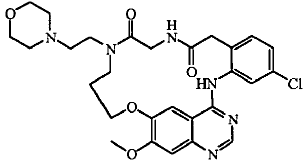 | 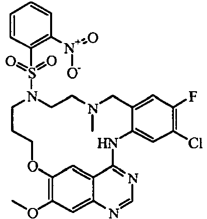 |
| Co. No. (15); Ex. B1 | Co. No. (16); Ex. B9 |
| 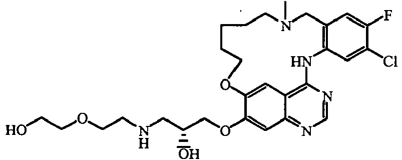 | 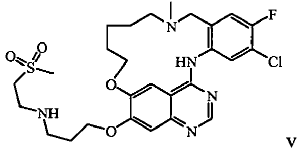 |
| Co. No. (17); Ex. B11; R-configuration | Co. No. (18); Ex. B 12 |
| 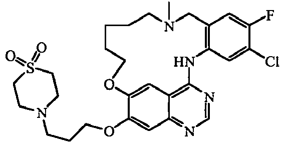 | 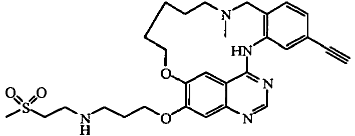 |
| Co. No. (19); Ex. B 12 | Co. No. (20); Ex. B 13; M.P.: 197.7-199.5°C (decomposition) |
| 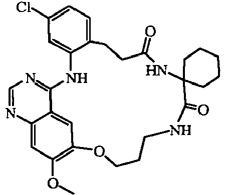 | 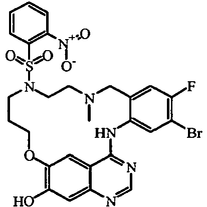 |
| Co. No. (21); Ex. B6 | Co. No. (22); Ex. B9 |
| 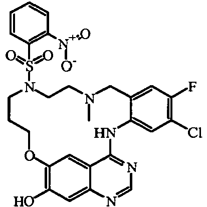 | |
| Co. No. (23); Ex. B9 | |

### Compound identification

### LCMS-methods:

The HPLC gradient was supplied by a Waters Alliance HT 2790 system with a column heater set at 40°C. Flow from the column was split to a Waters 996 photodiode array (PDA) detector and a Waters-Micromass ZQ mass spectrometer with an electrospray ionization source operated in positive and negative ionization mode.

### Method 1:

Reversed phase HPLC was carried out on a Xterra MS C18 column (3.5 mm, 4.6 x 100 mm) with a flow rate of 1.6 ml/min. Three mobile phases (mobile phase A 95% 25mM ammonium acetate + 5% acetonitrile; mobile phase B: acetonitrile; mobile phase C: methanol) were employed to run a gradient condition from 100 % A to 50% B and 50% C in 6.5 minutes, to 100 % B in 1 minute, 100% B for 1 minute and reequilibrate with 100 % A for 1.5 minutes. An injection volume of 10 uL was used.

### Method 2:

Reversed phase HPLC was carried out on a Chromolith (4.6 x 25 mm) with a flow rate of 3 ml/min. Three mobile phases (mobile phase A 95% 25mM ammoniumacetate + 5% acetonitrile; mobile phase B: acetonitrile; mobile phase C: methanol) were employed to run a gradient condition from 96 % A to 2% B and 2% C in 0.9 minutes, to 49 % B and 49 % C in 0.3 minute, 100% B for 0.2 minute. An injection volume of 2 uL was used.

### Method 3:

Reversed phase HPLC was carried out on a Xterra MS C18 column (3.5 mm, 4.6 x 100 mm) with a flow rate of 1.6 ml/min. Two mobile phases (mobile phase A methanol/H2O; mobile phase B 0.1 % formic acid) were employed to run a gradient condition from 100 % B to 5 % B 12 minutes. An injection volume of 10 uL was used.

### Method 4:

Reversed phase HPLC was carried out on a Xterra MS C18 column (3.5 mm, 4.6 x 100 mm) with a flow rate of 1.6 ml/min. Three mobile phases (mobile phase A 95% 25mM ammonium acetate + 5% acetonitrile; mobile phase B: acetonitrile; mobile phase C: methanol) were employed to run a gradient condition from 100 % A to 30% A, 35 % B; 35 % C in 3 minutes to 50 % B and 50% C in 3.5 minutes, to 100 % B in 0.5 minute. An injection volume of 10 uL was used.

### Method 5:

Reversed phase HPLC was carried out on a Kromasil C18 column (3.5 mm, 4.6 x 100 mm) with a flow rate of 1 ml/min. Three mobile phases (mobile phase A ammonium acetate; mobile phase B: acetonitrile; mobile phase C: formic acid) were employed to run a gradient condition from 30 % A, 40 % B, 30 % C for 1 minute to 100 % B for 5 minutes. An injection volume of 10 uL was used.

**Table : retention time (RT in minutes) and molecular weight as the MH⁺**

| **Comp. No.** | **Rt** | **MW(MH+)** | **LC/GC/MS Method** |
|---|---|---|---|
| Int. 35 | 3.84 | 317 | 1 |
| Int. 41 | 1.24 | 457 | 1 |
| Int. 40 | 6.01 | 475 | 1 |
| Int. 1 | 3.99 | 271 | 1 |
| Int. 2 | 5.31 | 487 | 1 |
| Int. 22 | 10.12 | 652 | 1 |
| Int. 25 | 9.62 | 684 | 1 |
| 10 | 6.24 | 675 | 1 |
| 16 | 6.21 | 631 | 1 |
| 22 | 5.92 | 661 | 1 |
| 23 | 5.89 | 617 | 1 |
| 17 | 8.48 | 578 | 1 |
| Int. 39 | 6.32 | 559 | 1 |
| Int. 37 | 6.47 | 373 | 1 |
| Int. 36 | 5.53 | 331 | 1 |
| Int. 3 | 4.62 | 445 | 1 |
| Int. 4 | 5.63 | 551 | 1 |
| Int. 5 | 4.39 | 601 | 1 |
| Int. 23 | 8.26 | 729 | 1 |
| Int. 21 | 1 | 531 | 2 |
| Int. 43 | 1.2 | 545 | 2 |
| Int. 42 | 1.16 | 443 | 2 |
| Int. 44 | 1.29 | 563 | 2 |
| Int. 49 | 1.12 | 573 | 2 |
| Int. 47 | 1.03 | 493 | 2 |
| 11 | 1.27 | 600 | 2 |
| 12 | 1.13 | 634 | 2 |
| Int. 51 | 1.2 | 499 | 2 |
| 14 | 1.18 | 606 | 2 |
| Int. 45 | 1.13 | 307 | 2 |
| 19 | 1.17 | 592 | 2 |
| 18 | 1.17 | 580 | 2 |
| Int. 20 | 1.05 | 575 | 2 |
| Int. 52 | 1.27 | 565 | 2 |
| Int. 48 | 0.98 | 451 | 2 |
| 20 | 7.41 | 551 | 3 |
| 8 | 9.24 | 566 | 3 |
| 21 | 8.11 | 538 | 3 |
| 13 | 6.43 | 585 | 4 |
| Int. 12 | 6.57 | 490 | 5 |
| Int. 13 | 8.62 | 474 | 5 |
| Int. 10 | 4.95 | 449 | 5 |
| Int. 15 | 6.32 | 536 | 5 |
| Int. 17 | 4.88 | 586 | 5 |

### C. Pharmacological examples

### C1 Kinase profling

The *in vitro* inhibition of a panel of kinases was assessed using the glass-fiber filter technology as described by Davies, S.P. et al., Biochem J. (2000), 351; p.95-105.

In the glass-fiber filter technology the activity of the kinase of interest is measured using an appropriate substrate that is incubated with the aforementioned kinase protein in the presence of (³³P) radiolabeled ATP. (³³P) Phosporylation of the substrate is subsequently measured as radioactivity bound on a glassfiber-filter.

### Detailed description

All kinases are pre-diluted to a 10x working concentration prior to addition into the assay. The composition of the dilution buffer for each kinase is detailed below.

| **Buffer Composition** | **Kinase(s)** |
|---|---|
| 50 mM Tris pH 7.5, 0.1 mM EGTA, 0.1 mM Na3VO4, 0.1% β-mercaptoethanol, 1 mg/ml BSA | Blk, Fyn, Lck, Lyn |
| 20 mM MOPS pH 7.0, 1 mM EDTA, 0.1% β-mercaptoethanol, 0.01% Brij-35, 5% glycerol, 1 mg/ml BSA | Abl, Bmx, EGFR, Fes, Fgr, Fms, Flt1, CDK5/p35, CDK6/cyclinD3, ErbB4, cSRC, Ret, Yes, Hck |

All substrates are dissolved and diluted to working stocks in de-ionised water.

### Abl human

In a final reaction volume of 25 µl, Abl (h) (5-10 mU) is incubated with 8 mM MOPS pH 7.0, 0.2 mM EDTA, 50 µM EAIYAAPFAKKK, 10 mM MgAcetate and [γ-³³P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Blk mouse

In a final reaction volume of 25 µl, Blk (m) (5-10 mU) is incubated with 50 mM Tris pH 7.5, 0.1 mM EGTA, 0.1 mM Na3VO4, 0.1% β-mercaptoethanol, 0.1 mg/ml poly(Glu, Tyr) 4:1, 10 mM MgAcetate and [γ-33P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Bmx human

In a final reaction volume of 25 µl, Bmx (h) (5-10 mU) is incubated with 8 mM MOPS pH 7.0, 0.2 mM EDTA, 0.1 mg/ml poly(Glu, Tyr) 4:1, 10 mM MgAcetate and [γ-33P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### CDK5/p35 human

In a final reaction volume of 25 µl, CDK5/p35 human (5-10 mU) is incubated with 8 mM MOPS pH 7.0, 0.2 mM EDTA, 0.1 mg/ml histone HI, 10 mM MgAcetate and [γ-³³P-ATP] (specific activity approx. 500cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### CDK6/cyclinD3 human

In a final reaction volume of 25 µl, CDK6/cyclinD3 human (5-10 mU) is incubated with 8 mM MOPS pH 7.0, 0.2 mM EDTA, 0.1 mg/ml histone HI, 10 mM MgAcetate and [γ-³³P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### cSRC human

In a final reaction volume of 25 µl, cSRC (h) (5-10 mU) is incubated with 8 mM MOPS pH 7.0, 0.2 mM EDTA, 250 µM KVEKIGEGTYGVVYK (Cdc2 peptide), 10 mM MgAcetate and [γ-³³P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### EGFR human

In a final reaction volume of 25 µl, EGFR (h) (5-10 mU) is incubated with 8 mM MOPS pH 7.0, 0.2 mM EDTA, 10mM MnCl2, 0.1 mg/ml poly(Glu, Tyr) 4:1, 10 mM MgAcetate and [γ-³³P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### ErbB4 human

In a final reaction volume of 25 µl, ErbB4 (h) (5-10 mU) is incubated with 8 mM MOPS pH 7.0, 0.2 mM EDTA, 10 mM MnCl2, 0.1 mg/ml poly(Glu, Tyr) 4:1, 10 mM MgAcetate and [γ-33P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Fgr human

In a final reaction volume of 25 µl, Fgr human (5-10 mU) is incubated with 8 mM MOPS pH 7.0, 0.2 mM EDTA, 0.1 mg/ml poly(Glu, Tyr) 4:1, 10 mM MgAcetate and [γ-³³P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Fyn human

In a final reaction volume of 25 µl, Fyn human (5-10 mU) is incubated with 50 mM Tris pH 7.5, 0.1 mM EGTA, 0.1 mM Na₃VO₄, 250 µM KVEKIGEGTYGVVYK (Cdc2 peptide), 10 mM MgAcetate and [γ-³³P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Lck human

In a final reaction volume of 25 µl, Lck (h) (5-10 mU) is incubated with 50 mM Tris pH 7.5, 0.1 mM EGTA, 0.1 mM Na3VO4, 250 µM KVEKIGEGTYGVVYK (Cdc2 peptide), 10 mM MgAcetate and [γ-³³P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution.
10 µl of the reaction is then spotted onto a P30 filtermat and washed three times for 5 minutes in 75 mM phosphoric acid and once in methanol prior to drying and scintillation counting.

### Lyn human

In a final reaction volume of 25 µl, Lyn (h) (5-10 mU) is incubated with 50 mM Tris pH 7.5, 0.1 mM EGTA, 0.1 mM Na3VO4, 0.1% β-mercaptoethanol, 0.1 mg/ml poly(Glu, Tyr) 4:1, 10 mM MgAcetate and [γ-³³P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Ret human

In a final reaction volume of 25 µl, Ret human (5-10 mU) is incubated with 8 mM MOPS pH 7.0, 0.2 mM EDTA, 250 µM KKKSPGEYVNIEFG, 10 mM MgAcetate and [γ-³³P-ATP] (specific activity approx. 500 cpm/pmol, concentration as required).
The reaction is initiated by the addition of the MgATP mix. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Yes human

In a final reaction volume of 25 µl, Yes (h) (5-10 mU) is incubated with 8 mM MOPS pH 7.0, 0.2 mM EDTA, 0.1 mg/ml poly(Glu, Tyr) 4:1, 10 mM MgAcetate and [γ-³³P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a Filtermat A and washed three times for 5 minutes in 75 mM phosphoric acid and once in methanol prior to drying and scintillation counting.

### Flt1 human

In a final reaction volume of 25 µl, Flt1 human (5-10 mU) is incubated with 8 mM MOPS pH 7.0, 0.2 mM EDTA, 250 µM KKKSPGEYVNIEFG, 10 mM MgAcetate and [γ-³³P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

### Hck human

In a final reaction volume of 25 µl, Hck human (5-10 mU) is incubated with 8 mM MOPS pH 7.0, 0.2 mM EDTA, 250 µM KVEKIGEGTYGVVYK (Cdc2 peptide), 10 mM MgAcetate and [γ-³³P-ATP] (specific activity approx. 500 cpm/pmol, concentration as required). The reaction is initiated by the addition of the MgATP mix. After incubation for 40 minutes at room temperature, the reaction is stopped by the addition of 5 µl of a 3% phosphoric acid solution. 10 µl of the reaction is then spotted onto a P30 filtermat and washed three times for 5 minutes in 75 mM phosphoric acid and once in methanol prior to drying and scintillation counting.

The following tables provides the scores for the compounds according to the invention, obtained at a test concentration of 10⁻⁶ M using the above mentioned kinase assays. Score 1 = 10-30% inhibition, Score 2 = 30-60% inhibition, Score 3 = 60-80% inhibition and Score 4 = > 80% inhibition.

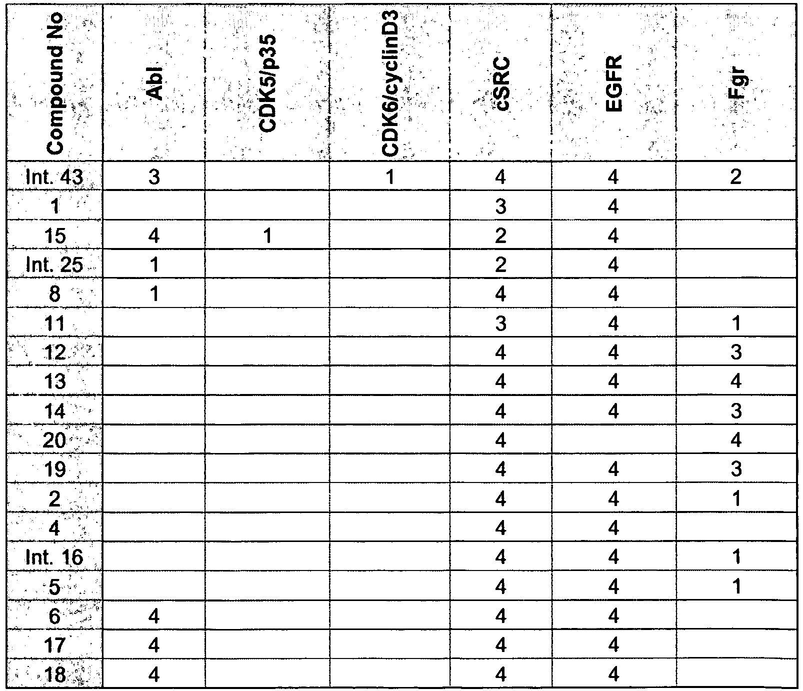

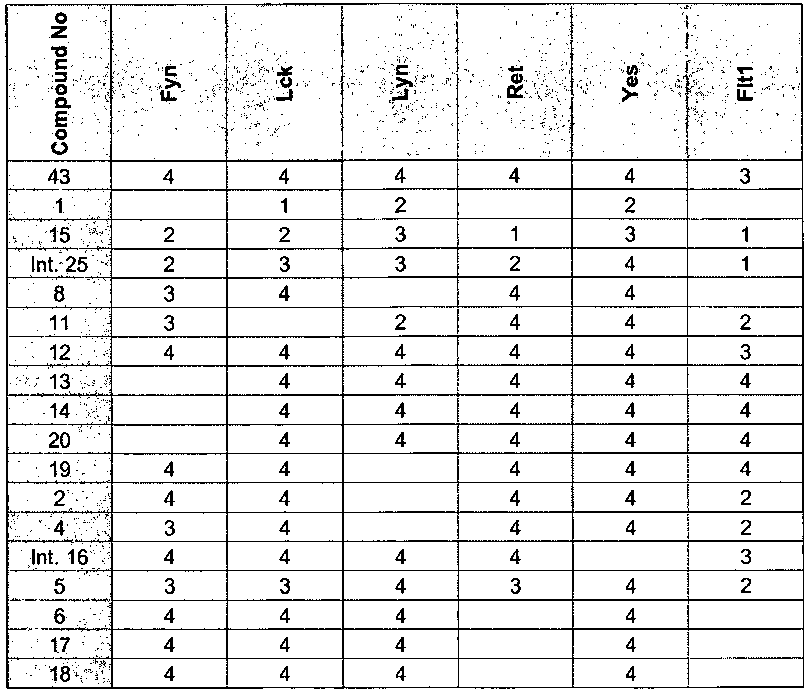

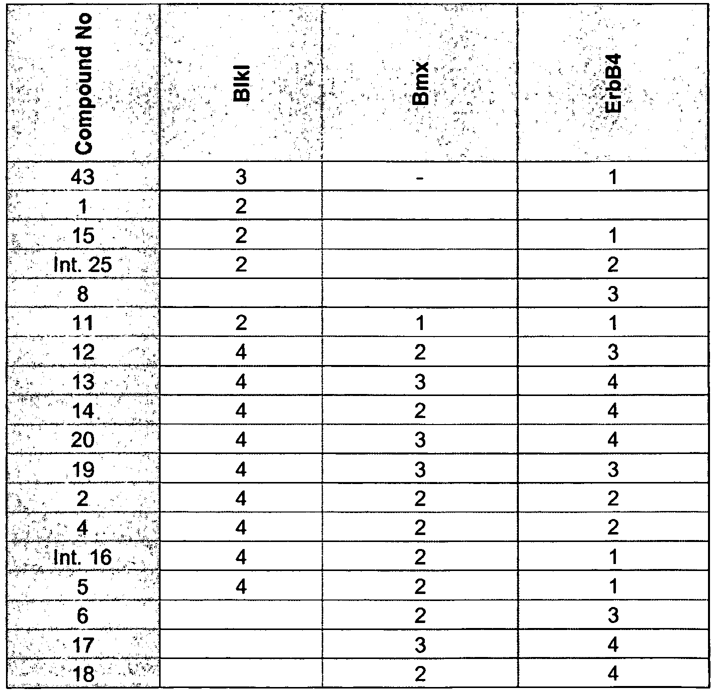

### D. Composition examples

The following formulations exemplify typical pharmaceutical compositions suitable for systemic administration to animal and human subjects in accordance with the present invention.
"Active ingredient" (A.I.) as used throughout these examples relates to a compound of formula (I) or a pharmaceutically acceptable addition salt thereof.

### Example D.1 : film-coated tablets

### Preparation of tablet core

A mixture of A.I. (100 g), lactose (570 g) and starch (200 g) was mixed well and thereafter humidified with a solution of sodium dodecyl sulfate (5 g) and polyvinyl-pyrrolidone (10 g) in about 200ml of water. The wet powder mixture was sieved, dried and sieved again. Then there was added microcrystalline cellulose (100 g) and hydrogenated vegetable oil (15 g). The whole was mixed well and compressed into tablets, giving 10.000 tablets, each comprising 10 mg of the active ingredient.

### Coating

To a solution of methyl cellulose (10 g) in denaturated ethanol (75ml) there was added a solution of ethyl cellulose (5 g) in DCM (150ml). Then there were added DCM (75ml) and 1,2,3-propanetriol (2.5ml). Polyethylene glycol (10 g) was molten and dissolved in dichloromethane (75ml). The latter solution was added to the former and then there were added magnesium octadecanoate (2.5 g), polyvinyl-pyrrolidone (5 g) and concentrated color suspension (30ml) and the whole was homogenated. The tablet cores were coated with the thus obtained mixture in a coating apparatus.

## Claims

1. A compound having the formula 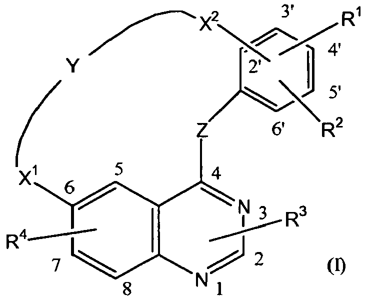 the *N-*oxide forms, the pharmaceutically acceptable addition salts and the stereochemically isomeric forms thereof, wherein
Z represents NH;
Y represents -C₃₋₉alkyl-, -C₂₋₉alkenyl-, -C₁₋₅alkyl-oxy-C₁₋₅alkyl-, -C₁₋₅alkyl-NR¹³-C₁₋₅alkyl-, -C₁₋₅alkyl-NR¹⁴-CO-C₁₋₅alkyl-, -C₁₋₆alkyl-NH-CO-, -NH-CO-C₁₋₆alkyl-, -CO-C₁₋₇alkyl-, -C₁₋₇alkyl-CO-, C₁₋₆alkyl-CO-C₁₋₆alkyl, -C₁₋₂alkyl-NR²³-CO-CR¹⁶R¹⁷-NH-, -C₁₋₂alkyl-CO-NH-CR¹⁸R¹⁹-CO-, -C₁₋₂alkyl-CO-NR²⁰-C₁₋₃alkyl-CO-, -C₁₋₂alkyl-NR²¹-CH₂-CO-NH-C₁₋₃alkyl-, -NR²²-CO-C₁₋₃alkyl-NH-, -C₁₋₃alkyl-NH-CO-Het²⁰-, C₁₋₂alkyl-CO-Het²¹-CO-, or -Het²²-CH₂-CO-NH-C₁₋₃alkyl-;
X¹ represents O, -O-C₁₋₂alkyl-, -O-N=CH-, NR¹¹ or -NR¹¹-C₁₋₂alkyl-;
X² represents a direct bond, C₁₋₂alkyl, O, -O-C₁₋₂alkyl-, CO, -CO-C₁₋₂alkyl-, -O-N=CH-, NR¹² or NR¹²-C₁₋₂alkyl-;
R¹ represents hydrogen, cyano, halo or hydroxy;
R² represents hydrogen, cyano, halo, hydroxy, hydroxycarbonyl-, C₁₋₄alkyloxycarbonyl-, Het¹⁶-carbonyl-, C₁₋₄alkyl-, C₂₋₆alkynyl-, Ar⁵, Het¹ or dihydroxyborane;
R³ represents hydrogen, cyano, halo, hydroxy, formyl, C₁₋₆alkoxy-, C₁₋₆alkyl-, C₁₋₆alkoxy- substituted with halo, or R³ represents C₁₋₄alkyl substituted with one or where possible two or more substituents selected from hydroxy or halo;
R⁴ represents Ar⁴-C₁₋₄alkyloxy-, C₁₋₄alkyloxy- or R⁴ represents C₁₋₄alkyloxy substituted with one or where possible two or more substituents selected from hydroxy-, halo, C₁₋₄alkyloxy-, C₁₋₄alkyloxy-C₁₋₄alkyloxy-, NR³⁷R³⁸-carbonyloxy-, Het⁵-carbonyloxy-, NR⁷R⁸, NR⁹R¹⁰-carbonyl-, Het³-carbonyl-, Het¹³-oxy- or Het²-;
R⁷ represents hydrogen, hydroxy-C₁₋₄alkyl- or C₁₋₄alkyl;
R⁸ represents C₃₋₆cycloalkyl; Het⁶-carbonyl-; Het⁷-aminocarbonyl-; Het⁸; Het⁹-oxycarbonyl-; Het¹⁰-sulfonyl-; C₁₋₄alkyloxycarbonyl;
mono- or di(C₁₋₄alkyl)aminocarbonyl-; mono- or di(C₁₋₄alkyl)aminocarbonyl substituted with C₁₋₄alkylsulfonyl-; or
C₁₋₄alkylcarbonyl optionally substituted with one or more substituents selected from C₁₋₄alkylsulfonyl, hydroxy- and C₁₋₄alkyloxy-; or
R⁸ represents C₁₋₄alkyl substituted with one or more substituents selected from C₁₋₄alkylsulfonyl-, NR²⁵R²⁶, aminocarbonyloxy-, C₁₋₄alkylcarbonyloxy-, aminocarbonyl-, hydroxy-C₁₋₄alkyloxy-, C₁₋₄alkyloxy-C₁₋₄alkyloxy-, and Het¹¹;
R⁹ represents hydrogen or C₁₋₄alkyl-;
R¹⁰ represents Het⁴ or C₁₋₄alkyl- substituted with C₁₋₄alkylsulfonyl-;
R¹¹ represents hydrogen, C₁₋₄alkyl- or C₁₋₄alkyl-oxy-carbonyl-;
R¹² represents hydrogen, C₁₋₄alkyl-, C₁₋₆alkyloxycarbonyl- or C₁₋₆alkyloxycarbonyl-substituted with phenyl;
R¹³ represents hydrogen, Het¹⁴-C₁₋₄alkyl, C₁₋₆alkyloxycarbonyl optionally substituted with phenyl or R¹³ represents Ar⁶-sulfonyl or Het²⁴-C₁₋₄alkylcarbonyl;
R¹⁴ and R¹⁵ are each independently selected from hydrogen, C₁₋₄alkyl, Het¹⁵-C₁₋₄alkyl- or C₁₋₄alkyloxyC₁₋₄alkyl-;
R¹⁶ and R¹⁷ each independently represents hydrogen, C₁₋₄alkyl or C₁₋₄alkyl substituted with hydroxy-, C₃₋₆cycloalkyl or phenyl; or R¹⁶ and R¹⁷ taken together with the carbon atom to which they are attached form a C₃₋₆cycloalkyl;
R¹⁸ represents hydrogen or C₁₋₄alkyl optionally substituted with hydroxy or phenyl; R¹⁹ represents hydrogen or C₁₋₄alkyl;
R²⁰ represents hydrogen or C₁₋₄alkyl;
R²¹ represents hydrogen, C₁₋₄alkyl, Het²³-C₁₋₄alkylcarbonyl- or R²¹ represents mono-or di(C₁₋₄alkyl)amino-C₁₋₄alkyl-carbonyl- optionally substituted with hydroxy, pyrimidinyl, dimethylamine or C₁₋₄alkyloxy;
R²² represents hydrogen or C₁₋₄alkyl optionally substituted with hydroxy or C₁₋₄alkyloxy;
R²³ represents C₁₋₄alkyl optionally substituted with hydroxy-, C₁₋₄alkyloxy- or Het²⁵; R²³ may also represent hydrogen when R¹⁶ and R¹⁷ taken together with the carbon atom to which they are attached form a C₃₋₆cycloalkyl;
R²⁵ and R²⁶ each independently represent hydrogen, C₁₋₄alkyl, C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R²⁷ and R²⁸ each independently represent hydrogen, C₁₋₄alkyl, C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-; or for those compounds of formula (I) wherein Het² represents a heterocycle selected from morpholinyl, piperazinyl, piperidinyl, pyrrolidinyl or thiomorpholinyl substituted with NR²⁷R²⁸-C₁₋₄alkyl said R²⁷ and R²⁸ each independently represent C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R²⁹ and R³⁰ each independently represent hydrogen, aminosulfonyl, aminocarbonyl, mono- or di(C₁₋₄alkyl)aminocarbonyl-, mono- or di(C₁₋₄alkyl)aminosulfonyl-, or C₁₋₄alkyl- optionally substituted with one or more substituents selected from NR³¹R³², C₁₋₄alkylsulfonyl, aminocarbonyloxy-, hydroxy-, C₁₋₄alkyloxy-, aminocarbonyl- and mono- or di(C₁₋₄alkyl)aminocarbonyl-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-;
R³¹ and R³² each independently represent hydrogen, C₁₋₄alkyl, C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R³³ represents hydrogen or C₁₋₄alkyl;
R³⁴ represents C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R³⁵ represents hydrogen or C₁₋₄alkyl;
R³⁶ represents C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R³⁷ and R³⁸ each independently represent hydrogen, C₁₋₄alkyl, C₁₋₄alkylsulfonyl-, Het¹² or C₁₋₄alkyl substituted with one or more substituents selected from C₁₋₄alkylsulfonyl-, hydroxy- and C₁₋₄alkyloxy-;
R³⁹ and R⁴⁰ each independently represent aminosulfonyl, aminocarbonyl, mono- or di(C₁₋₄alkyl)aminocarbonyl-, mono- or di(C₁-₄alkyl)aminosulfonyl-, or C₁₋₄alkyl- substituted with one or more substituents selected from NR³¹R³², C₁₋₄alkylsulfonyl, aminocarbonyloxy-, hydroxy-, C₁₋₄alkyloxy-, aminocarbonyl- and mono- or di(C₁₋₄alkyl)aminocarbonyl-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-;
Het¹ represents thiazolyl or 2-bora-1,3-dioxolanyl wherein said Het¹ is optionally substituted with one or where possible two, three, four or more substituents selected from amino, C₁₋₄alkyl, hydroxy-C₁₋₄alkyl-, phenyl, phenyl-C₁₋₄alkyl-, C₁₋₄alkyl-oxy-C₁₋₄alkyl- mono- or di(C₁₋₄alkyl)amino- or amino-carbonyl-;
Het² represents a heterocycle selected from tetrahydropyranyl, tetrahydrofuranyl, furanyl, 1,1-dioxothiomorpholinyl, piperazininonyl, tetrahydro-1,1-dioxido-2H-thiopyranyl, piperidinonyl, azetidinyl or 2-azetidinonyl wherein said Het² is optionally substituted with one or where possible two or more substituents selected from hydroxy, amino, NR²⁹R³⁰, aminocarbonyl, mono- or di(C₁₋₄alkyl)aminocarbonyl, C₁₋₄alkylsulfonyl or
C_{I}-₄alkyl- optionally substituted with one or more substituents selected from NR²⁷R²⁸, C₁₋₄alkylsulfonyl, aminocarbonyloxy-, aminocarbonyl- and mono- or di(C₁₋₄alkyl)aminocarbonyl-, or
C₁₋₄alkyloxy- optionally substituted with C₁₋₄alkyloxy-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-; or
Het² represents a heterocycle selected from morpholinyl, piperazinyl, piperidinyl, pyrrolidinyl, thiomorpholinyl or 1,1-dioxothiomorpholinyl wherein said Het² is optionally substituted with one or where possible two or more substituents selected from
C₁₋₄alkyl- optionally substituted with one or more substituents selected from NR²⁷R²⁸, C₁₋₄alkylsulfonyl, aminocarbonyloxy-, aminocarbonyl- and mono- or di(C₁₋₄alkyl)aminocarbonyl-, or
C₁₋₄alkyloxy- optionally substituted with C₁₋₄alkyloxy-, or C₁₋₄alkyloxycarbonyl optionally substituted with one or more substituents selected from hydroxy, C₁₋₄alkyloxy- and C₁₋₄alkylsulfonyl-, or
C₁-₄alkylcarbonyl optionally substituted with one or more substituents selected from hydroxy-, C₁₋₄alkyloxy- and C₁₋₄alkylsulfonyl-;
Het³ represents a heterocycle selected from tetrahydropyranyl, tetrahydrofuranyl, furanyl, 1,1-dioxothiomorpholinyl, piperazininonyl, tetrahydro-1,1-dioxido-2H-thiopyranyl, piperidinonyl, azetidinyl or 2-azetidinonyl wherein said Het³ is optionally substituted with one or where possible two or more substituents hydroxy-, amino, C₁₋₄alkyl-, C₃₋₆cycloalkyl-C₁₋₄alkyl-, aminosulfonyl-, mono- or di(C₁₋₄alkyl)aminosulfonyl-, amino-C₁₋₄alkyl-, Mono- or di(C₁₋₄alkyl)amino-C₁₋₄alkyl, NR³⁵R³⁶, C₁₋₄alkyl-sulfonyl-C₁₋₄alkyl- or C₁₋₄alkyloxy- optionally substituted with C₁₋₄alkyloxy- or hydroxy; or
Het³ represents a heterocycle selected from morpholinyl, piperazinyl, piperidinyl, furanyl or pyrrolidinyl wherein said Het³ is substituted with one or where possible two or more substituents selected from NR³⁵R³⁶, C₁₋₄alkyl-sulfonyl-C₁₋₄alkyl- or C₁₋₄alkyloxy- optionally substituted with C₁₋₄alkyloxy- or hydroxy;
Het⁴ represents a heterocycle selected from morpholinyl, piperazinyl, piperidinyl, furanyl, pyrazolyl, dioxolanyl, thiazolyl, oxazolyl, imidazolyl, isoxazolyl, oxadiazolyl, pyridinyl or pyrrolidinyl wherein said Het⁴ is substituted with one or where possible two or more substituents selected from C₁₋₄alkyl-sulfonyl-C₁₋₄alkyl-, C₁₋₄alkyloxy- optionally substituted with C₁₋₄alkyloxy- or hydroxy;
Het⁵ represents a heterocycle selected from furanyl, piperazinyl, 1,1-dioxothiomorpholinyl, piperazininonyl, piperidinyl, tetrahydro-1,1-dioxido-2H-thiopyranyl, piperidinonyl, morpholinyl or pyrrolidinyl wherein said Het⁵ is optionally substituted with hydroxy, amino, mono- or di(C₁₋₄alkyl)-amino-, C₁₋₄alkyl,
Het⁶ and Het⁷ each independently represents a heterocycle selected from piperazinyl, piperidinyl or pyrrolidinyl wherein said heterocycles are optionally substituted with one or more substituents selected from hydroxy-, amino, hydroxy-C₁₋₄alkyl-, C₁₋₄alkyloxy-C₁₋₄alkyl- and C₁₋₄alkyl-;
Het⁸ represents a heterocycle selected from tetrahydropyranyl, tetrahydrofuranyl, 1,1 -dioxothiomorpholinyl, piperazininonyl, tetrahydro-1,1 -dioxido-2H-thiopyranyl, piperidinonyl, azetidinyl or 2-azetidinonyl wherein said Het⁸ is optionally substituted with aminosulfonyl, aminocarbonyl,
mono- or di(C₁₋₄alkyl)aminocarbonyl-, mono- or di(C₁₋₄alkyl)aminosulfonyl-, or C₁₋₄alkyl- optionally substituted with one or more substituents selected from amino, mono- or di(C₁₋₄alkyl)amino-, NR³³R³⁴, C₁₋₄alkylsulfonyl, aminocarbonyloxy-, hydroxy-, C₁₋₄alkyloxy-, aminocarbonyl- and mono- or di(C₁₋₄alkyl)aminocarbonyl-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy, C₁₋₄alkyloxy- and C₁₋₄alkylsulfonyl-; or
Het⁸ represents a heterocycle selected from furanyl, piperidinyl or piperazinyl wherein said Het⁸ is substituted with aminocarbonyl,
mono- or di(C₁₋₄alkyl)aminocarbonyl-, mono- or di(C₁₋₄alkyl)aminosulfonyl-, or
C₁₋₄alkyl- substituted with one or more substituents selected from NR³³R³⁴, C₁₋₄alkylsulfonyl, aminocarbonyloxy-, hydroxy-, C₁₋₄alkyloxy-, aminocarbonyl- and mono- or di(C₁₋₄alkyl)aminocarbonyl-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy, C₁₋₄alkyloxy- and C₁₋₄alkylsulfonyl-;
Het⁹ and Het¹⁰ each independently represents a heterocycle selected from piperazinyl, piperidinyl or pyrrolidinyl wherein said heterocycles are optionally substituted with one or more substituents selected from hydroxy-, amino, hydroxy-C₁₋₄alkyl-, C₁₋₄alkyloxy-C₁₋₄alkyl- and C₁₋₄alkyl-;
Het¹¹ represents 2-imidazolidinonyl- or 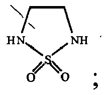 Het¹² represents a heterocycle selected from morpholinyl, piperazinyl, piperidinyl or pyrrolidinyl wherein said Het¹² is optionally substituted with one or where possible two or more substituents selected from hydroxy, amino or C₁₋₄alkyl-;
Het¹³ represents a heterocycle selected from furanyl, piperazinyl, 1,1-dioxothiomorpholinyl, piperazininonyl, piperidinyl, tetrahydro-1,1-dioxido-2H-thiopyranyl, piperidinonyl, morpholinyl, piperazinyl or pyrrolidinyl;
Het¹⁴ and Het¹⁵ each independently represent a heterocycle selected from morpholinyl, piperazinyl, piperidinyl or pyrrolidinyl wherein said Het¹⁴ and Het¹⁵ are optionally substituted with one or where possible two or more substituents selected from hydroxy, amino or C₁₋₄alkyl;
Het¹⁶ represents a heterocycle selected from piperidinyl or pyrrolidinyl;
Het²⁰ represents pyrrolidinyl, 2-pyrrolidinonyl, piperidinyl or hydroxy-pyrrolidinyl;
Het²¹ represents pyrrolidinyl or hydroxy-pyrrolidinyl;
Het²² represents pyrrolidinyl, piperazinyl or piperidinyl;
Het²³ and Het²⁵ each independently represents a heterocycle selected from morpholinyl, pyrrolidinyl, piperazinyl or piperidinyl wherein said Het²³ is optionally substituted with one or where possible two or more substituents selected from C₁₋₄alkyl, C₃₋₆cycloalkyl, hyclroxy-C₁₋₄alkyl-, C₁₋₄alkyloxyC₁₋₄alkyl or polyhydroxy-C₁₋₄alkyl-;
Het²⁴ represents morpholinyl, pyrrolidinyl, piperazinyl or piperidinyl;
Ar⁴, Ar⁵ or Ar⁶ each independently represent phenyl optionally substituted with nitro, cyano, C₁₋₄alkylsulfonyl-, C₁₋₄alkylsulfonylamino-, aminosulfonylamino-, hydroxy-C₁₋₄alkyl, aminosulfonyl-, hydroxy-, C₁₋₄alkyloxy- or C₁₋₄alkyl;
further **characterised in that** either
Y represents -C₁₋₂alkyl-NR²³-CO-CR¹⁶R¹⁷-NH-;
Het¹ represents 2-bora-1,3-dioxolanyl optionally substituted with one or where possible two, three, four or more substituents selected from amino, C₁₋₄alkyl, hydroxy-C₁₋₄alkyl-, phenyl, phenyl-C₁₋₄alkyl-, C₁₋₄alkyl-oxy-C₁₋₄alkyl- mono-or di(C₁₋₄alkyl)amino- or amino-carbonyl-;
R¹³ represents C₁₋₆alkyloxycarbonyl optionally substituted with phenyl or R¹³ represents Ar⁶-sulfonyl or Het²⁴-C₁₋₄alkylcarbonyl; or
R⁴ represents C₁₋₄alkyloxy substituted with at least one substituent selected from C₁₋₄alkyloxy-C₁₋₄alkyloxy-, NR³⁷R³⁸-carbonyloxy-, Het⁵-carbonyloxy-, NR⁷R⁸, NR⁹R¹⁰-carbonyl-, Het³-carbonyl-, Het¹³-oxy- or Het²-; wherein
R⁸ represents Het⁷-aminocarbonyl-; Het⁹-oxycarbonyl-; Het¹⁰-sulfonyl-; C₁₋₄alkyloxycarbonyl; mono- or di(C₁₋₄alkyl)aminocarbonyl-; mono- or di(C₁₋₄alkyl)aminocarbonyl substituted with C₁₋₄alkylsulfonyl-; or C₁₋₄alkylcarbonyl optionally substituted with one or more substituents selected from C₁₋₄alkylsulfonyl, hydroxy- and C₁₋₄alkyloxy-; or
R⁸ represents C₁₋₄alkyl substituted with one or more substituents selected from C₁₋₄alkylsulfonyl-, NR²⁵R²⁶, aminocarbonyloxy-, C₁₋₄alkylcarbonyloxy-, aminocarbonyl-, C₁₋₄alkyloxy-C₁₋₄alkyloxy-, and Het¹¹, and
Het² represents a heterocycle selected from morpholinyl, piperazinyl, piperidinyl, pyrrolidinyl or thiomorpholinyl said Het² substituted with one or where possible two or more substituents selected from C₁₋₄alkyl- substituted with one or more substituents selected from NR²⁷R²⁸, C₁₋₄alkylsulfonyl, aminocarbonyloxy-, aminocarbonyl- and mono- or di(C₁₋₄alkyl)aminocarbonyl-; or
C₁₋₄alkyloxy- optionally substituted with C₁₋₄alkyloxy-; or C₁₋₄alkyloxycarbonyl optionally substituted with one or more substituents selected from hydroxy, C₁₋₄alkyloxy- and C₁₋₄alkylsulfonyl-; or C₁₋₄alkylcarbonyl optionally substituted with one or more substituents selected from hydroxy-, C₁₋₄alkyloxy- and C₁₋₄alkylsulfonyl-;
or Het² represents 1,1-dioxothiomorpholinyl optionally substituted with C₁₋₄alkyl- optionally substituted with one or more substituents selected from NR²⁷R²⁸, C₁₋₄alkylsulfonyl, aminocarbonyloxy-, aminocarbonyl- and mono- or di(C₁₋₄alkyl)aminocarbonyl-; or
C₁₋₄alkyloxy- optionally substituted with C₁₋₄alkyloxy-; or C₁₋₄alkyloxycarbonyl optionally substituted with one or more substituents selected from hydroxy, C₁₋₄alkyloxy- and C₁₋₄alkylsulfonyl-; or C₁₋₄alkylcarbonyl optionally substituted with one or more substituents selected from hydroxy-, C₁₋₄alkyloxy- and C₁₋₄alkylsulfonyl-.

2. A compound according to claim 1 wherein;
Z represents NH;
Y represents -C₃₋₉alkyl-, -C₁₋₅alkyl-NR¹³-C₁₋₅alkyl-, -C₁₋₅alkyl-NR¹⁴-CO-C₁₋₅alkyl-, -C₁₋₆alkyl-CO-NH-, -C_{I}-₆alkyl-NH-CO-, -C₁₋₂alkyl-NR²³-CO-CR¹⁶R¹⁷-NH-, -C₁₋₂alkyl-NR²¹-CH₂-CO-NH-C₁₋₃alkyl or C₁₋₃alkyl-NH-CO-Het²⁰-;
X¹ represents O, -O-C₁₋₂alkyl-, NR¹¹, or -NR¹¹-C₁₋₂alkyl-;
X² represents a direct bond, -C₁₋₂alkyl-, CO-C₁₋₂alkyl or NR¹²-C₁₋₂alkyl-;
R¹ represents hydrogen, cyano, halo or hydroxy;
R² represents hydrogen, halo, cyano, C₂-₆alkynyl, hydroxy, hydroxycarbonyl, C₁₋₄alkyloxycarbonyl- or Het¹;
R³ represents hydrogen, cyano, halo, hydroxy, formyl, C₁₋₆alkyloxy or C₁₋₆alkyloxy-substituted with halo;
R⁴ represents Ar⁴-C₁₋₄alkyloxy, C₁₋₄alkyloxy-, or C₁₋₄alkyloxy- substituted with one or where possible two or more substituents selected from hydroxy, C₁₋₄alkyloxy-, C₁₋₄alkyloxy-C₁₋₄alkyloxy, NR⁷R⁸ or Het²;
R⁷ represents hydrogen, hydroxyC₁₋₄alkyl- or C₁₋₄alkyl;
R⁸ represents C₁₋₄alkyloxycarbonyl or C₁₋₄alkyl- substituted with one or more substituents selected from C₁₋₄alkylsulfonyl-, C₁₋₄alkylcarbonyloxy or NR²⁵R²⁶; in particular R⁸ represents C₁₋₄alkyl- substituted with one or more substituents selected from C₁₋₄alkylsulfonyl- or NR²⁵R²⁶;
R¹¹ represents hydrogen, C₁₋₄alkyloxycarbonyl or C₁₋₄alkyl;
R¹² represents hydrogen or C₁₋₄alkyl;
R¹³ represents C₁₋₆alkyloxycarbonyl optionally substituted with phenyl or R¹³ represents Ar⁶-sulfonyl or Het²⁴-C₁₋₄alkylcarbonyl;
R¹⁴ and R¹⁵ each independently represent hydrogen or C₁₋₄alkyl;
R¹⁶ and R¹⁷ each independently represent hydrogen or C₁₋₄alkyl optionally substituted with C₃₋₆cycloalkyl or R¹⁶ and R¹⁷ taken together with the carbon atom to which they are attached form a C₃₋₆cycloalkyl;
R²¹ represents hydrogen;
R²³ represents C₁₋₄alkyl optionally substituted with hydroxy-, C₁₋₄alkyloxy- or Het²⁵; R²³ may also represent hydrogen when R¹⁶ and R¹⁷ taken together with the carbon atom to which they are attached form a C₃₋₆cycloalkyl;
R²⁵ and R²⁶ each independently represent hydrogen, C₁₋₄alkyl, C₁₋₄alkylsulfonyl, C₁₋₄alkyloxycarbonyl or C₁₋₄alkylcarbonyl;
R²⁷ and R²⁸ each independently represent hydrogen, C₁₋₄alkyl, C₁₋₄alkylsulfonyl, C₁₋₄alkyloxycarbonyl or C₁₋₄alkylcarbonyl;
Het¹ represents 2-bora-1,3-dioxolanyl- optionally substituted with one or where possible two, three, four or more substituents selected from amino, C₁₋₄alkyl, hydroxy-C₁₋₄alkyl-, phenyl, phenyl-C₁₋₄alkyl, C₁₋₄alkyloxyC₁₋₄alkyl-, mono- or di(C₁₋₄alkyl)amino- or aminocarbonyl-;
Het² represents 1,1-dioxothiomorpholinyl optionally substituted with C₁₋₄alkyloxycarbonyl or C₁₋₄alkyl-NR²⁷R²⁸ ; or Het² represents piperidinyl or piperazinyl substituted with C₁₋₄alkyloxycarbonyl or -C₁₋₄alkyl-NR²⁷R²⁸;
Het²⁰ represents pyrrolidinyl, 2-pyrrolidinonyl, piperidinyl or hydroxy-pyrrolidinyl;
Het²⁵ represents a heterocycle selected from morpholinyl or piperazinyl wherein said heterocycle is optionally substituted with C₁₋₄alkyl, hydroxy-C₁₋₄alkyl, C₁₋₄alkyloxy-C₁₋₄allcyl or polyhydroxy-C₁₋₄alkyl; or
Ar⁴, Ar⁵ or Ar⁶ each independently represents phenyl optionally substituted with nitro, cyano, hydroxy, hydroxyC₁₋₄alkyl, C₁₋₄alkyl or C₁₋₄alkyloxy;
further **characterised in that** either
Y represents -C₁-₂alkyl-NR²³-CO-CR¹⁶R¹⁷-NH-; or
R⁴ represents C₁₋₄alkyloxy substituted with at least one substituent selected from C₁₋₄alkyloxy-C₁₋₄alkyloxy-, NR⁷R⁸ or Het².

3. A compound according to claims 1 or 2 wherein;
Z represents NH;
Y represents -C₃₋₉alkyl-, -C₁₋₅alkyl-NR¹³-C₁₋₅alkyl-, -C₁₋₅alkyl-NR¹⁴-CO-C₁₋₅alkyl-, -C₁₋₆alkyl-CO-NH-, -C₁₋₆alkyl-NH-CO-, -C₁₋₂alkyl-NR²³-CO-CR¹⁶R17-NH-, -C₁₋₂alkyl-NR²¹-CH₂-CO-NH-C₁₋₃alkyl or C₁₋₃alkyl-NH-CO-Het²⁰-;
X¹ represents O, -O-C₁₋₂alkyl-, NR¹¹, or -NR¹¹-C₁₋₂alkyl-;
X² represents a direct bond, -C₁₋₂alkyl-, CO-C₁₋₂alkyl orNR¹²-C₁₋₂alkyl-;
R¹ represents hydrogen or halo;
R² represents hydrogen, halo, C₂₋₆alkynyl, cyano or Het¹;
R³ represents hydrogen;
R⁴ represents Ar⁴-C₁₋₄alkyloxy, C₁₋₄alkyloxy-, or C₁₋₄alkyloxy- substituted with one or where possible two or more substituents selected from hydroxy, C₁₋₄alkyloxy-, C₁₋₄alkyloxy-C₁₋₄alkyloxy, NR⁷R⁸ or Het²;
R⁷ represents hydrogen or C₁₋₄alkyl;
R⁸ represents C₁₋₄alkyloxycarbonyl or C₁₋₄alkyl- substituted with one or more substituents selected from C₁₋₄alkylsutfonyl-, C₁₋₄alkylcarbonyloxy or NR²⁵R²⁶
R¹¹ represents hydrogen or C₁₋₄alkyl;
R¹² represents hydrogen or C₁₋₄alkyl;
R¹³ represents Ar⁶-sulfonyl or C₁₋₆alkyloxycarbonyl optionally substituted with phenyl;
R¹⁴ and R¹⁵ represent hydrogen;
R¹⁶ and R¹⁷ each independently represent hydrogen or C₁₋₄alkyl optionally substituted with C₃₋₆cycloalkyl or R¹⁶ and R¹⁷ taken together with the carbon atom to which they are attached form a C₃₋₆cycloalkyl;
R²¹ represents hydrogen;
R²³ represents C₁₋₄alkyl optionally substituted with hydroxy-, C₁₋₄alkyloxy- or Het²⁵; R²³ may also represent hydrogen when R¹⁶ and R¹⁷ taken together with the carbon atom to which they are attached form a C₃₋₆cycloalkyl;
R²⁵ and R²⁶ each independently represent hydrogen or C₁₋₄alkylcarbonyl;
R²⁷ and R²⁸ each independently represent hydrogen or C₁₋₄alkylcarbonyl;
Het¹ represents 2-bora-1,3-dioxolanyl-;
Het² represents 1,1-dioxothiomorpholinyl, piperidinyl or piperazinyl wherein said Het² is optionally substituted with C₁₋₄alkyloxycarbonyl or -C₁₋₄allcyl-NR²⁷R²⁸;
Het²⁰ represents pyrrolidinyl;
Het²⁵ represents a heterocycle selected from morpholinyl or piperazinyl wherein said heterocycle is optionally substituted with C₁₋₄alkyl, hydroxy-C₁₋₄alkyl, C₁₋₄alkyloxy-C₁₋₄alkyl or polyhydroxy-C₁₋₄alkyl;
Ar⁴ represents phenyl;
Ar⁵ represents phenyl; or
Ar⁶ represents phenyl optionally substituted with nitro;
further **characterised in that** either
Y represents -C₁₋₄alkyl-NR²³-CO-CR¹⁶R¹⁷-NH-; or
R⁴ represents C₁₋₄alkyloxy substituted with at least one substituent selected from C₁₋₄alkyloxy-C₁₋₄alkyloxy-, NR⁷R⁸ or Het²; in particular C₁₋₄alkyloxy substituted with C₁₋₄alkyloxy-C₁₋₄alkyloxy- or NR⁷R⁸.

4. A compound according to any one of claims 1 to 3 wherein;
Z represents NH;
Y represents -C₃₋₉alkyl-,-C₁₋₅alkyl-NR¹³-C₁₋₅alkyl-, -C₁₋₅alkyl-NR¹⁴-CO-C₁₋₅alkyl-, -C₁₋₂alkyl-NR²¹-CH₂-CO-NH-C₁₋₃alkyl- or -C₁₋2alkyl-NR²³-CO-CR¹⁶R¹⁷-NH-;
X¹ represents O or -O-C₁₋₂alkyl-;
X² represents a direct bond, C₁₋₂alkyl, -CO-C₁₋₂alkyl or NR¹²-C₁₋₂alkyl;
R¹ represents hydrogen or halo; in particular R¹ represents hydrogen;
R² represents halo, acetylene or Het¹; in particular R² represents halo or Het¹;
R³ represents hydrogen;
R⁴ represents Ar^{a}-C₁₋₄alkyloxy-, C₁₋₄alkyloxy- or C₁₋₄alkyloxy substituted with one or where possible two or more substituents selected from Het², NR⁷R⁸, hydroxy and C₁₋₄alkyloxy-C₁₋₄alkyloxy-;
R⁷ represents hydrogen or C₁₋₄alkyl;
R⁸ represents C₁₋₄alkyl substituted with NR²⁵R²⁶ or C₁₋₄alkylsulfonyl;
R¹² represents hydrogen or C₁₋₄alkyl-;
R¹³ represents Ar⁶-sulfonyl or C₁₋₆alkyloxycarbonyl optionally substituted with phenyl;
R¹⁶ and R¹⁷ represents hydrogen, C₁₋₄alkyl or R¹⁶ and R¹⁷ taken together with the carbon atom to which they are attached from a C₃₋₆cycloalkyl;
R²³ represents hydrogen or C₁₋₄alkyl;
R²⁵ and R²⁶ each independently represent hydrogen or C₁₋₄alkylcarbonyl;
R²⁷ and R²⁸ each independently represent hydrogen or C₁₋₄alkylcarbonyl;
Het¹ represents 2-bora-1,3-dioxolanyl;
Het² represents piperidinyl, piperazinyl, morpholinyl, thiomorpholinyl or 1,1-dioxothiomorpholinyl wherein said Het² is optionally substituted with C₁₋₄alkyloxycarbonyl or NR²⁷R²⁸-C₁₋₄alkyl;
Ar⁴ represents phenyl;
Ar⁵ represents phenyl; or
Ar⁶ represents phenyl optionally substituted with nitro.

5. A compound according to any one of claims 1 to 3 wherein;
Z represents NH;
Y represents -C₃₋₉alkyl-,-C₁₋₅alkyl-NR¹³-C₁₋₅alkyl- or -C₁₋₂alkyl-NR²³-CO-CR¹⁶R¹⁷-NH-;
X¹ represents O; X² represents a direct bond or NR¹²-C₁₋₂alkyl-;
R¹ represents hydrogen; R² represents halo or Het¹; R³ represents hydrogen;
R⁴ represents Ar⁴-C₁₋₄alkyloxy-, C₁₋₄alkyloxy- or C₁₋₄alkyloxy substituted with C₁₋₄alkyloxy-C₁₋₄alkyloxy-;
R¹² represents hydrogen or C₁₋₄alkyl-;
R¹³ represents Ar⁶-sulfonyl or C₁₋₆alkyloxycarbonyl optionally substituted with phenyl;
R¹⁶ and R¹⁷ taken together with the carbon atom to which they are attached from a C₃₋₆cycloalkyl;
R²³ represents hydrogen or C₁₋₄alkyl;
Het¹ represents 2-bora-1,3-dioxolanyl;
Ar⁴ represents phenyl; Ar⁵ represents phenyl;
Ar⁶ represents phenyl optionally substituted with nitro.

6. A compound according to claim 1 wherein
Z represents NH;
Y represents -C₃₋₉alkyl-, -C₂₋₉alkenyl-, -C₁₋₅alkyl-oxy-C₁₋₅alkyl-, -C₁₋₅alkyl-NR¹³-C₁₋₅alkyl-, -C₁₋₅alkyl-NR¹⁴-CO-C₁₋₅alkyl-, -C₁₋₆alkyl-NH-CO-, -NH-CO-C₁₋₆alkyl-, -CO-C₁₋₇alkyl-, -C₁₋₇alkyl-CO-, C₁₋₆alkyl-CO-C₁₋₆alkyl, -C₁₋₂alkyl-NR²³-CO-CR¹⁶R¹⁷-NH-, -C₁₋₂alyl-CO-NH-CR¹⁸R¹⁹-CO-, -C₁₋₂alkyl-CO-NR²⁰-C₁₋₃alkyl-CO-, -C₁₋₂alkyl-NR²¹-CH₂-CO-NH-C₁₋₃alkyl-, -NR²²-CO-C₁₋₃alkyl-NH-, -C₁₋₃alkyl-NH-CO-Het²⁰-, C₁₋₂alkyl-CO-Het²¹-CO-, or -Het²²-CH₂-CO-NH-C₁₋₃alkyl-;
X¹ represents O, -O-C₁₋₂alkyl-, -O-N=CH-, NR¹¹ or -NR11-C₁₋₂alkyl-;
X² represents a direct bond, C₁₋₂alkyl, O, -O-C₁₋₂alkyl-, CO, -CO-C₁₋₂alkyl-, -O-N=CH-, NR¹² or NR¹²-C₁₋₂alkyl-;
R¹ represents hydrogen, cyano, halo or hydroxy;
R² represents hydrogen, cyano, halo, hydroxy, hydroxycarbonyl-, C₁₋₄alkyloxycarbonyl-, Het¹⁶-carbonyl-, C₁₋₄alkyl-, C₂₋₆alkynyl-, Ar⁵, Het¹ or dihydroxyborane;
R³ represents hydrogen, cyano, halo, hydroxy, formyl, C₁₋₆alkoxy-, C₁₋₆alkyl-, C₁₋₆alkoxy- substituted with halo, or R³ represents C₁₋₄alkyl substituted with one or where possible two or more substituents selected from hydroxy or halo;
R⁴ represents Ar⁴-C₁₋₄alkyloxy-, C₁₋₄alkyloxy- or R⁴ represents C₁₋₄alkyloxy substituted with one or where possible two or more substituents selected from hydroxy-, halo, C₁₋₄alkloxy-, C₁₋₄alkyloxy-C₁₋₄alkyloxy-, NR³⁷R³⁸-carbonyloxy-, Het⁵-carbonyloxy-, NR⁷R⁸, NR⁹R¹⁰-carbonyl-, Het³-carbonyl-, Het¹³-oxy- or Het²-;
R⁷ represents hydrogen or C₁₋₄alkyl;
R⁸ represents C₃₋₆cycloalkyl, Het⁶-carbonyl-, Het⁷-aminocarbonyl-, Het⁸, Het⁹-oxycarbonyl-, Het¹⁰-sulfonyl-, mono- or di(C₁₋₄alkyl)aminocarbonyl-, mono- or di(C₁₋₄alkyl)aminocarbonyl substituted with C₁₋₄alkylsulfonyl-, or C₁₋₄alkylcarbonyl optionally substituted with one or more substituents selected from C₁₋₄alkylsulfonyl, hydroxy- and C₁₋₄alkyloxy-, or R⁸ represents C₁₋₄alkyl substituted with one or more substituents selected from C₁₋₄alkylsulfonyl-, NR²⁵R²⁶, aminocarbonyloxy-, aminocarbonyl-, C₁₋₄alyloxy-C₁₋₄alkyloxy-, and Het¹¹;
R⁹ represents hydrogen or C₁₋₄alkyl-;
R¹⁰ represents Het⁴ or C₁₋₄alkyl- substituted with C₁₋₄alkylsulfonyl-,
R¹¹ represents hydrogen, C₁₋₄alkyl- or C₁₋₄alkyl-oxy-carbonyl-;
R¹⁰ represents hydrogen, C₁₋₄alkyl-, C₁₋₆alkyloxycarbonyl- or C₁-₆alkyloxycarbonyl- substituted with phenyl;
R¹³ represents hydrogen, Het¹⁴ -C₁₋₄alkyl, C₁₋₆allcyloxycarbonyl optionally substituted with phenyl or R¹³ represents Ar⁶-sulfonyl or Het²⁴-C₁₋₄alkylcarbonyl;
R¹⁴ and R¹⁵ are each independently selected from hydrogen, C₁₋₄alkyl, Het¹⁵ -C₁₋₄alkyl- or C₁₋₄alkyloxyC₁₋₄alkyl-;;
R¹⁶ and R¹⁷ each independently represents hydrogen, C₁₋₄alkyl or C₁₋₄alkyl substituted with hydroxy- or phenyl; or R¹⁶ and R¹⁷ taken together with the carbon atom to which they are attached form a C₃₋₆cycloalkyl;
R¹⁸ represents hydrogen or C₁₋₄alkyl optionally substituted with hydroxy or phenyl;
R¹⁹ represents hydrogen or C₁₋₄alkyl;
R²⁰ represents hydrogen or C₁₋₄alkyl;
R²¹ represents hydrogen, C₁₋₄alkyl, Het²³-C₁₋₄alkylcarbonyl- or R²¹ represents mono-or di(C₁₋₄alkyl)amino-C₁₋₄alkyl-carbonyl- optionally substituted with hydroxy, pyrimidinyl, dimethylamine or C₁₋₄alkyloxy;
R²² represents hydrogen or C₁₋₄alkyl optionally substituted with hydroxy or C₁₋₄alkyloxy;
R²³ represents C₁₋₄alkyl optionally substituted with hydroxy-, C₁₋₄alkyloxy- or Het²³; R²³ may also represent hydrogen when R¹⁶ and R¹⁷ taken together with the carbon atom to which they are attached form a C₃₋₆cycloalkyl;
R²⁵ and R²⁶ each independently represent hydrogen, C₁₋₄alkyl, C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R²⁷ and R²⁸ each independently represent hydrogen, C₁₋₄alkyl, C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R²⁹ and R³⁰ each independently represent hydrogen, aminosulfonyl, aminocarbonyl, mono- or di(C₁₋₄alkyl)aminocarbonyl-, mono- or di(C₁-₄alkyl)aminosulfonyl-, or
C₁₋₄alkyl- optionally substituted with one or more substituents selected from NR³¹R³², C₁₋₄alkylsulfonyl, aminocarbonyloxy-, hydroxy-, C₁₋₄alkyloxy-, aminocarbonyl- and mono- or di(C₁₋₄alkyl)aminocarbonyl-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-;
R³¹ and R³² each independently represent hydrogen, C₁₋₄alkyl, C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R³³ represents hydrogen or C₁₋₄alkyl;
R³⁴ represents C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R³⁵ represents hydrogen or C₁₋₄alkyl;
R3⁶ represents C₁₋₄alkylsulfonyl-, aminocarbonyl-, mono- or di(C₁₋₄alkyl)aminocarbonyl-, C₁₋₄alkylcarbonyl-, C₁₋₄alkyloxycarbonyl- or C₁₋₄alkyl substituted with one or more substituents selected from C₁₋₄alkylsulfonyl-, hydroxy- and C₁₋₄alkyloxy-;
R³⁷ and R³⁸ each independently represent hydrogen, C₁₋₄alkyl, C₁₋₄alkylsulfonyl-, Het¹² or C₁₋₄alkyl substituted with one or more substituents selected from C₁₋₄alkylsulfonyl-, hydroxy- and C₁₋₄alkyloxy-;
R³⁹ and R⁴⁰ each independently represent aminosulfonyl, aminocarbonyl, mono- or di(C₁₋₄alkyl)aminocarbonyl-, mono- or di(C₁₋₄alkyl)aminosulfonyl-, or
C₁₋₄alkyl- substituted with one or more substituents selected from NR³¹R³², C₁₋₄alkylsulfonyl, aminocarbonyloxy-, hydroxy-, C₁₋₄alkyloxy-, aminocarbonyl- and mono- or di(C₁₋₄alkyl)aminocarbonyl-, or
C₁₋₄alkyloxycarbonyl optionally substituted with one or more substituents selected from hydroxy, C₁₋₄akloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-;
Het¹ represents thiazolyl or 2-bora-1,3-dioxolanyl wherein said Het¹ is optionally substituted with one or where possible two, three, four or more substituents selected from amino, C₁₋₄alkyl, hydroxy-C₁-₄alkyl-, phenyl, phenyl-C₁₋₄alkyl-, C₁₋₄alkyl-oxy-C₁₋₄alkyl-, mono- or di(C₁₋₄alkyl)amino- or amino-carbonyl-;
Het² represents a heterocycle selected from tetrahydropyranyl, tetrahydrofuranyl, furanyl, 1,1-dioxothiomorpholinyl, piperazininonyl, tetrahydro-1,1-dioxido-2H-thiopyranyl, piperidinonyl, azetidinyl or 2-azetidinonyl wherein said Het² is optionally substituted with one or where possible two or more substituents selected from hydroxy, amino, NR²⁹R³⁰, aminocarbonyl, mono- or di(C₁₋₄alkyl)aminocarbonyl, C₁₋₄alkylsulfonyl or
C₁₋₄alkyl- optionally substituted with one or more substituents selected from NR²⁷R²⁸, C₁₋₄alkylsulfonyl, aminocarbonyloxy-, aminocarbonyl- and mono- or di(C₁₋₄alkyl)aminocarbonyl-, or
C₁₋₄alkyloxy- optionally substituted with C₁₋₄alkyloxy-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-; or
Het² represents a heterocycle selected from morpholinyl, piperazinyl, piperidinyl or pyrrolidinyl wherein said morpholinyl, piperazinyl, piperidinyl or pyrrolidinyl are optionally substituted with one or where possible two or more substituents selected from
C₁₋₄alkyl- optionally substituted with one or more substituents selected from NR²⁷R²⁸, C₁₋₄alkylsulfonyl, aminocarbonyloxy-, aminocarbonyl- and mono- or di(C₁₋₄alkyl)aminocarbonyl-, or
C₁₋₄alkyloxy- optionally substituted with C₁₋₄alkyloxy-, or
C₁₋₄alkyloxycarbonyl optionally substituted with one or more substituents selected from hydroxy, C₁₋₄alkyloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-;
Het³ represents a heterocycle selected from tetrahydropyranyl, tetrahydrofuranyl, furanyl, 1,1-dioxothiomorpholinyl, piperazininonyl, tetrahydro-1,1-dioxido-2H-thiopyranyl, piperidinonyl, azetidinyl or 2-azetidinonyl wherein said Het³ is optionally substituted with one or where possible two or more substituents hydroxy-, amino, C₁₋₄alkyl-, C₃₋₆cycloalkyl-C₁₋₄alkyl-, aminosulfonyl-, mono-or di(C₁₋₄alkyl)aminosulfonyl-, amino-C₁₋₄alkyl-, Mono- or di(C₁₋₄alkyl)amino-C₁₋₄alkyl, NR³⁵R³⁶, C₁₋₄alkyl-sulfonyl-C₁₋₄alkyl- or C₁₋₄alkyloxy- optionally substituted with C₁₋₄alkyloxy- or hydroxy; or
Het³ represents a heterocycle selected from morpholinyl, piperazinyl, piperidinyl or pyrrolidinyl wherein said Het³ is optionally substituted with one or where possible two or more substituents selected from NR³⁵R³⁶, C₁₋₄alkyl-sulfonyl-C₁₋₄alkyl- or C₁₋₄alkyloxy- optionally substituted with C₁₋₄alkyloxy- or hydroxy;
Het⁴ represents a heterocycle selected from morpholinyl, piperazinyl, piperidinyl, furanyl, pyrazolyl, dioxolanyl, thiazolyl, oxazolyl, imidazolyl, isoxazolyl, oxadiazolyl, pyridinyl or pyrrolidinyl wherein said Het⁴ is substituted with one or where possible two or more substituents selected from C₁₋₄alkyl-sulfonyl-C₁₋₄alkyl-, C₁₋₄alkyloxy- optionally substituted with C₁₋₄alkyloxy- or hydroxy;
Het⁵ represents a heterocycle selected from furanyl, piperazinyl, 1,1-dioxothiomorpholinyl, piperazininonyl, piperidinyl, tetrahydro-1,1-dioxido-2H-thiopyranyl, piperidinonyl, morpholinyl or pyrrolidinyl wherein said Het⁵ is optionally substituted with hydroxy, amino, mono- or di(C₁₋₄alkyl)-amino-,
C₁₋₄alkyl,
Het⁶ and Het⁷ each independently represents a heterocycle selected from piperazinyl, piperidinyl or pyrrolidinyl wherein said heterocycles are optionally substituted with one or more substituents selected from hydroxy-, amino-, hydroxy-C₁₋₄alkyl-, C₁₋₄alkyloxy-C₁₋₄alkyl- and C₁₋₄alkyl-;
Het⁸ represents a heterocycle selected from tetrahydropyranyl, tetrahydrofuranyl, 1,1-dioxothiomorpholinyl, piperazininonyl, tetrahydro-1,1-dioxido-2H-thiopyranyl, piperidinonyl, azetidinyl or 2-azetidinonyl wherein said Het⁸ is optionally substituted with aminosulfonyl, aminocarbonyl,
mono- or di(C₁₋₄alkyl)aminocarbonyl-, mono- or di(C₁₋₄alkyl)aminosulfonyl-, or
C₁₋₄alkyl- optionally substituted with one or more substituents selected from amino, mono- or di(C₁₋₄alkyl)amino-, NR³³R³⁴, C₁₋₄alkylsulfbnyl, aminocarbonyloxy-, hydroxy-, C₁₋₄alkyloxy-, aminocarbonyl- and mono- or di(C₁₋₄alkyl)aminocarbonyl-, or
C₁₋₄alkyloxycarbonyl optionally substituted with one or more substituents selected from hydroxy-, C₁₋₄alkyloxy- and C₁₋₄alkylsulfonyl-, or C₁₋₄alkylcarbonyl optionally substituted with one or more substituents selected from hydroxy-, C₁₋₄alkyloxy- and C₁₋₄alkylsulfonyl-; or
Het⁸ represents a heterocycle selected from furanyl, piperidinyl or piperazinyl wherein said Het⁸ is substituted with aminocarbonyl,
mono- or di(C₁₋₄alkyl)aminocarbonyl-, mono- or di(C₁₋₄alkyl)aminosulfonyl-, or
C₁₋₄alkyl- substituted with one or more substituents selected from NR³³R³⁴, C₁₋₄alkylsulfonyl, aminocarbonyloxy-, hydroxy-, C₁₋₄alkyloxy-, aminocarbonyl- and mono- or di(C₁₋₄alkyl)aminocarbonyl-, or
C₁₋₄alkyloxycarbonyl optionally substituted with one or more substituents selected from hydroxy-, C₁₋₄alkyloxy- and C₁₋₄alkylsulfonyl-, or
C₁₋₄alkylcarbonyl optionally substituted with one or more substituents selected from hydroxy-, C₁₋₄alkyloxy- and C₁₋₄alkylsulfonyl-;
Het⁹ and Het¹⁰ each independently represents a heterocycle selected from piperazinyl, piperidinyl or pyrrolidinyl wherein said heterocycles are optionally substituted with one or more substituents selected from hydroxy-, amino, hydroxy-C₁₋₄alkyl-, C₁₋₄alkyloxy-C₁₋₄alkyl- and C₁₋₄alkyl-;
Het¹¹ represents 2-imidazolidinonyl- or 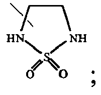 Het¹² represents a heterocycle selected from morpholinyl, piperazinyl, piperidinyl or pyrrolidinyl wherein said Het¹² is optionally substituted with one or where possible two or more substituents selected from hydroxy-, amino or C₁₋₄alkyl-;
Het¹³ represents a heterocycle selected from furanyl, piperazinyl, 1,1-dioxothiomorpholinyl, piperazininonyl, piperidinyl, tetrahydro-1,1-dioxido-2H-thiopyranyl, piperidinonyl, morpholinyl, piperazinyl or pyrrolidinyl
Het¹⁶ represents a heterocycle selected from piperidinyl or pyrrolidinyl;
Het²⁰ represents pyrrolidinyl, 2-pyrrolidinonyl, piperidinyl or hydroxy-pyrrolidinyl, preferably pyrrolidinyl or hydroxy-pyrrolidinyl;
Het²¹ represents pyrrolidinyl or hydroxy-pyrrolidinyl;
Het²² represents pyrrolidinyl, piperazinyl or piperidinyl;
Het²³ represents a heterocycle selected from morpholinyl, pyrrolidinyl, piperazinyl or piperidinyl wherein said Het²³ is optionally substituted with one or where possible two or more substituents selected from C₁₋₄alkyl, C₃₋₆cycloalkyl, hydroxy-
C₁₋₄alkyl-, C₁₋₄alkyloxyC₁₋₄alkyl or polyhydroxy-C₁₋₄alkyl-;
Ar⁴, Ar⁵ or Ar⁶ each independently represent phenyl optionally substituted with nitro, cyano, C₁₋₄alkylsulfonyl-, C₁₋₄alkylsulfonylamino-, aminosulfonylamino-, hydroxy-C₁₋₄alkyl, aminosulfonyl-, hydroxy-, C₁₋₄alkyloxy- or C₁₋₄alkyl, preferably Ar⁴ or Ar⁵ each independently represent phenyl optionally substituted with cyano.

7. A compound according to any one of claims 1 to 6 wherein X² substituent is at position 2', the R¹ substituent represents hydrogen or halo and is at position 4', the R² substituent represents halo and is at position 5', the R³ substituent is at position 2 and the R⁴ substituent at position 7 of the structure of formula (I).

8. A compound according to any one of claims 1 to 4 or 6 to 7 wherein
R⁴ represents C₁₋₄alkyloxy substituted with hydroxy and one substituent selected from NR⁷R⁸ or Het²-.

9. A kinase inhibitor of formula (I) as defined in claim 1.

10. A compound as claimed in any one of claims 1 to 8 for use as a medicine.

11. Use of a compound as claimed in any one of claims 1 to 8 in the manufacture of a medicament for treating cell proliferative disorders such as atherosclerosis, restenosis and cancer.

12. A pharmaceutical composition comprising a pharmaceutically acceptable carrier and, as active ingredient, an effective kinase inhibitory amount of a compound as described in any one of the claims 1 to 8.

## Patentansprüche

1. Verbindung der Formel 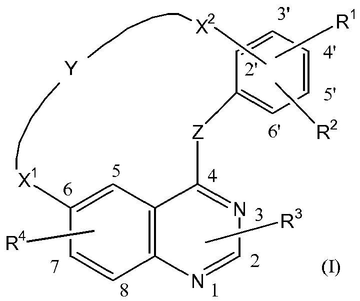 die N-Oxid-Formen, die pharmazeutisch unbedenklichen Additionssalze und die stereochemisch isomeren Formen davon, wobei
Z für NH steht;
Y für -C₃₋₉-Alkyl-, -C₂₋₉-Alkenyl-, -C₁₋₅-Alkyloxy-C₁₋₅-alkyl-, -C₁₋₅-Alkyl-NR¹³-C₁₋₅-alkyl-, -C₁₋₅-Alkyl-NR¹⁴-CO-C₁₋₅-alkyl-, -C₁₋₆-Alkyl-NH-CO-, -NH-CO-C₁₋₆-Alkyl-, -CO-C₁₋₇-Alkyl-, -C₁₋₇-Alkyl-CO-, C₁₋₆-Alkyl-CO-C₁₋₆-alkyl, -C₁₋₂-Alkyl-NR²³-CO-CR¹⁶R¹⁷-NH-, -C₁₋₂-Alkyl-CO-NH-CR¹⁸R¹⁹-CO-, -C₁₋₂-Alkyl-CO-NR²⁰-C₁₋₃-alkyl-CO-, -C₁₋₂-Alkyl-NR²¹-CH₂-CO-NH-C₁₋₃-alkyl-, -NR²²-CO-C₁₋₃-Alkyl-NH-, -C₁₋₃-Alkyl-NH-CO-Het²⁰-, C₁₋₂-Alkyl-CO-Het²¹-CO- oder -Het²²-CH₂-CO-NH-C₁₋₃-Alkyl- steht;
X¹ für O, -O-C₁₋₂-Alkyl-, -O-N=CH-, NR¹¹ oder -NR¹¹-C₁₋₂-Alkyl- steht;
X² für eine direkte Bindung, C₁₋₂-Alkyl, O, -O-C₁₋₂-Alkyl-, CO, -CO-C₁₋₂-Alkyl-, -O-N=CH-, NR¹² oder NR¹²-C₁₋₂-Alkyl- steht;
R¹ für Wasserstoff, Cyano, Halogen oder Hydroxy steht;
R² für Wasserstoff, Cyano, Halogen, Hydroxy, Hydroxycarbonyl-, C₁₋₄-Alkyloxycarbonyl-, Het¹⁶-Carbonyl-, C₁₋₄-Alkyl-, C₂₋₆-Alkinyl-, Ar⁵, Het¹ oder Dihydroxyboran steht;
R³ für Wasserstoff, Cyano, Halogen, Hydroxy, Formyl, C₁₋₆-Alkoxy-, C₁₋₆-Alkyl-, C₁₋₆-Alkoxy-, das durch Halogen substituiert ist, oder für C₁₋₄-Alkyl, das durch einen oder, sofern möglich, zwei oder mehr Substituenten, die aus Hydroxy oder Halogen ausgewählt sind, substituiert ist, steht;
R⁴ für Ar⁴-C₁₋₄⁻Alkyloxy-, C₁₋₄-Alkyloxy- oder für C₁-₄-Alkyloxy, das durch einen oder, sofern möglich, zwei oder mehr Substituenten, die aus Hydroxy-, Halogen, C₁₋₄-Alkyloxy-, C₁₋₄-Alkyloxy-C₁₋₄-alkyl-oxy-, NR³¹R³⁸-Carbonyloxy-, Het⁵-Carbonyloxy-, NR⁷R⁸, NR⁹R¹⁰-Carbonyl-, Het³-Carbonyl-, Het¹³-Oxy- oder Het²- ausgewählt sind, substituiert ist, steht;
R⁷ für Wasserstoff, Hydroxy-C₁₋₄-alkyl- oder C₁₋₄-Alkyl steht;
R⁸ für C₃₋₆-Cycloalkyl; Het⁶-Carbonyl-; Het⁷-Aminocarbonyl-; Het⁸; Het⁹-Oxycarbonyl-; Het¹⁰-Sulfonyl-; C₁₋₄-Alkyloxycarbonyl; Mono- oder Di(C₁₋₄-alkyl)aminocarbonyl-; Mono- oder Di(C₁₋₄-alkyl)aminocarbonyl, das durch C₁₋₄-Alkylsulfonyl-substituiert ist; oder C₁₋₄-Alkylcarbonyl, das gegebenenfalls durch einen oder mehrere Substituenten, die aus C₁₋₄-Alkylsulfonyl, Hydroxy- und C₁₋₄-Alkyloxy- ausgewählt sind, substituiert ist; oder für C₁₋₄-Alkyl, das durch einen oder mehrere Substituenten, die aus C₁₋₄-Alkylsulfonyl-, NR²⁵R²⁶, Aminocarbonyloxy-, C₁₋₄-Alkylcarbonyloxy-, Aminocarbonyl-, Hydroxy-C₁₋₄-alkyloxy-, C₁₋₄-Alkyloxy-C₁₋₄-alkyloxy- und Het¹¹ ausgewählt sind, substituiert ist, steht;
R⁹ für Wasserstoff oder C₁₋₄-Alkyl- steht;
R¹⁰ für Het⁴ oder C₁₋₄-Alkyl-, das durch C₁₋₄-Alkylsulfonyl- substituiert ist, steht;
R¹¹ für Wasserstoff, C₁₋₄-Alkyl- oder C₁₋₄-Alkyloxy-carbonyl- steht;
R¹² für Wasserstoff, C₁₋₄-Alkyl-, C₁₋₆-Alkyloxy-carbonyl- oder C₁₋₆-Alkyloxycarbonyl-, das durch Phenyl substituiert ist, steht;
R¹³ für Wasserstoff, Het¹⁴-C₁₋₄-Alkyl, C₁₋₆-Alkyloxy-carbonyl, das gegebenenfalls durch Phenyl substituiert ist, oder für Ar⁶-Sulfonyl oder Het²⁴-C₁₋₄-Alkylcarbonyl steht;
R¹⁴ und R¹⁵ jeweils unabhängig aus Wasserstoff, C₁₋₄-Alkyl, Het¹⁵-C₁₋₄-Alkyl- oder C₁₋₆-Alkyloxy-C₁₋₄-alkyl ausgewählt sind;
R¹⁶ und R¹⁷ jeweils unabhängig für Wasserstoff, C₁₋₄-Alkyl oder C₁₋₄-Alkyl, das durch Hydroxy-, C₃₋₆-Cycloalkyl oder Phenyl substituiert ist, stehen oder R¹⁶ und R¹⁷ zusammen mit dem Kohlenstoffatom, an das sie gebunden sind, ein C₃₋₆-Cycloalkyl bilden;
R¹⁸ für Wasserstoff oder C₁₋₄-Alkyl, das gegebenenfalls durch Hydroxy oder Phenyl substituiert ist, steht;
R¹⁹ für Wasserstoff oder C₁₋₄-Alkyl steht;
R²⁰ für Wasserstoff oder C₁₋₄-Alkyl steht;
R²¹ für Wasserstoff, C₁₋₄-Alkyl, Het²³-C₁₋₄-Alkylcarbonyl oder für Mono- oder Di(C₁₋₄alkyl)amino-C₁₋₄-alkylcarbonyl-, das gegebenenfalls durch Hydroxy, Pyrimidinyl, Dimethylamin oder C₁₋₄-Alkyloxy substituiert ist, steht;
R²² für Wasserstoff oder C₁₋₄-Alkyl, das gegebenenfalls durch Hydroxy oder C₁₋₄-Alkyloxy substituiert ist, steht;
R²³ für C₁₋₄-Alkyl, das gegebenenfalls durch Hydroxy-, C₁₋₄-Alkyloxy oder Het²⁵ substituiert ist, steht; R²³ auch für Wasserstoff stehen kann, wenn R¹⁶ und R¹⁷ zusammen mit dem Kohlenstoffatom, an das sie gebunden sind, ein C₃₋₆-Cycloalkyl bilden;
R²⁵ und R²⁶ jeweils unabhängig für Wasserstoff, C₁₋₄-Alkyl, C₁₋₄-Alkylsulfonyl-, Aminocarbonyl-, Mono- oder Di(C₁₋₄-alkyl)aminocarbonyl-, C₁₋₄-Alkyl-carbonyl-, C₁₋₄-Alkyloxycarbonyl- oder C₁₋₄-Alkyl, das durch einen oder mehrere Substituenten, die aus C₁₋₄-Alkylsulfonyl-, Hydroxy- und C₁₋₄-Alkyloxyausgewählt sind, substituiert ist, stehen;
R²⁷ und R²⁸ unabhängig voneinander für Wasserstoff, C₁₋₄-Alkyl, C₁₋₄-Alkylsulfonyl-, Aminocarbonyl-, Mono- oder Di(C₁₋₄-alkyl) aminocarbonyl-, C₁₋₄-Alkylcarbonyl-, C₁₋₄-Alkyloxycarbonyl- oder C₁₋₄-Alkyl, das durch einen oder mehrere Substituenten, die aus C₁₋₄-Alkylsulfonyl-, Hydroxy- und C₁₋₄-Alkyloxy- ausgewählt sind, substituiert ist, stehen; oder für diejenigen Verbindungen der Formel (I), wobei Het² für einen aus Morpholinyl, Piperazinyl, Piperidinyl, Pyrrolidinyl oder Thiomorpholinyl ausgewählten Heterocyclus, der durch NR²⁷R²⁸-C₁₋₄-Alkyl substituiert ist, steht, das R²⁷ und R²⁸ jeweils unabhängig für C₁₋₄-Alkylsulfonyl-, Aminocarbonyl-, Mono- oder Di(C₁₋₄-alkyl)aminocarbonyl-, C₁₋₄-Alkylcarbonyl-, C₁₋₄-Alkyloxycarbonyl- oder C₁₋₄-Alkyl, das durch einen oder mehrere Substituenten, die aus C₁₋₄-Alkylsulfonyl-, Hydroxy- und C₁₋₄-Alkyloxy- ausgewählt sind, substituiert ist, stehen;
R²⁹ und R³⁰ jeweils unabhängig für Wasserstoff, Aminosulfonyl, Aminocarbonyl, Mono- oder Di(C₁₋₄-alkyl)aminocarbonyl-, Mono- oder Di (C₁₋₄-alkyl) aminosulfonyl- oder C₁₋₄-Alkyl-, das gegebenenfalls durch einen oder mehrere Substituenten, die aus NR³¹R³², C₁₋₄-Alkylsulfonyl, Aminocarbonyloxy-, Hydroxy-, C₁₋₄-Alkyloxy-, Aminocarbonyl- und Mono- oder Di(C₁₋₄-alkyl)-aminocarbonyl- ausgewählt sind, substituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonylausgewählt sind, substituiert ist, oder C₁₋₄-Alkylcarbonyl, das gegebenenfalls durch einen oder mehrere Substituenten, die aus Hydroxy-, C₁₋₄-Alkyloxy- und C₁₋₄-Alkylsulfonyl- ausgewählt sind, substituiert ist, stehen;
R³¹ und R³² jeweils unabhängig für Wasserstoff, C₁₋₄-Alkyl, C₁₋₄-Alkylsulfonyl-, Aminocarbonyl-, Mono- oder Di(C₁₋₄-alkyl)aminocarbonyl-, C₁₋₄-Alkyl-carbonyl-, C₁₋₄-Alkyloxycarbonyl- oder C₁₋₄-Alkyl, das durch einen oder mehrere Substituenten, die aus C₁₋₄-Alkylsulfonyl-, Hydroxy- und C₁₋₄-Alkyloxy-ausgewählt sind, substituiert ist, stehen;
R³³ für Wasserstoff oder C₁₋₄-Alkyl steht;
R³⁴ für C₁₋₄-Alkylsulfonyl-, Aminocarbonyl-, Mono- oder Di(C₁₋₄-alkyl)aminocarbonyl-, C₁₋₄-Alkyl-carbonyl-, C₁₋₄-Alkyloxycarbonyl- oder C₁₋₄-Alkyl, das durch einen oder mehrere Substituenten, die aus C₁₋₄-Alkylsulfonyl-, Hydroxy- und C₁₋₄-Alkyloxy-ausgewählt sind, substituiert ist, steht;
R³⁵ für Wasserstoff oder C₁₋₄-Alkyl steht;
R³⁶ für C₁₋₄-Alkylsulfonyl-, Aminocarbonyl-, Mono- oder Di(C₁₋₄-alkyl)aminocarbonyl-, C₁₋₄-Alkyl-carbonyl-, C₁₋₄-Alkyloxycarbonyl- oder C₁₋₄-Alkyl, das durch einen oder mehrere Substituenten, die aus C₁₋₄-Alkylsulfonyl-, Hydroxy- und C₁₋₄-Alkyloxy-ausgewählt sind, substituiert ist, steht;
R³⁷ und R³⁸ jeweils unabhängig für Wasserstoff, C₁₋₄-Alkyl, C₁₋₄-Alkylsulfonyl-, Het¹² oder C₁₋₄-Alkyl, das durch einen oder mehrere Substituenten, die aus C₁₋₄-Alkylsulfonyl-, Hydroxy- und C₁₋₄-Alkyloxy-ausgewählt sind, substituiert ist, stehen;
R³⁹ und R⁴⁰ jeweils unabhängig für Aminosulfonyl, Aminocarbonyl, Mono- oder Di (C₁₋₄-alkyl)-aminocarbonyl-, Mono- oder Di (C₁₋₄-alkyl)-aminosulfonyl- oder C₁₋₄-Alkyl-, das durch einen oder mehrere Substituenten, die aus NR³¹R³², C₁₋₄-Alkylsulfonyl, Aminocarbonyloxy-, Hydroxy-, C₁₋₄-Alkyloxy-, Aminocarbonyl- und Mono- oder Di(C₁₋₄-alkyl)aminocarbonyl- ausgewählt sind, substituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonyl-ausgewählt sind, substituiert ist, oder C₁₋₄-Alkylcarbonyl, das gegebenenfalls durch einen oder mehrere Substituenten, die aus Hydroxy-, C₁₋₄-Alkyloxy- und C₁₋₄-Alkylsulfonyl- ausgewählt sind, substituiert ist, stehen;
Het¹ für Thiazolyl oder 2-Bora-1,3-dioxolanyl steht, wobei das Het¹ gegebenenfalls durch einen oder, sofern möglich, zwei, drei, vier oder mehr Substituenten, die aus Amino, C₁₋₄-Alkyl, Hydroxy-C₁₋₄-alkyl-, Phenyl, Phenyl-C₁₋₄-alkyl-, C₁₋₄-Alkyloxy-C₁₋₄-alkyl-, Mono- oder Di (C₁₋₄-alkyl)amino- oder Aminocarbonyl- ausgewählt sind, substituiert ist;
Het² für einen aus Tetrahydropyranyl, Tetrahydrofuranyl, Furanyl, 1,1-Dioxothiomorpholinyl, Piperazininonyl, Tetrahydro-1,1-dioxido-2H-thiopyranyl, Piperidinonyl, Azetidinyl oder 2-Azetidinonyl ausgewählten Heterocyclus steht, wobei das Het² gegebenenfalls durch einen oder, sofern möglich, zwei oder mehr Substituenten, die aus Hydroxy, Amino, NR²⁹R³⁰, Aminocarbonyl, Mono- oder Di (C₁₋₄-alkyl) aminocarbonyl, C₁₋₄-Alkylsulfonyl oder C₁₋₄-Alkyl-, das gegebenenfalls durch einen oder mehrere Substituenten, die aus NR²⁷R²⁸, C₁₋₄-Alkylsulfonyl, Aminocarbonyloxy-, Aminocarbonyl- und Mono- oder Di(C₁₋₄-alkyl)aminocarbonyl- ausgewählt sind, substituiert ist, oder C₁₋₄-Alkyloxy-, das gegebenenfalls durch C₁₋₄-Alkyloxy-substituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonyl- ausgewählt sind, substituiert ist, oder C₁₋₄-Alkylcarbonyl, das gegebenenfalls durch einen oder mehrere Substituenten, die aus Hydroxy-, C₁₋₄-Alkyloxy- und C₁₋₄-Alkylsulfonyl-ausgewählt sind, substituiert ist, ausgewählt sind, substituiert ist; oder
Het² für einen aus Morpholinyl, Piperazinyl, Piperidinyl, Pyrrolidinyl, Thiomorpholinyl oder 1,1-Dioxothiomorpholinyl ausgewählten Heterocyclus steht, wobei das Het² gegebenenfalls durch einen oder, sofern möglich, zwei oder mehr Substituenten, die aus C₁₋₄-Alkyl-, das gegebenenfalls durch einen oder mehrere Substituenten, die aus NR²⁷R²⁸, C₁₋₄-Alkylsulfonyl, Aminocarbonyloxy-, Aminocarbonyl- und Mono- oder Di(C₁₋₄-alkyl)aminocarbonyl- ausgewählt sind, substituiert ist, oder C₁₋₄-Alkyloxy-, das gegebenenfalls durch C₁₋₄-Alkyloxy- substituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonyl-ausgewählt sind, substituiert ist, oder C₁₋₄-Alkylcarbonyl, das gegebenenfalls durch einen oder mehrere Substituenten, die aus Hydroxy-, C₁₋₄-Alkyloxy- und C₁₋₄-Alkylsulfonyl- ausgewählt sind, substituiert ist, ausgewählt sind, substituiert ist;
Het³ für einen aus Tetrahydropyranyl, Tetrahydrofuranyl, Furanyl, 1,1-Dioxothiomorpholinyl, Piperazininonyl, Tetrahydro-1,1-dioxido-2H-thiopyranyl, Piperidinonyl, Azetidinyl oder 2-Azetidinonyl ausgewählten Heterocyclus steht, wobei das Het³ gegebenenfalls durch einen oder, sofern möglich, zwei oder mehr Substituenten, die aus Hydroxy-, Amino, C₁₋₄-Alkyl-, C₃₋₆-Cycloalkyl-C₁₋₄-alkyl-, Aminosulfonyl-, Mono- oder Di (C₁₋₄-alkyl)aminosulfonyl-, Amino-C₁₋₄-alkyl-, Mono- oder Di (C₁₋₄-alkyl) amino-C₁₋₄-alkyl, NR³⁵R³⁶, C₁₋₄-Alkylsulfonyl-C₁₋₄-alkyl- oder C₁₋₄-Alkyloxy-, das gegebenenfalls durch C₁₋₄-Alkyloxy- oder Hydroxy substituiert ist, ausgewählt sind, substituiert ist; oder
Het³ für einen aus Morpholinyl, Piperazinyl, Piperidinyl, Furanyl oder Pyrrolidinyl ausgewählten Heterocyclus steht, wobei das Het³ durch einen oder, sofern möglich, zwei oder mehr Substituenten, die aus NR³⁵R³⁶, C₁₋₄-Alkylsulfonyl-C₁₋₄-alkyl- oder C₁₋₄-Alkyloxy-, das gegebenenfalls durch C₁₋₄-Alkyloxy- oder Hydroxy substituiert ist, ausgewählt sind, substituiert ist;
Het⁴ für einen aus Morpholinyl, Piperazinyl, Piperidinyl, Furanyl, Pyrazolyl, Dioxolanyl, Thiazolyl, Oxazolyl, Imidazolyl, Isoxazolyl, Oxadiazolyl, Pyridinyl oder Pyrrolidinyl ausgewählten Heterocyclus steht, wobei das Het⁴ durch einen oder, sofern möglich, zwei oder mehr Substituenten, die aus C₁₋₄-Alkylsulfonyl-C₁₋₄-alkyl- oder C₁₋₄-Alkyloxy-, das gegebenenfalls durch C₁₋₄-Alkyloxy- oder Hydroxy substituiert ist, ausgewählt sind, substituiert ist;
Het⁵ für einen aus Furanyl, Piperazinyl, 1,1-Dioxothiomorpholinyl, Piperazininonyl, Piperidinyl, Tetrahydro-1,1-dioxido-2H-thio-pyranyl, Piperidinonyl, Morpholinyl oder Pyrrolidinyl ausgewählten Heterocyclus steht, wobei das Het⁵ gegebenenfalls durch Hydroxy, Amino, Mono- oder Di (C₁₋₄alkyl) -amino-, C₁₋₄-Alkyl substituiert ist;
Het⁶ und Het⁷ jeweils unabhängig für einen aus Piperazinyl, Piperidinyl oder Pyrrolidinyl ausgewählten Heterocyclus stehen, wobei die Heterocyclen gegebenenfalls durch einen oder mehrere Substituenten, die aus Hydroxy, Amino, Hydroxy-C₁₋₄-alkyl-, C₁₋₄-Alkyloxy-C₁₋₄-alkyl- und C₁₋₄-Alkyl- ausgewählt sind, substituiert sind;
Het⁸ für einen aus Tetrahydropyranyl, Tetrahydrofuranyl, 1,1-Dioxothiomorpholinyl, Piperazininonyl, Tetrahydro-1,1-dioxido-2H-thiopyranyl, Piperidinonyl, Azetidinyl oder 2-Azetidinonyl ausgewählten Heterocyclus steht, wobei das Het⁸ gegebenenfalls durch Aminosulfonyl, Aminocarbonyl, Mono- oder Di(C₁₋₄-alkyl)amino-carbonyl-, Mono- oder Di(C₁₋₄-alkyl)aminosulfonyl- oder C₁₋₄-Alkyl-, das gegebenenfalls durch einen oder mehrere Substituenten, die aus Amino, Mono- oder Di(C₁₋₄-alkyl)amino-, NR³³R³⁴, C₁₋₄-Alkyl-sulfonyl, Aminocarbonyloxy-, Hydroxy-, C₁₋₄-Alkyloxy-, Aminocarbonyl- und Mono- oder Di(C₁₋₄-alkyl)aminocarbonyl- ausgewählt sind, substituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonylausgewählt sind, substituiert ist, oder C₁₋₄-Alkylcarbonyl, das gegebenenfalls durch einen oder mehrere Substituenten, die aus Hydroxy, C₁₋₄-Alkyloxy- und C₁₋₄-Alkylsulfonyl- ausgewählt sind, substituiert ist, substituiert ist; oder
Het⁸ für einen aus Furanyl, Piperidinyl oder Piperazinyl ausgewählten Heterocyclus steht, wobei das Het⁸ durch Aminocarbonyl, Mono- oder Di(C₁₋₄-alkyl) aminocarbonyl-, Mono- oder Di(C₁₋₄-alkyl)-aminosulfonyl- oder C₁₋₄-Alkyl-, das durch einen oder mehrere Substituenten, die aus NR³³R³⁴, C₁₋₄-Alkylsulfonyl, Aminocarbonyloxy-, Hydroxy-, C₁₋₄-Alkyloxy-, Aminocarbonyl- und Mono- oder Di(C₁₋₄-alkyl)aminocarbonyl- ausgewählt sind, substituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonylausgewählt sind, substituiert ist, oder C₁₋₄-Alkylcarbonyl, das gegebenenfalls durch einen oder mehrere Substituenten, die aus Hydroxy, C₁₋₄-Alkyloxy- und C₁₋₄-Alkylsulfonyl- ausgewählt sind, substituiert ist, substituiert ist;
Het⁹ und Het¹⁰ jeweils unabhängig für einen aus Piperazinyl, Piperidinyl oder Pyrrolidinyl ausgewählten Heterocyclus stehen, wobei die Heterocyclen gegebenenfalls durch einen oder mehrere Substituenten, die aus Hydroxy-, Amino, Hydroxy-C₁₋₄-alkyl-, C₁₋₄-Alkyloxy-C₁₋₄-alkyl- und C₁₋₄-Alkyl- ausgewählt sind, substituiert sind;
Het¹¹ für 2-Imidazolidinonyl oder 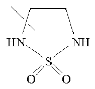 steht;
Het¹² für einen aus Morpholinyl, Piperazinyl, Piperidinyl oder Pyrrolidinyl ausgewählten Heterocyclus steht, wobei das Het¹² gegebenenfalls durch einen oder, sofern möglich, zwei oder mehr Substituenten, die aus Hydroxy, Amino oder C₁₋₄-Alkyl- ausgewählt sind, substituiert ist;
Het¹³ für einen aus Furanyl, Piperazinyl, 1,1-Dioxothiomorpholinyl, Piperazininonyl, Piperidinyl, Tetrahydro-1,1-dioxido-2H-thio-pyranyl, Piperidinonyl, Morpholinyl, Piperazinyl oder Pyrrolidinyl ausgewählten Heterocyclus steht; Het¹⁴ und Het¹⁵ jeweils unabhängig für einen aus Morpholinyl, Piperazinyl, Piperidinyl oder Pyrrolidinyl ausgewählten Heterocyclus stehen, wobei das Het¹⁴ und Het¹⁵ gegebenenfalls durch einen oder, sofern möglich, zwei oder mehr Substituenten, die aus Hydroxy, Amino oder C₁₋₄-Alkyl ausgewählt sind, substituiert sind;
Het¹⁶ für einen aus Piperidinyl oder Pyrrolidinyl ausgewählten Heterocyclus steht;
Het²⁰ für Pyrrolidinyl, 2-Pyrrolidinonyl, Piperidinyl oder Hydroxypyrrolidinyl steht;
Het²¹ für Pyrrolidinyl oder Hydroxypyrrolidinyl steht;
Het²² für Pyrrolidinyl, Piperazinyl oder Piperidinyl steht;
Het²³ und Het²⁵ jeweils unabhängig für einen aus Morpholinyl, Pyrrolidinyl, Piperazinyl oder Piperidinyl ausgewählten Heterocyclus stehen, wobei das Het²³ gegebenenfalls durch einen oder, sofern möglich, zwei oder mehr Substituenten, die aus C₁₋₄-Alkyl, C₃₋₆-Cycloalkyl, Hydroxy-C₁₋₄alkyl-, C₁₋₄-Alkyloxy-C₁₋₄-alkyl oder Polyhydroxy-C₁₋₄-alkyl-ausgewählt sind, substituiert ist;
Het²⁴ für Morpholinyl, Pyrrolidinyl, Piperazinyl oder Piperidinyl steht;
Ar⁴, Ar⁵ oder Ar⁶ jeweils unabhängig für Phenyl, das gegebenenfalls durch Nitro, Cyano, C₁₋₄-Alkylsulfonyl-, C₁₋₄-Alkylsulfonylamino-, Amino-sulfonylamino-, Hydroxy-C₁₋₄-alkyl, Aminosulfonyl-, Hydroxy-, C₁₋₄-Alkyloxy- oder C₁₋₄-Alkyl substituiert ist, stehen;
ferner **dadurch gekennzeichnet, dass** entweder Y für -C₁₋₂-Alkyl-NR²³-CO-CR¹⁶R¹⁷-NH- steht;
Het¹ für 2-Bora-1,3-dioxolanyl, das gegebenenfalls durch einen oder, sofern möglich, zwei, drei, vier oder mehr Substituenten, die aus Amino, C₁₋₄-Alkyl, Hydroxy-C₁₋₄-alkyl-, Phenyl, Phenyl-C₁₋₄-alkyl-, C₁₋₄-Alkyloxy-C₁₋₄-alkyl-, Mono- oder Di (C₁₋₄-alkyl)amino- oder Aminocarbonyl- ausgewählt sind, substituiert ist;
R¹³ für C₁₋₆-Alkyloxycarbonyl, das gegebenenfalls durch Phenyl substituiert ist, oder für Ar⁶-Sulfonyl oder Het²⁴-C₁₋₄-Alkylcarbonyl steht; oder R⁴ für C₁₋₄-Alkyloxy, das durch mindestens einen Substituenten, der aus C₁₋₄-Alkyloxy-C₁₋₄-alkyloxy-, NR³⁷R³⁸-Carbonyloxy-, Het⁵ -Carbonyloxy-, NR⁷R⁸, NR⁹R¹⁰-Carbonyl-, Het³-Carbonyl-, Het¹³-Oxy- oder Het²- ausgewählt ist, substituiert ist, steht; wobei
R⁸ für Het⁷-Aminocarbonyl-; Het⁹-Oxycarbonyl-; Het¹⁰-Sulfonyl-; C₁₋₄-Alkyloxycarbonyl; Mono- oder Di(C₁₋₄-alkyl)aminocarbonyl-; Mono- oder Di (C₁₋₄-alkyl) aminocarbonyl, das durch C₁₋₄-Alkylsulfonyl- substituiert ist; oder C₁₋₄-Alkylcarbonyl, das gegebenenfalls durch einen oder mehrere Substituenten, die aus C₁₋₄-Alkylsulfonyl, Hydroxy- und C₁₋₄-Alkyloxy- ausgewählt sind, substituiert ist; oder für C₁₋₄-Alkyl, das durch einen oder mehrere Substituenten, die aus C₁₋₄-Alkylsulfonyl-, NR²⁵R²⁶, Aminocarbonyloxy-, C₁₋₄-Alkylcarbonyloxy-, Aminocarbonyl-, C₁₋₄-Alkyloxy-C1₋₄-alkyloxy- und Het¹¹ ausgewählt sind, substituiert ist, steht; und
Het² für einen aus Morpholinyl, Piperazinyl, Piperidinyl, Pyrrolidinyl oder Thiomorpholinyl ausgewählten Heterocyclus steht, wobei das Het² durch einen oder, sofern möglich, zwei oder mehr Substituenten, die aus C₁₋₄-Alkyl-, das durch einen oder mehrere Substituenten, die aus NR²⁷R²⁸, C₁₋₄-Alkylsulfonyl, Aminocarbonyloxy-, Aminocarbonyl- und Mono- oder Di (C₁₋₄-alkyl)aminocarbonyl- ausgewählt sind, substituiert ist, oder C₁₋₄-Alkyloxy-, das gegebenenfalls durch C₁₋₄-Alkyloxy- substituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonyl- ausgewählt sind, substituiert ist, oder C₁₋₄-Alkylcarbonyl, das gegebenenfalls durch einen oder mehrere Substituenten, die aus Hydroxy-, C₁₋₄-Alkyloxy- und C₁₋₄-Alkylsulfonyl- ausgewählt sind, substituiert ist, ausgewählt sind, substituiert ist;
oder Het² für 1,1-Dioxothiomorpholinyl steht, das gegebenenfalls durch C₁₋₄-Alkyl-, das gegebenenfalls durch einen oder mehrere Substituenten, die aus NR²⁷R²⁸, C₁₋₄Alkylsulfonyl, Aminocarbonyloxy-, Aminocarbonyl- und Mono- oder Di(C₁₋₄-alkyl)aminocarbonyl- ausgewählt sind, substituiert ist, oder C₁₋₄-Alkyloxy-, das gegebenenfalls durch C₁₋₄-Alkyloxy-substituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonyl- ausgewählt sind, substituiert ist, oder C₁₋₄-Alkylcarbonyl, das gegebenenfalls durch einen oder mehrere Substituenten, die aus Hydroxy-, ₁₋₄-Alkyloxy- und C₁₋₄-Alkylsulfonyl- ausgewählt sind, substituiert ist, substituiert ist.

2. Verbindung nach Anspruch 1, wobei:
Z für NH steht;
Y für -C₃₋₉-Alkyl-, -C₁₋₅-Alkyl-NR¹³-C₁₋₅alkyl-, -C₁₋₅-Alkyl-NR¹⁴-CO-C₁₋₅-alkyl-, -C₁₋₆-Alkyl-CO-NH-, -C₁₋₆-Alkyl-NH-CO-, -C₁₋₂-Alkyl-NR²³-CO-CR¹⁶R¹⁷-NH-, -C₁₋₂-Alkyl-NR²¹-CH₂-CO-NH-C₁₋₃-alkyl oder C₁₋₃-Alkyl-NH-CO-Het²⁰- steht;
X¹ für O, -O-C₁₋₂-Alkyl-, NR¹¹ oder -NR¹¹-C₁₋₂-Alkyl-steht;
X² für eine direkte Bindung, C₁₋₂-Alkyl, -CO-C₁₋₂-Alkyl- oder NR¹²-C₁₋₂-Alkyl- steht;
R¹ für Wasserstoff, Cyano, Halogen oder Hydroxy steht;
R² für Wasserstoff, Halogen, Cyano, C₂₋₆-Alkinyl, Hydroxy, Hydroxycarbonyl, C₁₋₄-Alkyloxycarbonyl- oder Het¹ steht;
R³ für Wasserstoff, Cyano, Halogen, Hydroxy, Formyl, C₁₋₆-Alkyloxy- oder C₁₋₆-Alkyloxy-, das durch Halogen substituiert ist, steht;
R⁴ für Ar⁴-C₁₋₄-Alkyloxy-, C₁₋₄-Alkyloxy- oder C₁₋₄-Alkyloxy, das durch einen oder, sofern möglich, zwei oder mehr Substituenten, die aus Hydroxy, C₁₋₄-Alkyloxy-, C₁₋₄-Alkyloxy-C₁₋₄-alkyloxy-, NR⁷R⁸ oder Het² ausgewählt sind, substituiert ist, steht;
R⁷ für Wasserstoff, Hydroxy-C₁₋₄-alkyl- oder C₁₋₄-Alkyl steht;
R⁸ für C₁₋₄-Alkyloxycarbonyl oder C₁₋₄-Alkyl, das durch einen oder mehrere Substituenten, die aus C₁₋₄-Alkylsulfonyl-, C₁₋₄-Alkylcarbonyloxy oder NR²⁵R²⁶ ausgewählt sind, substituiert ist, steht;
insbesondere R⁸ für C₁₋₄-Alkyl, das durch einen oder mehr Substituenten, die aus C₁₋₄-Alkylsulfonyl- oder NR²⁵R²⁶ ausgewählt sind, substituiert ist, steht;
R¹¹ für Wasserstoff, C₁₋₄-Alkyloxycarbonyl oder C₁₋₄-Alkyl steht;
R¹² für Wasserstoff oder C₁₋₄-Alkyl steht;
R¹³ für C₁₋₆-Alkyloxycarbonyl, das gegebenenfalls durch Phenyl substituiert ist, oder für Ar⁶-Sulfonyl oder Het²⁴-C₁₋₄-Alkylcarbonyl steht;
R¹⁴ und R¹⁵ jeweils unabhängig für Wasserstoff oder C₁₋₄-Alkyl stehen;
R¹⁶ und R¹⁷ jeweils unabhängig für Wasserstoff oder C₁₋₄-Alkyl, das gegebenenfalls durch C₃₋₆-Cycloalkyl substituiert ist, stehen oder R¹⁶ und R¹⁷ zusammen mit dem Kohlenstoffatom, an das sie gebunden sind, ein C₃₋₆-Cycloalkyl bilden;
R²¹ für Wasserstoff steht;
R²³ für C₁₋₄-Alkyl, das gegebenenfalls durch Hydroxy-, C₁₋₄-Alkyloxy- oder Het²⁵ substituiert ist, steht; R²³ auch für Wasserstoff stehen kann, wenn R¹⁶ und R¹⁷ zusammen mit dem Kohlenstoffatom, an das sie gebunden sind, ein C₃₋₆-Cycloalkyl bilden;
R²⁵ und R²⁶ jeweils unabhängig für Wasserstoff, C₁₋₄-Alkyl, C₁₋₄-Alkylsulfonyl, C₁₋₄-Alkyloxycarbonyl oder C₁₋₄-Alkylcarbonyl stehen;
R²⁷ und R²⁸ unabhängig voneinander für Wasserstoff, C₁₋₄-Alkyl, C₁₋₄-Alkylsulfonyl, C₁₋₄-Alkyloxycarbonyl- oder C₁₋₄-Alkylcarbonyl stehen;
Het¹ für 2-Bora-1,3-dioxolanyl steht, das gegebenenfalls durch einen oder, sofern möglich, zwei, drei, vier oder mehr Substituenten, die aus Amino, C₁₋₄-Alkyl, Hydroxy-C₁₋₄-alkyl-, Phenyl, Phenyl-C₁₋₄-alkyl-, C₁₋₄-Alkyloxy-C₁₋₄-alkyl-, Mono- oder Di(C₁₋₄-alkyl)amino- oder Aminocarbonyl- ausgewählt sind, substituiert ist;
Het² für 1,1-Dioxothiomorpholinyl, das gegebenenfalls durch C₁₋₄-Alkyloxycarbonyl oder C₁₋₄-Alkyl-NR²⁷R²⁸ substituiert ist; oder für Piperidinyl oder Piperazinyl, das durch C₁₋₄-Alkyloxycarbonyl oder C₁₋₄-Alkyl-NR²⁷R²⁸ substituiert ist, steht;
Het²⁰ für Pyrrolidinyl, 2-Pyrrolidinonyl, Piperidinyl oder Hydroxypyrrolidinyl steht;
Het²⁵ für einen aus Morpholinyl oder Piperazinyl ausgewählten Heterocyclus steht, wobei der Heterocyclus gegebenenfalls durch C₁₋₄-Alkyl, Hydroxy-C₁₋₄alkyl, C₁₋₄-Alkyloxy-C₁₋₄-alkyl oder Polyhydroxy-C₁₋₄-alkyl substituiert ist; oder
Ar⁴, Ar⁵ oder Ar⁶ jeweils unabhängig für Phenyl, das gegebenenfalls durch Nitro, Cyano, Hydroxy, Hydroxy-C₁₋₄-alkyl, C₁₋₄-Alkyl oder C₁₋₄-Alkyloxy substituiert ist, stehen;
ferner **dadurch gekennzeichnet, dass** entweder Y für -C₁₋₂-Alkyl-NR²³-CO-CR¹⁶R¹⁷-NH- steht oder R⁴ für C₁₋₄-Alkyloxy, das durch mindestens einen Substituenten, der aus C₁₋₄-Alkyloxy-C₁₋₄-alkyloxy-, NR⁷R⁸ oder Het² ausgewählt ist, substituiert ist, steht.

3. Verbindung nach Anspruch 1 oder 2, wobei:
Z für NH steht;
Y für -C₃₋₉-Alkyl-, -C₁₋₅-Alkyl-NR¹³-C₁₋₅alkyl-, -C₁₋₅-Alkyl-NR¹⁴-CO-C₁₋₅-alkyl-, -C₁₋₆-Alkyl-CO-NH-, -C₁₋₆-Alkyl-NH-CO-, -C₁₋₂-Alkyl-NR²³-CO-CR¹⁶R¹⁷-NH-, -C₁₋₂-Alkyl-NR²¹-CH₂-CO-NH-C₁₋₃-alkyl oder C₁₋₃-Alkyl-NH-CO-Het²⁰- steht;
X¹ für O, -O-C₁₋₂-Alkyl-, NR¹¹ oder -NR¹¹-C₁₋₂-Alkylsteht;
X² für eine direkte Bindung, C₁₋₂-Alkyl, -CO-C₁₋₂-Alkyl- oder NR¹²-C₁₋₂-Alkyl- steht;
R¹ für Wasserstoff oder Halogen steht;
R² für Wasserstoff, Halogen, C₂₋₆-Alkinyl, Cyano oder Het¹ steht;
R³ für Wasserstoff steht;
R⁴ für Ar⁴-C₁₋₄-Alkyloxy-, C₁₋₄-Alkyloxy- oder C₁₋₄-Alkyloxy, das durch einen oder, sofern möglich, zwei oder mehr Substituenten, die aus Hydroxy, C₁₋₄-Alkyloxy-, C₁₋₄-Alkyloxy-C₁₋₄-alkyloxy-, NR⁷R⁸ oder Het² ausgewählt sind, substituiert ist, steht;
R⁷ für Wasserstoff oder C₁₋₄-Alkyl steht;
R⁸ für C₁₋₄-Alkyloxycarbonyl oder C₁₋₄-Alkyl, das durch einen oder mehrere Substituenten, die aus C₁₋₄-Alkylsulfonyl-, C₁₋₄-Alkylcarbonyloxy oder NR²⁵R²⁶ ausgewählt sind, substituiert ist, steht;
R¹¹ für Wasserstoff oder C₁₋₄-Alkyl steht;
R¹² für Wasserstoff oder C₁₋₄-Alkyl steht;
R¹³ für Ar⁶-Sulfonyl oder C₁₋₆-Alkyloxycarbonyl, das gegebenenfalls durch Phenyl substituiert ist, steht;
R¹⁴ und R¹⁵ für Wasserstoff stehen;
R¹⁶ und R¹⁷ jeweils unabhängig für Wasserstoff oder C₁₋₄-Alkyl, das gegebenenfalls durch C₃₋₆-Cycloalkyl substituiert ist, stehen oder R¹⁶ und R¹⁷ zusammen mit dem Kohlenstoffatom, an das sie gebunden sind, ein C₃₋₆-Cycloalkyl bilden;
R²¹ für Wasserstoff steht;
R²³ für C₁₋₄-Alkyl, das gegebenenfalls durch Hydroxy-, C₁₋₄-Alkyloxy- oder Het²⁵ substituiert ist, steht; R²³ auch für Wasserstoff stehen kann, wenn R¹⁶ und R¹⁷ zusammen mit dem Kohlenstoffatom, an das sie gebunden sind, ein C₃₋₆-Cycloalkyl bilden;
R²⁵ und R²⁶ jeweils unabhängig für Wasserstoff oder C₁₋₄-Alkylcarbonyl- stehen;
R²⁷ und R²⁸ jeweils unabhängig voneinander für Wasserstoff oder C₁₋₄-Alkylcarbonyl stehen;
Het¹ für 2-Bora-1,3-dioxolanyl steht;
Het² für 1,1-Dioxothiomorpholinyl, Piperidinyl oder Piperazinyl steht, wobei das Het² gegebenenfalls durch C₁₋₄-Alkyloxycarbonyl oder -C₁₋₄-Alkyl-NR²⁷R²⁸ substituiert ist;
Het²⁰ für Pyrrolidinyl steht;
Het²⁵ für einen aus Morpholinyl oder Piperazinyl ausgewählten Heterocyclus steht, wobei der Heterocyclus gegebenenfalls durch C₁₋₄-Alkyl, Hydroxy-C₁₋₄alkyl, C₁₋₄-Alkyloxy-C₁₋₄-alkyl oder Polyhydroxy-C₁₋₄-alkyl substituiert ist; oder
Ar⁴ für Phenyl steht;
Ar⁵ für Phenyl steht; oder
Ar⁶ für Phenyl, das gegebenenfalls durch Nitro substituiert ist, steht;
ferner **dadurch gekennzeichnet, dass** entweder Y für -C₁₋₂-Alkyl-NR²³-CO-CR¹⁶R¹⁷-NH- steht oder R⁴ für C₁₋₄-Alkyloxy, das durch mindestens einen Substituenten, der aus C₁₋₄-Alkyloxy-C₁₋₄-alkyloxy-, NR⁷R⁸ oder Het²- ausgewählt ist, substituiert ist;
insbesondere C₁₋₄-Alkyloxy, das durch C₁₋₄-Alkyloxy-C₁₋₄-alkyloxy- oder NR⁷R⁸ substituiert ist, steht.

4. Verbindung nach einem der Ansprüche 1 bis 3, wobei:
Z für NH steht;
Y für -C₃₋₉-Alkyl-, -C₁₋₅-Alkyl-NR¹³-C₁₋₅alkyl-, -C₁₋₅-Alkyl-NR¹⁴-CO-C₁₋₅-alkyl-, -C₁₋₂-Alkyl-NR²¹-CH₂-CO-NH-C₁₋₃-alkyl oder -C₁₋₂-Alkyl-NR²³-CO-CR¹⁶R¹⁷-NH- steht;
X¹ für O oder -O-C₁₋₂-Alkyl- steht;
X² für eine direkte Bindung, C₁₋₂-Alkyl, -CO-C₁₋₂-Alkyl- oder NR¹²-C₁₋₂-Alkyl- steht;
R¹ für Wasserstoff oder Halogen steht; insbesondere R¹ für Wasserstoff steht;
R² für Halogen, Acetylen oder Het¹ steht;
insbesondere R² für Halogen oder Het¹ steht;
R³ für Wasserstoff steht;
R⁴ für Ar⁴-C₁₋₄-Alkyloxy-, C₁₋₄-Alkyloxy- oder C₁₋₄-Alkyloxy, das durch einen oder, sofern möglich, zwei oder mehr Substituenten, die aus Het², NR⁷R⁸, Hydroxy und C₁₋₄-Alkyloxy-C₁₋₄-alkyloxy- ausgewählt sind, substituiert ist, steht;
R⁷ für Wasserstoff oder C₁₋₄-Alkyl steht;
R⁸ für C₁₋₄-Alkyl, das durch NR²⁵R²⁶ oder C₁₋₄-Alkylsulfonyl- substituiert ist, steht;
R¹² für Wasserstoff oder C₁₋₄-Alkyl- steht;
R¹³ für Ar⁶-Sulfonyl oder C₁₋₆-Alkyloxycarbonyl, das gegebenenfalls durch Phenyl substituiert ist, steht;
R¹⁶ und R¹⁷ für Wasserstoff oder C₁₋₄-Alkyl stehen oder R¹⁶ und R¹⁷ zusammen mit dem Kohlenstoffatom, an das sie gebunden sind, ein C₃₋₆-Cycloalkyl bilden;
R²³ für Wasserstoff oder C₁₋₄-Alkyl steht;
R²⁵ und R²⁶ jeweils unabhängig für Wasserstoff oder C₁₋₄-Alkylcarbonyl stehen;
R²⁷ und R²⁸ unabhängig voneinander für Wasserstoff oder C₁₋₄-Alkylcarbonyl stehen;
Het¹ für 2-Bora-1,3-dioxolanyl steht;
Het² für Piperidinyl, Piperazinyl, Morpholinyl, Thiomorpholinyl oder 1,1-Dioxothiomorpholinyl steht, wobei das Het² gegebenenfalls durch C₁₋₄-Alkyloxycarbonyl oder NR²⁷R²⁸-C₁₋₄-Alkyl substituiert ist;
Ar⁴ für Phenyl steht;
Ar⁵ für Phenyl steht; oder
Ar⁶ für Phenyl, das gegebenenfalls durch Nitro substituiert ist, steht.

5. Verbindung nach einem der Ansprüche 1 bis 3, wobei:
Z für NH steht;
Y für -C₃₋₉-Alkyl-, -C₁₋₅-Alkyl-NR¹³-C₁₋₅alkyl- oder -C₁₋₂-Alkyl-NR²³-CO-CR¹⁶R¹⁷-NH- steht;
X¹ für O steht; X² für eine direkte Bindung oder NR¹²-C₁₋₂-Alkyl- steht;
R¹ für Wasserstoff steht; R² für Halogen oder Het¹ steht; R³ für Wasserstoff steht;
R⁴ für Ar4-C₁₋₄-Alkyloxy-, C₁₋₄-Alkyloxy- oder C₁₋₄-Alkyloxy, das durch C₁₋₄-Alkyloxy-C₁₋₄-alkyloxy-substituiert ist, steht;
R¹² für Wasserstoff oder C₁₋₄-Alkyl- steht;
R¹³ für Ar⁶-Sulfonyl oder C₁₋₆-Alkyloxycarbonyl, das gegebenenfalls durch Phenyl substituiert ist, steht;
R¹⁶ und R¹⁷ zusammen mit dem Kohlenstoffatom, an das sie gebunden sind, ein C₃₋₆-Cycloalkyl bilden;
R²³ für Wasserstoff oder C₁₋₄-Alkyl steht;
Het¹ für 2-Bora-1,3-dioxolanyl steht;
Ar⁴ für Phenyl steht; Ar⁵ für Phenyl steht;
Ar⁶ für Phenyl, das gegebenenfalls durch Nitro substituiert ist, steht.

6. Verbindung nach Anspruch 1, wobei
Z für NH steht;
Y für -C₃₋₉-Alkyl-, -C₂₋₉-Alkenyl-, -C₁₋₅Alkyl-oxy-C₁₋₅-alkyl-, -C₁₋₅-Alkyl-NR¹³-C₁₋₅-alkyl-, -C₁₋₅-Alkyl-NR¹⁴-CO-C₁₋₅-alkyl-, -C₁₋₆-Alkyl-NH-CO-, -NH-CO-C₁₋₆-Alkyl-, -CO-C₁₋₇-Alkyl-, -C₁₋₇-Alkyl-CO-, C₁₋₆-Alkyl-CO-C₁₋₆-alkyl, -C₁₋₂-Alkyl-NR²³-CO-CR¹⁶R¹⁷-NH-, -C₁₋₂-Alkyl-CO-NH-CR¹⁸R¹⁹-CO-, -C₁₋₂-Alkyl-CO-NR²⁰-C₁₋₃-alkyl-CO-, -C₁₋₂-Alkyl-NR²¹-CH₂-CO-NH-C₁₋₃-alkyl-, -NR²²-CO-C₁₋₃-Alkyl-NH-, -C₁₋₃-Alkyl-NH-CO-Het²⁰-, C₁₋₂-Alkyl-CO-Het²¹-CO- oder -Het²²-CH₂-CO-NH-C₁₋₃-Alkyl- steht;
X¹ für O, -O-C₁₋₂-Alkyl-, -O-N=CH-, NR¹¹ oder -NR¹¹-C₁₋₂-Alkyl- steht;
X² für eine direkte Bindung, C₁₋₂-Alkyl, O, -O-C₁₋₂-Alkyl-, CO, -CO-C₁₋₂-Alkyl-, -O-N=CH-, NR¹² oder NR¹²-C₁₋₂-Alkyl- steht;
R¹ für Wasserstoff, Cyano, Halogen oder Hydroxy steht;
R² für Wasserstoff, Cyano, Halogen, Hydroxy, Hydroxycarbonyl-, C₁₋₄-Alkyloxycarbonyl-, Het¹⁶-Carbonyl-, C₁₋₄-Alkyl-, C₂₋₆-Alkinyl-, Ar⁵, Het¹ oder Dihydroxyboran steht;
R³ für Wasserstoff, Cyano, Halogen, Hydroxy, Formyl, C₁₋₆-Alkoxy-, C₁₋₆-Alkyl-, C₁₋₆-Alkoxy-, das durch Halogen substituiert ist, oder für C₁₋₄-Alkyl, das durch einen oder, sofern möglich, zwei oder mehr Substituenten, die aus Hydroxy oder Halogen ausgewählt sind, substituiert ist, steht; R⁴ für Ar⁴-C₁₋₄-Alkyloxy-, C₁₋₄-Alkyloxy- oder für C₁₋₄-Alkyloxy, das durch einen oder, sofern möglich, zwei oder mehr Substituenten, die aus Hydroxy-, Halogen, C₁₋₄-Alkyloxy-, C₁₋₄-Alkyloxy-C₁₋₄-alkyloxy-, NR³⁷R³⁸-Carbonyloxy-, Het⁵-Carbonyloxy-, NR⁷R⁸, NR⁹R¹⁰-Carbonyl-, Het³-Carbonyl-, Het¹³-Oxy- oder Het²- ausgewählt sind, substituiert ist, steht;
R⁷ für Wasserstoff oder C₁₋₄-Alkyl steht;
R⁸ für C₃₋₆-Cycloalkyl, Het⁶-Carbonyl-, Het⁷-Aminocarbonyl-, Het⁸, Het⁹-Oxycarbonyl-, Het¹⁰-Sulfonyl-, Mono- oder Di(C₁₋₄-alkyl)aminocarbonyl-, Mono- oder Di(C₁₋₄-alkyl)aminocarbonyl, das durch C₁₋₄-Alkylsulfonyl- substituiert ist, oder C₁₋₄-Alkylcarbonyl, das gegebenenfalls durch einen oder mehrere Substituenten, die aus C₁₋₄-Alkylsulfonyl, Hydroxy- und C₁₋₄-Alkyloxy- ausgewählt sind, substituiert ist; oder für C₁₋₄-Alkyl, das durch einen oder mehrere Substituenten, die aus C₁₋₄-Alkylsulfonyl-, NR²⁵R²⁶, Aminocarbonyloxy-, Amino-carbonyl-, C₁₋₄-Alkyloxy-C₁₋₄-alkyloxy- und Het¹¹ ausgewählt sind, substituiert ist, steht;
R⁹ für Wasserstoff oder C₁₋₄-Alkyl- steht;
R¹⁰ für Het⁴ oder C₁₋₄-Alkyl-, das durch C₁₋₄-Alkylsulfonyl- substituiert ist, steht;
R¹¹ für Wasserstoff, C₁₋₄-Alkyl- oder C₁₋₄-Alkyloxycarbonyl- steht;
R¹² für Wasserstoff, C₁₋₄-Alkyl-, C₁₋₆-Alkyloxycarbonyl oder C₁₋₆-Alkyloxycarbonyl, das durch Phenyl substituiert ist, steht;
R¹³ für Wasserstoff, Het¹⁴-C₁₋₄-Alkyl, C₁₋₆-Alkyloxycarbonyl, das gegebenenfalls durch Phenyl substituiert ist, oder für Ar⁶-Sulfonyl oder Het²⁴-C₁₋₄-Alkylcarbonyl steht;
R¹⁴ und R¹⁵ jeweils unabhängig aus Wasserstoff, C₁₋₄-Alkyl, Het¹⁵-C₁₋₄-Alkyl- oder C₁₋₆-Alkyloxy-C₁₋₄-alkyl ausgewählt sind;
R¹⁶ und R¹⁷ jeweils unabhängig für Wasserstoff, C₁₋₄-Alkyl oder C₁₋₄-Alkyl, das durch Hydroxy- oder Phenyl substituiert ist, stehen oder R¹⁶ und R¹⁷ zusammen mit dem Kohlenstoffatom, an das sie gebunden sind, ein C₃₋₆-Cycloalkyl bilden;
R¹⁸ für Wasserstoff oder C₁₋₄-Alkyl, das gegebenenfalls durch Hydroxy oder Phenyl substituiert ist, steht;
R¹⁹ für Wasserstoff oder C₁₋₄-Alkyl steht;
R²⁰ für Wasserstoff oder C₁₋₄-Alkyl steht;
R²¹ für Wasserstoff, C₁₋₄-Alkyl, Het²³-C₁₋₄-Alkylcarbonyl oder für Mono- oder Di(C₁₋₄alkyl)amino-C₁₋₄-alkylcarbonyl-, das gegebenenfalls durch Hydroxy, Pyrimidinyl, Dimethylamin oder C₁₋₄-Alkyloxy substituiert ist, steht;
R²² für Wasserstoff oder C₁₋₄-Alkyl, das gegebenenfalls durch Hydroxy oder C₁₋₄-Alkyloxy substituiert ist, steht;
R²³ für C₁₋₄-Alkyl, das gegebenenfalls durch Hydroxy-, C₁₋₄-Alkyloxy- oder Het²³ substituiert ist, steht; R²³ auch für Wasserstoff stehen kann, wenn R¹⁶ und R¹⁷ zusammen mit dem Kohlenstoffatom, an das sie gebunden sind, ein C₃₋₆-Cycloalkyl bilden;
R²⁵ und R²⁶ jeweils unabhängig für Wasserstoff, C₁₋₄-Alkyl, C₁₋₄-Alkylsulfonyl-, Aminocarbonyl-, Mono- oder Di (C₁₋₄-alkyl) aminocarbonyl-, C₁₋₄-Alkylcarbonyl-, C₁₋₄-Alkyloxycarbonyl- oder C₁₋₄-Alkyl, das durch einen oder mehrere Substituenten, die aus C₁₋₄-Alkylsulfonyl-, Hydroxy- und C₁₋₄-Alkyloxy-ausgewählt sind, substituiert ist, stehen;
R²⁷ und R²⁸ unabhängig voneinander für Wasserstoff, C₁₋₄-Alkyl, C₁₋₄-Alkylsulfonyl-, Aminocarbonyl-, Mono- oder Di (C₁₋₄-alkyl) aminocarbonyl-, C₁₋₄-Alkylcarbonyl-, C₁₋₄-Alkyloxycarbonyl- oder C₁₋₄-Alkyl, das durch einen oder mehrere Substituenten, die aus C₁₋₄-Alkylsulfonyl-, Hydroxy- und C₁₋₄-Alkyloxy- ausgewählt sind, substituiert ist, stehen;
R²⁹ und R³⁰ jeweils unabhängig für Wasserstoff, Aminosulfonyl, Aminocarbonyl, Mono- oder Di(C₁₋₄-alkyl) aminocarbonyl-, Mono- oder Di (C₁₋₄-alkyl) aminosulfonyl- oder C₁₋₄-Alkyl-, das gegebenenfalls durch einen oder mehrere Substituenten, die aus NR³¹R³², C₁₋₄-Alkylsulfonyl, Aminocarbonyloxy-, Hydroxy-, C₁₋₄-Alkyloxy-, Aminocarbonyl- und Mono- oder Di(C₁₋₄-alkyl)-aminocarbonyl- ausgewählt sind, substituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonyl-ausgewählt sind, substituiert ist, oder C₁₋₄-Alkylcarbonyl, das gegebenenfalls durch einen oder mehrere Substituenten, die aus Hydroxy-, C₁₋₄-Alkyloxy- und C₁₋₄-Alkylsulfonyl- ausgewählt sind, substituiert ist, stehen;
R³¹ und R³² jeweils unabhängig für Wasserstoff, C₁₋₄-Alkyl, C₁₋₄-Alkylsulfonyl-, Aminocarbonyl-, Mono- oder Di(C₁₋₄-alkyl)aminocarbonyl-, C₁₋₄-Alkyl-carbonyl-, C₁₋₄-Alkyloxycarbonyl- oder C₁₋₄-Alkyl, das durch einen oder mehrere Substituenten, die aus C₁₋₄-Alkylsulfonyl-, Hydroxy- und C₁₋₄-Alkyloxy-ausgewählt sind, substituiert ist, stehen;
R³³ für Wasserstoff oder C₁₋₄-Alkyl steht;
R³⁴ für C₁₋₄-Alkylsulfonyl-, Aminocarbonyl-, Mono- oder Di(C₁₋₄-alkyl) aminocarbonyl-, C₁₋₄-Alkylcarbonyl-, C₁₋₄-Alkyloxycarbonyl- oder C₁₋₄-Alkyl, das durch einen oder mehrere Substituenten, die aus C₁₋₄-Alkylsulfonyl-, Hydroxy- und C₁₋₄-Alkyloxyausgewählt sind, substituiert ist, steht;
R³⁵ für Wasserstoff oder C₁₋₄-Alkyl steht;
R³⁶ für C₁₋₄-Alkylsulfonyl-, Aminocarbonyl-, Mono- oder Di(C₁₋₄-alkyl)aminocarbonyl-, C₁₋₄-Alkylcarbonyl-, C₁₋₄-Alkyloxycarbonyl- oder C₁₋₄-Alkyl, das durch einen oder mehrere Substituenten, die aus C₁₋₄-Alkylsulfonyl-, Hydroxy- und C₁₋₄-Alkyloxyausgewählt sind, substituiert ist, steht;
R³⁷ und R³⁸ jeweils unabhängig für Wasserstoff, C₁₋₄-Alkyl, C₁₋₄-Alkylsulfonyl-, Het¹² oder C₁₋₄-Alkyl, das durch einen oder mehrere Substituenten, die aus C₁₋₄-Alkylsulfonyl-, Hydroxy- und C₁₋₄-Alkyloxyausgewählt sind, substituiert ist, stehen;
R³⁹ und R⁴⁰ jeweils unabhängig für Aminosulfonyl, Aminocarbonyl, Mono- oder Di(C₁₋₄-alkyl)-aminocarbonyl-, Mono- oder Di(C₁₋₄-alkyl)-aminosulfonyl- oder C₁₋₄-Alkyl-, das durch einen oder mehrere Substituenten, die aus NR³¹R³², C₁₋₄-Alkylsulfonyl, Aminocarbonyloxy-, Hydroxy-, C₁₋₄-Alkyloxy-, Aminocarbonyl- und Mono- oder Di(C₁₋₄-alkyl)aminocarbonyl- ausgewählt sind, substituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonyl-ausgewählt sind, substituiert ist, oder C₁₋₄-Alkylcarbonyl, das gegebenenfalls durch einen oder mehrere Substituenten, die aus Hydroxy-, C₁₋₄-Alkyloxy- und C₁₋₄-Alkylsulfonyl- ausgewählt sind, substituiert ist, stehen;
Het¹ für Thiazolyl oder 2-Bora-1,3-dioxolanyl steht, wobei das Het¹ gegebenenfalls durch einen oder, sofern möglich, zwei, drei, vier oder mehr Substituenten, die aus Amino, C₁₋₄-Alkyl, Hydroxy-C₁₋₄-alkyl-, Phenyl, Phenyl-C₁₋₄-alkyl-, C₁₋₄-Alkyloxy-C₁₋₄-alkyl-, Mono- oder Di(C₁₋₄-alkyl)amino- oder Aminocarbonyl- ausgewählt sind, substituiert ist;
Het² für einen aus Tetrahydropyranyl, Tetrahydrofuranyl, Furanyl, 1,1-Dioxothiomorpholinyl, Piperazininonyl, Tetrahydro-1,1-dioxido-2H-thiopyranyl, Piperidinonyl, Azetidinyl oder 2-Azetidinonyl ausgewählten Heterocyclus steht, wobei das Het² gegebenenfalls durch einen oder, sofern möglich, zwei oder mehr Substituenten, die aus Hydroxy, Amino, NR²⁹R³⁰, Aminocarbonyl, Mono- oder Di(C₁₋₄-alkyl)aminocarbonyl, C₁₋₄-Alkylsulfonyl oder C₁₋₄-Alkyl-, das gegebenenfalls durch einen oder mehrere Substituenten, die aus NR²⁷R²⁸, C₁₋₄-Alkylsulfonyl, Aminocarbonyloxy-, Aminocarbonyl- und Mono- oder Di(C₁₋₄-alkyl)aminocarbonyl- ausgewählt sind, substituiert ist, oder C₁₋₄-Alkyloxy-, das gegebenenfalls durch C₁₋₄-Alkyloxysubstituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonyl- ausgewählt sind, substituiert ist, oder C₁₋₄-Alkylcarbonyl, das gegebenenfalls durch einen oder mehrere Substituenten, die aus Hydroxy-, C₁₋₄-Alkyloxy- und C₁₋₄-Alkylsulfonyl-ausgewählt sind, substituiert ist, ausgewählt sind, substituiert ist; oder
Het² für einen aus Morpholinyl, Piperazinyl, Piperidinyl oder Pyrrolidinyl ausgewählten Heterocyclus steht, wobei das Morpholinyl, Piperazinyl, Piperidinyl oder Pyrrolidinyl gegebenenfalls durch einen oder, sofern möglich, zwei oder mehr Substituenten, die aus C₁₋₄-Alkyl-, das gegebenenfalls durch einen oder mehrere Substituenten, die aus NR²⁷R²⁸, C₁₋₄-Alkylsulfonyl, Aminocarbonyloxy-, Aminocarbonyl- und Mono- oder Di(C₁₋₄-alkyl)aminocarbonyl- ausgewählt sind, substituiert ist, oder C₁₋₄-Alkyloxy-, das gegebenenfalls durch C₁₋₄-Alkyloxy- substituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonyl-ausgewählt sind, substituiert ist, oder C₁₋₄-Alkylcarbonyl, das gegebenenfalls durch einen oder mehrere Substituenten, die aus Hydroxy-, C₁₋₄-Alkyloxy- und C₁₋₄-Alkylsulfonyl- ausgewählt sind, substituiert ist, ausgewählt sind, substituiert sind;
Het³ für einen aus Tetrahydropyranyl, Tetrahydrofuranyl, Furanyl, 1,1-Dioxothiomorpholinyl, Piperazininonyl, Tetrahydro-1,1-dioxido-2H-thiopyranyl, Piperidinonyl, Azetidinyl oder 2-Azetidinonyl ausgewählten Heterocyclus steht, wobei das Het³ gegebenenfalls durch einen oder, sofern möglich, zwei oder mehr Substituenten, die aus Hydroxy-, Amino, C₁₋₄-Alkyl-, C₃₋₆-Cycloalkyl-C₁₋₄-alkyl-, Aminosulfonyl-, Mono- oder Di(C₁₋₄-alkyl)aminosulfonyl-, Amino-C₁₋₄-alkyl-, Mono- oder Di(C₁₋₄-alkyl)amino-C₁₋₄-alkyl, NR³⁵R³⁶, C₁₋₄-Alkylsulfonyl-C₁₋₄-alkyl- oder C₁₋₄-Alkyloxy-, das gegebenenfalls durch C₁₋₄-Alkyloxy- oder Hydroxy substituiert ist, ausgewählt sind, substituiert ist; oder
Het³ für einen aus Morpholinyl, Piperazinyl, Piperidinyl oder Pyrrolidinyl ausgewählten Heterocyclus steht, wobei das Het³ gegebenenfalls durch einen oder, sofern möglich, zwei oder mehr Substituenten, die aus NR³⁵R³⁶, C₁₋₄-Alkylsulfonyl-C₁₋₄-alkyl- oder C₁₋₄-Alkyloxy-, das gegebenenfalls durch C₁₋₄-Alkyloxy- oder Hydroxy substituiert ist, ausgewählt sind, substituiert ist;
Het⁴ für einen aus Morpholinyl, Piperazinyl, Piperidinyl, Furanyl, Pyrazolyl, Dioxolanyl, Thiazolyl, Oxazolyl, Imidazolyl, Isoxazolyl, Oxadiazolyl, Pyridinyl oder Pyrrolidinyl ausgewählten Heterocyclus steht, wobei das Het⁴ durch einen oder, sofern möglich, zwei oder mehr Substituenten, die aus C₁₋₄-Alkylsulfonyl-C₁₋₄-alkyl- oder C₁₋₄-Alkyloxy-, das gegebenenfalls durch C₁₋₄-Alkyloxy- oder Hydroxy substituiert ist, ausgewählt sind, substituiert ist;
Het⁵ für einen aus Furanyl, Piperazinyl, 1,1-Dioxothiomorpholinyl, Piperazininonyl, Piperidinyl, Tetrahydro-1,1-dioxido-2H-thiopyranyl, Piperidinonyl, Morpholinyl oder Pyrrolidinyl ausgewählten Heterocyclus steht, wobei das Het⁵ gegebenenfalls durch Hydroxy, Amino, Mono- oder Di(C₁₋₄alkyl)-amino-, C₁₋₄-Alkyl substituiert ist;
Het⁶ und Het⁷ jeweils unabhängig für einen aus Piperazinyl, Piperidinyl oder Pyrrolidinyl ausgewählten Heterocyclus stehen, wobei die Heterocyclen gegebenenfalls durch einen oder mehrere Substituenten, die aus Hydroxy, Amino, Hydroxy-C₁₋₄-alkyl-, C₁₋₄-Alkyloxy-C₁₋₄-alkyl- und C₁₋₄-Alkyl- ausgewählt sind, substituiert sind;
Het⁸ für einen aus Tetrahydropyranyl, Tetrahydrofuranyl, 1,1-Dioxothiomorpholinyl, Piperazininonyl, Tetrahydro-1,1-dioxido-2H-thiopyranyl, Piperidinonyl, Azetidinyl oder 2-Azetidinonyl ausgewählten Heterocyclus steht, wobei das Het⁸ gegebenenfalls durch Aminosulfonyl, Aminocarbonyl, Mono- oder Di(C₁₋₄-alkyl)amino-carbonyl-, Mono- oder Di(C₁₋₄-alkyl)aminosulfonyl- oder C₁₋₄-Alkyl-, das gegebenenfalls durch einen oder mehrere Substituenten, die aus Amino, Mono- oder Di (C₁₋₄-alkyl) amino-, NR³³R³⁴, C₁₋₄-Alkylsulfonyl, Aminocarbonyloxy-, Hydroxy-, C₁₋₄-Alkyloxy-, Aminocarbonyl- und Mono- oder Di(C₁₋₄-alkyl)aminocarbonyl- ausgewählt sind, substituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonyl-ausgewählt sind, substituiert ist, oder C₁₋₄-Alkylcarbonyl, das gegebenenfalls durch einen oder mehrere Substituenten, die aus Hydroxy, C₁₋₄-Alkyloxy- und C₁₋₄-Alkylsulfonyl- ausgewählt sind, substituiert ist, substituiert ist; oder
Het⁸ für einen aus Furanyl, Piperidinyl oder Piperazinyl ausgewählten Heterocyclus steht, wobei das Het⁸ durch Aminocarbonyl, Mono- oder Di(C₁₋₄-alkyl)aminocarbonyl-, Mono- oder Di(C₁₋₄-alkyl)-aminosulfonyl- oder C₁₋₄-Alkyl-, das durch einen oder mehrere Substituenten, die aus NR³³R³⁴, C₁₋₄-Alkylsulfonyl, Aminocarbonyloxy-, Hydroxy-, C₁₋₄-Alkyloxy-, Aminocarbonyl- und Mono- oder Di(C₁₋₄-alkyl)aminocarbonyl- ausgewählt sind, substituiert ist, oder C₁₋₄-Alkyloxycarbonyl, das gegebenenfalls durch einen oder mehrere Substituenten, die aus Hydroxy, C₁₋₄-Alkyloxy- und C₁₋₄-Alkylsulfonyl-ausgewählt sind, substituiert ist, oder C₁₋₄-Alkylcarbonyl, das gegebenenfalls durch einen oder mehrere Substituenten, die aus Hydroxy, C₁₋₄-Alkyloxy- und C₁₋₄-Alkylsulfonyl- ausgewählt sind, substituiert ist, substituiert ist;
Het⁹ und Het¹⁰ jeweils unabhängig für einen aus Piperazinyl, Piperidinyl oder Pyrrolidinyl ausgewählten Heterocyclus stehen, wobei die Heterocyclen gegebenenfalls durch einen oder mehrere Substituenten, die aus Hydroxy-, Amino, Hydroxy-C₁₋₄-alkyl-, C₁₋₄-Alkyloxy-C₁₋₄-alkyl- und C₁₋₄-Alkyl- ausgewählt sind, substituiert sind;
Het¹¹ für 2-Imidazolidinonyl oder 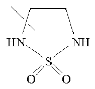 steht;
Het¹² für einen aus Morpholinyl, Piperazinyl, Piperidinyl oder Pyrrolidinyl ausgewählten Heterocyclus steht, wobei das Het¹² gegebenenfalls durch einen oder, sofern möglich, zwei oder mehr Substituenten, die aus Hydroxy, Amino oder C₁₋₄-Alkyl- ausgewählt sind, substituiert ist;
Het¹³ für einen aus Furanyl, Piperazinyl, 1,1-Dioxothiomorpholinyl, Piperazininonyl, Piperidinyl, Tetrahydro-1,1-dioxido-2H-thiopyranyl, Piperidinonyl, Morpholinyl, Piperazinyl oder Pyrrolidinyl ausgewählten Heterocyclus steht; Het¹⁶ für einen aus Piperidinyl oder Pyrrolidinyl ausgewählten Heterocyclus steht;
Het²⁰ für Pyrrolidinyl, 2-Pyrrolidinonyl, Piperidinyl oder Hydroxypyrrolidinyl, vorzugsweise Pyrrolidinyl oder Hydroxypyrrolidinyl, steht;
Het²¹ für Pyrrolidinyl oder Hydroxypyrrolidinyl steht;
Het²² für Pyrrolidinyl, Piperazinyl oder Piperidinyl steht;
Het²³ für einen aus Morpholinyl, Pyrrolidinyl, Piperazinyl oder Piperidinyl ausgewählten Heterocyclus steht, wobei das Het²³ durch einen oder, sofern möglich, zwei oder mehr Substituenten, die aus C₁₋₄-Alkyl, C₃₋₆-Cycloalkyl, Hydroxy-C₁₋₄alkyl-, C₁₋₄-Alkyloxy-C₁₋₄-alkyl oder Polyhydroxy-C₁₋₄-alkyl- ausgewählt sind, substituiert ist;
Ar⁴, Ar⁵ oder Ar⁶ jeweils unabhängig für Phenyl, das gegebenenfalls durch Nitro, Cyano, C₁₋₄-Alkylsulfonyl-, C₁₋₄-Alkylsulfonylamino-, Aminosulfonylamino-, Hydroxy-C₁₋₄-alkyl, Aminosulfonyl-, Hydroxy-, C₁₋₄-Alkyloxy- oder C₁₋₄-Alkyl substituiert ist, stehen, vorzugsweise Ar⁴ oder Ar⁵ jeweils unabhängig für Phenyl, das gegebenenfalls durch Cyano substituiert ist, stehen.

7. Verbindung nach einem der Ansprüche 1 bis 6, wobei der X²-Substi tuent in Position 2' steht, der R¹-Substituent für Wasserstoff oder Halogen steht und in Position 4' steht, der R²-Substituent für Halogen steht und in Position 5' steht, der R³-Substituent in Position 2 steht und der R⁴-Substituent in Position 7 der Struktur der Formel (I) steht.

8. Verbindung nach einem der Ansprüche 1 bis 4 oder 6 bis 7, wobei
R⁴ für C₁₋₄-Alkyloxy, das durch Hydroxy und einen Substituenten, der aus NR⁷R⁸ oder Het²- ausgewählt ist, substituiert ist, steht.

9. Kinaseinhibitor der Formel (I) gemäß Anspruch 1.

10. Verbindung nach einem der Ansprüche 1 bis 8 zur Verwendung als Medizin.

11. Verwendung einer Verbindung nach einem der Ansprüche 1 bis 8 bei der Herstellung eines Medikaments zur Behandlung von Zellproliferationsstörungen wie Atherosklerose, Restenose und Krebs.

12. Pharmazeutische Zusammensetzung, umfassend einen pharmazeutisch unbedenklichen Träger und als Wirkstoff eine wirksame kinasehemmende Menge einer Verbindung gemäß einem der Ansprüche 1 bis 8.

## Revendications

1. Composé ayant la formule 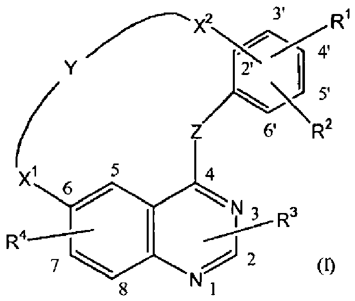 les formes de N-oxyde, les sels d'addition pharmaceutiquement acceptables et les formes d'isomère stéréochimique de celui-ci, dans lequel
Z représente NH ;
Y représente -(alkyle en C₃₋₉)-, -(alcényle en C₂₋₉)-, - (alkyle en C₁₋₅)-oxy-(alkyle en C₁₋₅)-, -(alkyle en C₁₋₅) NR¹³-(alkyle en C₁-₅)-, -(alkyle en C₁₋₅)-NR¹⁴-CO- (alkyle en C₁₋₅)-, -(alkyle en C₁₋₆) -NH-CO-, -NH-CO- (alkyle en C₁₋₆)-, -CO-(alkyle en C₁₋₇)-, -(alkyle en C₁₋₇)-CO-, (alkyle en C₁₋₆)-CO-(alkyle en C₁₋₆), - (alkyle en C₁₋₂)-NR²³-CO-CR¹⁶R¹⁷-NH-, -(alkyle en C₁₋₂)-CO-NH-CR¹⁸R¹⁹-CO-,-(alkyle en C₁₋₂)-CO-NR²⁰- (alkyle en C₁₋₃)-CO-, -(alkyle en C₁₋₂)-NR²¹-CH₂-CO-NH- (alkyle en C₁₋₃)-, -NR²²-CO-(alkyle en C₁₋₃)-NH-, -(alkyle en C₁₋₃) -NH-CO-Het²⁰-, (alkyle en C₁₋₂) -CO-Het²¹-CO-, ou -Het²²-CH₂-CO-NH-(alkyle en C₁₋₃)- ; X¹ représente O, -O-(alkyle en C₁₋₂)-, -O-N=CH-, NR¹¹ ou -NR¹¹-(alkyle en C₁₋₂)- ;
X² représente une liaison directe, alkyle en C₁₋₂, O, -O-(alkyle en C₁₋₂)-, CO, -CO-(alkyle en C₁₋₂)-, -O-N=CH-, NR¹² ou NR¹²-(alkyle en C₁₋₂)- ;
R¹ représente hydrogène, cyano, halogéno ou hydroxy ;
R² représente hydrogène, cyano, halogéno, hydroxy, hydroxycarbonyl-, (alkyloxy en C₁₋₄)carbonyl-, Het¹⁶-carbonyl-, (alkyle en C₁₋₄)-, (alcynyle en C₂₋₆)-, Ar⁵, Het¹ ou dihydroxyborane ;
R³ représente hydrogène, cyano, halogéno, hydroxy, formyle, (alcoxy en C₁₋₆)-, (alkyle en C₁₋₆)-, (alcoxy en C₁₋₆)- substitué par halogéno, ou R³ représente alkyle en C₁₋₄ substitué par un ou, si possible, deux substituants ou plus choisis parmi hydroxy ou halogéno ;
R⁴ représente Ar⁴- (alkyloxy en C₁₋₄)-, (alkyloxy en C₁₋₄)- ou R⁴ représente alkyloxy en C₁₋₄ substitué par un ou, si possible, deux substituants ou plus choisis parmi hydroxy-, halogéno, (alkyloxy en C₁₋₄)-, (alkyloxy en C₁₋₄)- (alkyloxy en C₁₋₄)-, NR³⁷R³⁸-carbonyloxy-, Het⁵-carbonyloxy-, NR⁷R⁸, NR⁹R¹⁰-carbonyl-, Het³-carbonyl-, Het¹³-oxy- ou Het²- ;
R⁷ représente hydrogène, hydroxy-(alkyle en C₁₋₄)- ou alkyle en C₁₋₄ ;
R⁸ représente cycloalkyle en C₃₋₆ ; Het⁶-carbonyl-Het⁷-aminocarbonyl- ; Het⁸; Het⁹-oxycarbonyl- ; Het¹⁰-sulfonyl- ; (alkyloxy en C₁₋₄) carbonyle ; mono- ou di (alkyle en C₁₋₄)aminocarbonyl- ; mono- ou di (alkyle en C₁₋₄)aminocarbonyle substitué par (alkyle en C₁₋₄)sulfonyl- ; ou
(alkyle en C₁₋₄)carbonyle facultativement substitué par un ou plusieurs substituants choisis parmi (alkyle en C₁₋₄)sulfonyle, hydroxy- et (alkyloxy en C₁₋₄)- ; ou
R⁸ représente alkyle en C₁₋₄ substitué par un ou plusieurs substituants choisis parmi (alkyle en C₁₋₄)sulfonyl-, NR²⁵R²⁶, aminocarbonyloxy-, (alkyle en C₁₋₄)carbonyloxy-, aminocarbonyl-, hydroxy- (alkyloxy en C₁₋₄)-, (alkyloxy en C₁₋₄)-(alkyloxy en C₁₋₄)-, et Het¹¹ ; R⁹ représente hydrogène ou (alkyle en C₁₋₄)- ;
R¹⁰ représente Het⁴ ou (alkyle en C₁₋₄)- substitué par (alkyle en C₁₋₄)sulfonyl- ;
R¹¹ représente hydrogène, (alkyle en C₁₋₄)- ou (alkyle en C₁₋₄)-oxy-carbonyl- ;
R¹² représente hydrogène, (alkyle en C₁₋₄)-, (alkyloxy en C₁₋₆) carbonyl- ou (alkyloxy en C₁₋₆) carbonyl- substitué par phényle ;
R¹³ représente hydrogène, Het¹⁴- (alkyle en C₁₋₄), (alkyloxy en C₁₋₆)carbonyle facultativement substitué par phényle ou R¹³ représente Ar⁶-sulfonyle ou Het²⁴-(alkyle en C₁₋₄)carbonyle ;
R¹⁴ et R¹⁵ sont chacun indépendamment choisis parmi hydrogène, alkyle en C₁₋₄, Het¹⁵-(alkyle en C₁₋₄)- ou (alkyloxy en C₁₋₄)-(alkyle en C₁-₄)- ;
R¹⁶ et R¹⁷ représentent chacun indépendamment hydrogène, alkyle en C₁₋₄ ou alkyle en C₁₋₄ substitué par hydroxy-, cycloalkyle en C₃₋₆ ou phényle ; ou
R¹⁶ et R¹⁷, conjointement avec l'atome de carbone auquel ils sont liés, forment un cycloalkyle en C₃₋₆ ;
R¹⁸ représente hydrogène ou alkyle en C₁₋₄ facultativement substitué par hydroxy ou phényle ;
R¹⁹ représente hydrogène ou alkyle en C₁₋₄ ;
R²⁰ représente hydrogène ou alkyle en C₁₋₄ ;
R²¹ représente hydrogène, alkyle en C₁₋₄, Het²³- (alkyle en C₁₋₄)carbonyl- ou
R²¹ représente mono-ou di (alkyle en C₁₋₄) amino- (alkyle en C₁₋₄)-carbonyl- facultativement substitué par hydroxy, pyrimidinyle, diméthylamine ou alkyloxy en C₁₋₄ ;
R²² représente hydrogène ou alkyle en C₁₋₄ facultativement substitué par hydroxy ou alkyloxy en C₁₋₄ ;
R²³ représente alkyle en C₁₋₄ facultativement substitué par hydroxy-, (alkyloxy en C₁₋₄)- ou Het²⁵ ; R²³ peut également représenter hydrogène lorsque R¹⁶ et R¹⁷, conjointement avec l'atome de carbone auquel ils sont liés, forment un cycloalkyle en C₃₋₆ ;
R²⁵ et R²⁶ représentent chacun indépendamment hydrogène, alkyle en C₁₋₄, (alkyle en C₁₋₄)sulfonyl-, aminocarbonyl-, mono- ou di (alkyle en C₁₋₄)aminocarbonyl-, (alkyle en C₁₋₄)carbonyl-, (alkyloxy en C₁₋₄)carbonyl- ou alkyle en C₁₋₄ substitué par un ou plusieurs substituants choisis parmi (alkyle en C₁₋₄)sulfonyl-, hydroxy- et (alkyloxy en C₁₋₄)- ;
R²⁷ et R²⁸ représentent chacun indépendamment hydrogène, alkyle en C₁₋₄, (alkyle en C₁₋₄)sulfonyl-, aminocarbonyl-, mono- ou di (alkyle en C₁₋₄)aminocarbonyl-, (alkyle en C₁₋₄)carbonyl-, (alkyloxy en C₁₋₄)carbonyl- ou alkyle en C₁₋₄ substitué par un ou plusieurs substituants choisis parmi (alkyle en C₁₋₄)sulfonyl-, hydroxy- et (alkyloxy en C₁₋₄)- ; ou, pour les composés de formule (I) dans lesquels Het² représente un hétérocycle choisi parmi morpholinyle, pipérazinyle, pipéridinyle, pyrrolidinyle ou thiomorpholinyle substitué par NR²⁷R²⁸- (alkyle en C₁₋₄), lesdits R²⁷ et R²⁸ représentent chacun indépendamment (alkyle en C₁₋₄)sulfonyl-, aminocarbonyl-, mono- ou di (alkyle en C₁₋₄)aminocarbonyl-, (alkyle en C₁₋₄)carbonyl-, (alkyloxy en C₁₋₄)carbonyl- ou alkyle en C₁₋₄ substitué par un ou plusieurs substituants choisis parmi (alkyle en C₁₋₄)sulfonyl-, hydroxy- et (alkyloxy en C₁₋₄)- ;
R²⁹ et R³⁰ représentent chacun indépendamment hydrogène, aminosulfonyle, aminocarbonyle, mono- ou di(alkyle en C₁₋₄)aminocarbonyl-, mono- ou di (alkyle en C₁₋₄)aminosulfonyl-, ou
(alkyle en C₁₋₄)- facultativement substitué par un ou plusieurs substituants choisis parmi NR³¹R³², (alkyle en C₁₋₄)sulfonyle, aminocarbonyloxy-, hydroxy-, (alkyloxy en C₁₋₄)-, aminocarbonyl- et mono- ou di (alkyle en C₁₋₄)aminocarbonyl-, ou
(alkyloxy en C₁₋₄)carbonyle facultativement substitué par un ou plusieurs substituants choisis parmi hydroxy, (alkyloxy en C₁₋₄)- et (alkyle en C₁₋₄)sulfonyl-, ou (alkyle en C₁₋₄)carbonyle facultativement substitué par un ou plusieurs substituants choisis parmi hydroxy-, (alkyloxy en C₁₋₄)- et (alkyle en C₁₋₄)sulfonyl- ;
R³¹ et R³² représentent chacun indépendamment hydrogène, alkyle en C₁₋₄, (alkyle en C₁₋₄)sulfonyl-, aminocarbonyl-, mono- ou di (alkyle en C₁₋₄)aminocarbonyl-, (alkyle en C₁₋₄)carbonyl-, (alkyloxy en C₁₋₄)carbonyl- ou alkyle en C₁₋₄ substitué par un ou plusieurs substituants choisis parmi (alkyle en C₁₋₄)sulfonyl-, hydroxy- et (alkyloxy en C₁₋₄)- ;
R³³ représente hydrogène ou alkyle en C₁₋₄ ;
R³⁴ représente (alkyle en C₁₋₄)sulfonyl-, aminocarbonyl-, mono- ou di (alkyle en C₁₋₄)aminocarbonyl-, (alkyle en C₁₋₄)carbonyl-, (alkyloxy en C₁₋₄)carbonyl- ou alkyle en C₁₋₄ substitué par un ou plusieurs substituants choisis parmi (alkyle en C₁₋₄)sulfonyl-, hydroxy- et (alkyloxy en C₁₋₄)- ;
R³⁵ représente hydrogène ou alkyle en C₁₋₄ ;
R³⁶ représente (alkyle en C₁₋₄)sulfonyl-, aminocarbonyl-, mono- ou di (alkyle en C₁₋₄)aminocarbonyl-, (alkyle en C₁₋₄)carbonyl-, (alkyloxy en C₁₋₄)carbonyl- ou alkyle en C₁₋₄ substitué par un ou plusieurs substituants choisis parmi (alkyle en C₁₋₄)sulfonyl-, hydroxy- et (alkyloxy en C₁₋₄)- ;
R³⁷ et R³⁸ représentent chacun indépendamment hydrogène, alkyle en C₁₋₄, (alkyle en C₁₋₄)sulfonyl-, Het¹² ou alkyle en C₁₋₄ substitué par un ou plusieurs substituants choisis parmi (alkyle en C₁₋₄)sulfonyl-, hydroxy- et (alkyloxy en C₁₋₄)- ;
R³⁹ et R⁴⁰ représentent chacun indépendamment aminosulfonyle, aminocarbonyl, mono- ou di(alkyle en C₁₋₄)aminocarbonyl-, mono- ou di (alkyle en C₁₋₄)aminosulfonyl-, ou (alkyle en C₁₋₄)- substitué par un ou plusieurs substituants choisis parmi NR³¹R³², (alkyle en C₁₋₄)sulfonyle, aminocarbonyloxy-, hydroxy-, (alkyloxy en C₁₋₄)-, aminocarbonyl- et mono- ou di (alkyle en C₁₋₄)aminocarbonyl-, ou
(alkyloxy en C₁₋₄)carbonyle facultativement substitué par un ou plusieurs substituants choisis parmi hydroxy, (alkyloxy en C₁₋₄)- et (alkyle en C₁₋₄)sulfonyl-, ou (alkyle en C₁₋₄)carbonyle facultativement substitué par un ou plusieurs substituants choisis parmi hydroxy-, (alkyloxy en C₁₋₄)- et (alkyle en C₁₋₄)sulfonyl- ;
Het¹ représente thiazolyle ou 2-bora-1,3-dioxolanyle, ledit Het¹ étant facultativement substitué par un ou, si possible, deux, trois, quatre substituants ou plus choisis parmi amino, alkyle en C₁₋₄, hydroxy-(alkyle en C₁₋₄)-, phényle, phényl- (alkyle en C₁₋₄)-, (alkyle en C₁₋₄)-oxy- (alkyle en C₁₋₄)- mono- ou di (alkyle en C₁₋₄)amino- ou amino-carbonyl- ;
Het² représente un hétérocycle choisi parmi tétrahydropyranyle, tétrahydrofuranyle, furanyle, 1,1-dioxothiomorpholinyle, pipérazininonyle, tétrahydro-1,1-dioxydo-2H- thiopyranyle, pipéridinonyle, azétidinyle ou 2-azétidinonyle, ledit Het² est facultativement substitué par un ou, si possible, deux substituants ou plus choisis parmi hydroxy, amino, NR²⁹R³⁰, aminocarbonyle, mono- ou di (alkyle en C₁₋₄)aminocarbonyle, (alkyle en C₁₋₄)sulfonyle ou
(alkyle en C₁₋₄)- facultativement substitué par un ou plusieurs substituants choisis parmi
NR²⁷R²⁸, (alkyle en C₁₋₄)sulfonyle, aminocarbonyloxy-, aminocarbonyl- et mono- ou di(alkyle en C₁₋₄)aminocarbonyl-, ou
(alkyloxy en C₁₋₄)- facultativement substitué par (alkyloxy en C₁₋₄)-, ou
(alkyloxy en C₁₋₄)carbonyle facultativement substitué par un ou plusieurs substituants choisis parmi hydroxy, (alkyloxy en C₁₋₄)- et (alkyle en C₁₋₄)sulfonyl-, ou
(alkyle en C₁₋₄)carbonyle facultativement substitué par un ou plusieurs substituants choisis parmi hydroxy-, (alkyloxy en C₁₋₄)- et (alkyle en C₁₋₄)sulfonyl- ; ou
Het² représente un hétérocycle choisi parmi morpholinyle, pipérazinyle, pipéridinyle, pyrrolidinyle, thiomorpholinyle ou 1,1-dioxothiomorpholinyle, ledit Het² étant facultativement substitué par un ou, si possible, deux substituants ou plus choisis parmi
(alkyle en C₁₋₄)- facultativement substitué par un ou plusieurs substituants choisis parmi
NR²⁷R²⁸, (alkyle en C₁₋₄)sulfonyle, aminocarbonyloxy-, aminocarbonyl- et mono- ou di(alkyle en C₁₋₄)aminocarbonyl-, ou
(alkyloxy en C₁₋₄)- facultativement substitué par (alkyloxy en C₁₋₄)-, ou
(alkyloxy en C₁₋₄)carbonyle facultativement substitué par un ou plusieurs substituants choisis parmi hydroxy, (alkyloxy en C₁₋₄)- et (alkyle en C₁₋₄)sulfonyl-, ou (alkyle en C₁₋₄)carbonyle facultativement substitué par un ou plusieurs substituants choisis parmi hydroxy-, (alkyloxy en C₁₋₄)- et (alkyle en C₁₋₄)sulfonyl- ;
Het³ représente un hétérocycle choisi parmi tétrahydropyranyle, tétrahydrofuranyle, furanyle, 1,1-dioxothiomorpholinyle, pipérazininonyle, tétrahydro-1,1-dioxydo-2H- thiopyranyle, pipéridinonyle, azétidinyle ou 2-azétidinonyle, ledit Het³ étant facultativement substitué par un ou, si possible, deux substituants ou plus hydroxy-, amino, (alkyle en C₁₋₄)-, (cycloalkyle en C₃₋₆)-(alkyle en C₁₋₄)-, aminosulfonyl-, mono- ou di (alkyle en C₁₋₄)aminosulfonyl-, amino-(alkyle en C₁₋₄)-, mono- ou di (alkyle en C₁₋₄)amino-(alkyle en C₁₋₄), NR³⁵R³⁶, (alkyle en C₁₋₄)-sulfonyl- (alkyle en C₁₋₄)- ou (alkyloxy en C₁₋₄)- facultativement substitué par (alkyloxy en C₁₋₄)- ou hydroxy ; ou
Het³ représente un hétérocycle choisi parmi morpholinyle, pipérazinyle, pipéridinyle, furanyle ou pyrrolidinyle, ledit Het³ étant substitué par un ou, si possible, deux substituants ou plus choisis parmi NR³⁵R³⁶, (alkyle en C₁₋₄)-sulfonyl- (alkyle en C₁₋₄)- ou (alkyloxy en C₁₋₄)- facultativement substitué par (alkyloxy en C₁₋₄)- ou hydroxy ;
Het⁴ représente un hétérocycle choisi parmi morpholinyle, pipérazinyle, pipéridinyle, furanyle, pyrazolyle, dioxolanyle, thiazolyle, oxazolyle, imidazolyle, isoxazolyle, oxadiazolyle, pyridinyle ou pyrrolidinyle, ledit Het⁴ étant substitué par un ou, si possible, deux substituants ou plus choisis parmi (alkyle en C₁₋₄)-sulfonyl- (alkyle en C₁₋₄)-, (alkyloxy en C₁₋₄)- facultativement substitué par (alkyloxy en C₁₋₄)- ou hydroxy ;
Het⁵ représente un hétérocycle choisi parmi furanyle, pipérazinyle, 1,1-dioxothiomorpholinyle, pipérazininonyle, pipéridinyle, tétrahydro-1,1-dioxydo-2H- thiopyranyle, pipéridinonyle, morpholinyle ou pyrrolidinyle, ledit Het⁵ étant facultativement substitué par hydroxy, amino, mono- ou di(alkyle en C₁₋₄) -amino-, alkyle en C₁₋₄,
Het⁶ et Het⁷ représentent chacun indépendamment un hétérocycle choisi parmi pipérazinyle, pipéridinyle ou pyrrolidinyle, lesdits hétérocycles étant facultativement substitués par un ou plusieurs substituants choisis parmi hydroxy-, amino, hydroxy-(alkyle en C₁₋₄)-, (alkyloxy en C₁₋₄)- (alkyle en C₁₋₄)- et (alkyle en C₁₋₄)- ;
Het⁸ représente un hétérocycle choisi parmi tétrahydropyranyle, tétrahydrofuranyle, 1,1-dioxothiomorpholinyle, pipérazininonyle, tétrahydro-1,1-dioxydo-2H-thiopyranyle, pipéridinonyle, azétidinyle ou 2-azétidinonyle, ledit Het⁸ étant facultativement substitué par aminosulfonyle, aminocarbonyle, mono- ou di(alkyle en C₁₋₄)aminocarbonyl-, mono- ou di (alkyle en C₁₋₄)aminosulfonyl-, ou (alkyle en C₁₋₄)- facultativement substitué par un ou plusieurs substituants choisis parmi amino, mono- ou di (alkyle en C₁₋₄)amino-, NR³³R³⁴, (alkyle en C₁₋₄)sulfonyle, aminocarbonyloxy-, hydroxy-, (alkyloxy en C₁₋₄)-, aminocarbonyl- et mono- ou di (alkyle en C₁₋₄)aminocarbonyl-, ou
(alkyloxy en C₁₋₄)carbonyle facultativement substitué par un ou plusieurs substituants choisis parmi hydroxy, (alkyloxy en C₁₋₄)- et (alkyle en C₁₋₄)sulfonyl-, ou
(alkyle en C₁₋₄)carbonyle facultativement substitué par un ou plusieurs substituants choisis parmi hydroxy, (alkyloxy en C₁₋₄)- et (alkyle en C₁₋₄)sulfonyl- ; ou
Het⁸ représente un hétérocycle choisi parmi furanyle, pipéridinyle ou pipérazinyle, ledit Het⁸ étant substitué par aminocarbonyle, mono- ou di(alkyle en C₁₋₄)aminocarbonyl-, mono- ou di (alkyle en C₁₋₄)aminosulfonyl-, ou
(alkyle en C₁₋₄)- substitué par un ou plusieurs substituants choisis parmi NR³³R³⁴, (alkyle en C₁₋₄)sulfonyle, aminocarbonyloxy-, hydroxy-, (alkyloxy en C₁₋₄)-, aminocarbonyl- et mono- ou di (alkyle en C₁₋₄) aminocarbonyl-, ou
(alkyloxy en C₁₋₄)carbonyle facultativement substitué par un ou plusieurs substituants choisis parmi hydroxy, (alkyloxy en C₁₋₄)- et (alkyle en C₁₋₄)sulfonyl-, ou
(alkyle en C₁₋₄)carbonyle facultativement substitué par un ou plusieurs substituants choisis parmi hydroxy, (alkyloxy en C₁₋₄)- et (alkyle en C₁₋₄)sulfonyl- ;
Het⁹ et Het¹⁰ représentent chacun indépendamment un hétérocycle choisi parmi pipérazinyle, pipéridinyle ou pyrrolidinyle, lesdits hétérocycles étant facultativement substitués par un ou plusieurs substituants choisis parmi hydroxy-, amino, hydroxy-(alkyle en C₁₋₄)-, (alkyloxy en C₁₋₄)-(alkyle en C₁₋₄)- et (alkyle en C₁₋₄)- ;
Het¹¹ représente 2-imidazolidinonyl-ou 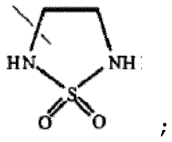 Het¹² représente un hétérocycle choisi parmi morpholinyle, pipérazinyle, pipéridinyle ou pyrrolidinyle, ledit Het¹² étant facultativement substitué par un ou, si possible, deux substituants ou plus choisis parmi hydroxy, amino ou (alkyle en C₁₋₄)- ;
Het¹³ représente un hétérocycle choisi parmi furanyle, pipérazinyle, 1,1-dioxothiomorpholinyle, pipérazininonyle, pipéridinyle, tétrahydro-1,1-dioxydo-2H- thiopyranyle, pipéridinonyle, morpholinyle, pipérazinyle ou pyrrolidinyle ;
Het¹⁴ et Het¹⁵ représentent chacun indépendamment un hétérocycle choisi parmi morpholinyle, pipérazinyle, pipéridinyle ou pyrrolidinyle, lesdits Het¹⁴ et Het¹⁵ étant facultativement substitués par un ou, si possible, deux substituants ou plus choisis parmi hydroxy, amino ou alkyle en C₁₋₄ ;
Het¹⁶ représente un hétérocycle choisi parmi pipéridinyle ou pyrrolidinyle ;
Het²⁰ représente pyrrolidinyle, 2-pyrrolidinonyle, pipéridinyle ou hydroxy-pyrrolidinyle ;
Het²¹ représente pyrrolidinyle ou hydroxy-pyrrolidinyle ;
Het²² représente pyrrolidinyle, pipérazinyle ou pipéridinyle ;
Het²³ et Het²⁵ représentent chacun indépendamment un hétérocycle choisi parmi morpholinyle, pyrrolidinyle, pipérazinyle ou pipéridinyle, ledit Het²³ étant facultativement substitué par un ou, si possible, deux substituants ou plus choisis parmi alkyle en C₁₋₄, cycloalkyle en C₃₋₆, hydroxy-(alkyle en C₁₋₄)-, (alkyloxy en C₁₋₄) alkyle en C₁₋₄ ou polyhydroxy- (alkyle en C₁₋₄)- ;
Het²⁴ représente morpholinyle, pyrrolidinyle, pipérazinyle ou pipéridinyle ;
Ar⁴, Ar⁵ ou Ar⁶ représentent chacun indépendamment phényle facultativement substitué par nitro, cyano, (alkyle en C₁₋₄)sulfonyl-, (alkyle en C₁₋₄)sulfonylamino-, aminosulfonylamino-, hydroxy-(alkyle en C₁₋₄), aminosulfonyl-, hydroxy-, (alkyloxy en C₁₋₄)- ou alkyle en C₁₋₄ ;
**caractérisé en outre en ce que**
Y¹ représente - (alkyle en C₁₋₂)-NR²³-CO-CR¹⁶R¹⁷-NH- ;
Het¹ représente 2-bora-1,3-dioxolanyle facultativement substitué par un ou, si possible, deux, trois, quatre substituants ou plus choisis parmi amino, alkyle en C₁₋₄, hydroxy-(alkyle en C₁₋₄)-, phényle, phényl- (alkyle en C₁₋₄)-, (alkyle en C₁₋₄)-oxy- (alkyle en C₁₋₄)- mono- ou di (alkyle en C₁₋₄)amino- ou amino-carbonyl- ;
R¹³ représente (alkyloxy en C₁₋₆) carbonyle facultativement substitué par phényle ou R¹³ représente Ar⁶-sulfonyle ou Het²⁴- (alkyle en C₁₋₄)carbonyle ; ou
R⁴ représente alkyloxy en C₁₋₄ substitué par au moins un substituant choisi parmi (alkyloxy en C₁₋₄)-(alkyloxy en C₁₋₄)-, NR³⁷R³⁸-carbonyloxy-, Het⁵ -carbonyloxy-, NR⁷R⁸, NR⁹R¹⁰-carbonyl-, Het³-carbonyl-, Het¹³-oxy- ou Het²- ; où R⁸ représente Het⁷-aminocarbonyl- ; Het⁹-oxycarbonyl- ; Het¹⁰-sulfonyl- ; (alkyloxy en C₁₋₄)carbonyle ; mono- ou di (alkyle en C₁₋₄)aminocarbonyl- ; mono- ou
di (alkyle en C₁₋₄)aminocarbonyle substitué par (alkyle en C₁₋₄)sulfonyl- ; ou
(alkyle en C₁₋₄)carbonyle facultativement substitué par un ou plusieurs substituants choisis parmi (alkyle en C₁₋₄)sulfonyle, hydroxy- et (alkyloxy en C₁₋₄)- ; ou
R⁸ représente alkyle en C₁₋₄ substitué par un ou plusieurs substituants choisis parmi (alkyle en C₁₋₄)sulfonyl-, NR²⁵R²⁶, aminocarbonyloxy-, (alkyle en C₁₋₄)carbonyloxy-, aminocarbonyl-, (alkyloxy en C₁₋₄)-(alkyloxy en C₁₋₄)-, et Het¹¹ ; et
Het² représente un hétérocycle choisi parmi morpholinyle, pipérazinyle, pipéridinyle, pyrrolidinyle ou thiomorpholinyle, ledit Het² étant substitué par un ou, si possible, deux substituants ou plus choisis parmi
(alkyle en C₁₋₄)- substitué par un ou plusieurs substituants choisis parmi NR²⁷R²⁸, (alkyle en C₁₋₄)sulfonyle, aminocarbonyloxy-, aminocarbonyl- et mono- ou di (alkyle en C₁₋₄)aminocarbonyl- ; ou
(alkyloxy en C₁₋₄)- facultativement substitué par (alkyloxy en C₁₋₄)- ;
ou (alkyloxy en C₁₋₄)carbonyle facultativement substitué par un ou plusieurs substituants choisis parmi hydroxy, (alkyloxy en C₁₋₄)- et (alkyle en C₁₋₄)sulfonyl- ;
ou (alkyle en C₁₋₄)carbonyle facultativement substitué par un ou plusieurs substituants choisis parmi hydroxy-, (alkyloxy en C₁₋₄)- et (alkyle en C₁₋₄)sulfonyl- ;
ou Het² représente 1,1-dioxothiomorpholinyle facultativement substitué par (alkyle en C₁₋₄)-facultativement substitué par un ou plusieurs substituants choisis parmi NR²⁷R²⁸, (alkyle en C₁₋₄)sulfonyle, aminocarbonyloxy-, aminocarbonyl- et mono- ou di (alkyle en C₁₋₄)aminocarbonyl- ; ou
(alkyloxy en C₁₋₄)- facultativement substitué par (alkyloxy en C₁₋₄)- ; ou
(alkyloxy en C₁₋₄)carbonyle facultativement substitué par un ou plusieurs substituants choisis parmi hydroxy, (alkyloxy en C₁₋₄)- et (alkyle en C₁₋₄)sulfonyl- ; ou
(alkyle en C₁₋₄)carbonyle facultativement substitué par un ou plusieurs substituants choisis parmi hydroxy-, (alkyloxy en C₁₋₄)- et (alkyle en C₁₋₄)sulfonyl-.

2. Composé selon la revendication 1 dans lequel ;
Z représente NH ;
Y représente - (alkyle en C₃₋₉)-, - (alkyle en C₁₋₅)-NR¹³-(alkyle en C₁₋₅)-, -(alkyle en C₁₋₅)-NR¹⁴-CO- (alkyle en C₁₋₅)-, -(alkyle en C₁₋₆)-CO-NH-, -(alkyle en C₁₋₆)-NH-CO-, -(alkyle en C₁₋₂)-NR²³-CO-CR¹⁶R¹⁷-NH-, -(alkyle en C₁₋₂)-NR²¹-CH₂-CO-NH-(alkyle en C₁₋₃) ou (alkyle en C₁₋₃)-NH-CO-Het²⁰- ;
X¹ représente O, -O-(alkyle en C₁₋₂)-, NR¹¹, ou -NR¹¹-(alkyle en C₁₋₂)- ;
X² représente une liaison directe, - (alkyle en C₁₋₂)-, CO-(alkyle en C₁₋₂) ou NR¹²-(alkyle en C₁₋₂)- ;
R¹ représente hydrogène, cyano, halogéno ou hydroxy ;
R² représente hydrogène, halogéno, cyano, alcynyle en C₂₋₆, hydroxy, hydroxycarbonyle, (alkyloxy en C₁₋₄)carbonyl- ou Het¹ ;
R³ représente hydrogène, cyano, halogéno, hydroxy, formyle, alkyloxy en C₁₋₆ ou (alkyloxy en C₁₋₆)-substitué par halogéno ;
R⁴ représente Ar⁴- (alkyloxy en C₁₋₄), (alkyloxy en C₁₋₄)-, ou (alkyloxy en C₁₋₄)- substitué par un ou, si possible, deux substituants ou plus choisis parmi hydroxy, (alkyloxy en C₁₋₄)-, (alkyloxy en C₁₋₄)-(alkyloxy en C₁₋₄), NR⁷R⁸ ou Het² ;
R⁷ représente hydrogène, hydroxy (alkyle en C₁₋₄)- ou alkyle en C₁₋₄ ;
R⁸ représente (alkyloxy en C₁₋₄)carbonyle ou (alkyle en C₁₋₄)- substitué par un ou plusieurs substituants choisis parmi (alkyle en C₁₋₄)sulfonyl-, (alkyle en C₁₋₄)carbonyloxy ou NR²⁵R²⁶; en particulier, R⁸ représente (alkyle en C₁₋₄)- substitué par un ou plusieurs substituants choisis parmi (alkyle en C₁₋₄)sulfonyl- ou NR²⁵R²⁶;
R¹¹ représente hydrogène, (alkyloxy en C₁₋₄)carbonyle ou alkyle en C₁₋₄ ;
R¹² représente hydrogène ou alkyle en C₁₋₄ ;
R¹³ représente (alkyloxy en C₁₋₆) carbonyle facultativement substitué par phényle ou R¹³ représente Ar⁶-sulfonyle ou Het²⁴- (alkyle en C₁₋₄)carbonyle ;
R¹⁴ et R¹⁵ représentent chacun indépendamment hydrogène ou alkyle en C₁₋₄ ;
R¹⁶ et R¹⁷ représentent chacun indépendamment hydrogène ou alkyle en C₁₋₄ facultativement substitué par cycloalkyle en C₃₋₆ ou R¹⁶ et R¹⁷, conjointement avec l'atome de carbone auquel ils sont liés, forment un cycloalkyle en C₃₋₆ ;
R²¹ représente hydrogène ;
R²³ représente alkyle en C₁₋₄ facultativement substitué par hydroxy-, (alkyloxy en C₁₋₄)- ou Het²⁵ ;
R²³ peut également représenter hydrogène lorsque R¹⁶ et R¹⁷, conjointement avec l'atome de carbone auquel ils sont liés, forment un cycloalkyle en C₃₋₆ ;
R²⁵ et R²⁶ représentent chacun indépendamment hydrogène, alkyle en C₁₋₄, (alkyle en C₁₋₄)sulfonyle, (alkyloxy en C₁₋₄)carbonyle ou (alkyle en C₁₋₄)carbonyle ;
R²⁷ et R²⁸ représentent chacun indépendamment hydrogène, alkyle en C₁₋₄, (alkyle en C₁₋₄)sulfonyle, (alkyloxy en C₁₋₄)carbonyle ou (alkyle en C₁₋₄)carbonyle ;
Het¹ représente 2-bora-1,3-dioxolanyl- facultativement substitué par un ou, si possible, deux, trois, quatre substituants ou plus choisis parmi amino, alkyle en C₁₋₄, hydroxy-(alkyle en C₁₋₄)-, phényle, phényl- (alkyle en C₁₋₄), (alkyloxy en C₁₋₄)-(alkyle en C₁₋₄)-, mono- ou di (alkyle en C₁₋₄)amino- ou aminocarbonyl- ;
Het² représente 1,1-dioxothiomorpholinyle facultativement substitué par (alkyloxy en C₁₋₄)carbonyle ou (alkyle en C₁₋₄)-NR²⁷R²⁸ ou Het² représente pipéridinyle ou pipérazinyle substitué par (alkyloxy en C₁₋₄)carbonyle ou - (alkyle en C₁₋₄)-NR²⁷R²⁸; Het²⁰ représente pyrrolidinyle, 2-pyrrolidinonyle, pipéridinyle ou hydroxy-pyrrolidinyle ;
Het²⁵ représente un hétérocycle choisi parmi morpholinyle ou pipérazinyle, ledit hétérocycle étant facultativement substitué par alkyle en C₁₋₄, hydroxy-(alkyle en C₁₋₄), (alkyloxy en C₁₋₄)-(alkyle en C₁₋₄) ou polyhydroxy- (alkyle en C₁₋₄); ou
Ar⁴, Ar⁵ ou Ar⁶ représentent chacun indépendamment phényle facultativement substitué par nitro, cyano, hydroxy, hydroxyalkyle en C₁₋₄, alkyle en C₁₋₄ ou alkyloxy en C₁₋₄ ;
**caractérisé en outre en ce que**
Y représente - (alkyle en C₁₋₂)-NR²³-CO-CR¹⁶R¹⁷-_{NH}- ; ou
R⁴ représente alkyloxy en C₁₋₄ substitué par au moins un substituant choisi parmi (alkyloxy en C₁₋₄)-(alkyloxy en C₁₋₄)-, NR⁷R⁸ ou Het².

3. Composé selon les revendications 1 ou 2 dans lequel ;
Z représente NH ;
Y représente - (alkyle en C₃₋₉)-, -(alkyle en C₁₋₅)-NR¹³-(alkyle en C₁₋₅)-, -(alkyle en C₁₋₅)-NR¹⁴-CO-(alkyle en C₁₋₅)-, -(alkyle en C₁₋₆)-CO-NH-, -(alkyle en C₁₋₆)-NH-CO-, -(alkyle en C₁₋₂)-NR²³-CO-CR¹⁶R¹⁷-NH-, -(alkyle en C₁₋₂)-NR²¹-CH₂-CO-NH-(alkyle en C₁₋₃) ou (alkyle en C₁₋₃)-NH-CO-Het²⁰- ;
X¹ représente O, -O-(alkyle en C₁₋₂)-, NR¹¹, ou -NR¹¹-(alkyle en C₁₋₂)- ;
X² représente une liaison directe, - (alkyle en C₁₋₂)-, CO- (alkyle en C₁₋₂) ou NR¹²-(alkyle en C₁₋₂)- ;
R¹ représente hydrogène ou halogéno ;
R² représente hydrogène, halogéno, alcynyle en C₂₋₆, cyano ou Het¹ ;
R³ représente hydrogène ;
R⁴ représente Ar⁴- (alkyloxy en C₁₋₄), (alkyloxy en C₁₋₄)-, ou (alkyloxy en C₁₋₄)- substitué par un ou, si possible, deux substituants ou plus choisis parmi hydroxy, (alkyloxy en C₁₋₄)-, (alkyloxy en C₁₋₄)- alkyloxy en C₁₋₄, NR⁷R⁸ ou Het² ;
R⁷ représente hydrogène ou alkyle en C₁₋₄ ;
R⁸ représente (alkyloxy en C₁₋₄) carbonyle ou (alkyle en C₁₋₄)- substitué par un ou plusieurs substituants choisis parmi (alkyle en C₁₋₄)sulfonyl-, (alkyle en C₁₋₄)carbonyloxy ou NR²⁵R²⁶;
R¹¹ représente hydrogène ou alkyle en C₁₋₄ ;
R¹² représente hydrogène ou alkyle en C₁₋₄ ;
R¹³ représente Ar⁶-sulfonyle ou (alkyloxy en C₁₋₆)carbonyle facultativement substitué par phényle ;
R¹⁴ et R¹⁵ représentent hydrogène ;
R¹⁶ et R¹⁷ représentent chacun indépendamment hydrogène ou alkyle en C₁₋₄ facultativement substitué par cycloalkyle en C₃₋₆ ou R¹⁶ et R¹⁷, conjointement avec l'atome de carbone auquel ils sont liés, forment un cycloalkyle en C₃₋₆ ;
R²¹ représente hydrogène ;
R²³ représente alkyle en C₁₋₄ facultativement substitué par hydroxy-, (alkyloxy en C₁₋₄)- ou Het²⁵ ; R²³ peut également représenter hydrogène lorsque R¹⁶ et R¹⁷, conjointement avec l'atome de carbone auquel ils sont liés, forment un cycloalkyle en C₃₋₆ ;
R²⁵ et R²⁶ représentent chacun indépendamment hydrogène ou (alkyle en C₁₋₄)carbonyle ;
R²⁷ et R²⁸ représentent chacun indépendamment hydrogène ou (alkyle en C₁₋₄)carbonyle ;
Het¹ représente 2-bora-1,3-dioxolanyl- ;
Het² représente 1,1-dioxothiomorpholinyle, pipéridinyle ou pipérazinyle, ledit Het² étant facultativement substitué par (alkyloxy en C₁₋₄)carbonyle ou-(alkyle en C₁₋₄)-NR²⁷R²⁸;
Het²⁰ représente pyrrolidinyle ;
Het²⁵ représente un hétérocycle choisi parmi morpholinyle ou pipérazinyle, ledit hétérocycle étant facultativement substitué par alkyle en C₁₋₄, hydroxy-(alkyle en C₁₋₄), (alkyloxy en C₁₋₄)-(alkyle en C₁₋₄) ou polyhydroxy- (alkyle en C₁₋₄) ;
Ar⁴ représente phényle ;
Ar⁵ représente phényle ; ou
Ar⁶ représente phényle facultativement substitué par nitro ;
**caractérisé en outre en ce que**
Y représente - (alkyle en C₁₋₂)-NR²³-CO-CR¹⁶R¹⁷-NH- ; ou
R⁴ représente alkyloxy en C₁₋₄ substitué par au moins un substituant choisi parmi (alkyloxy en C₁₋₄)-(alkyloxy en C₁₋₄)-, NR⁷R⁸ ou Het² ; en particulier alkyloxy en C₁₋₄ substitué par (alkyloxy en C₁₋₄)-(alkyloxy en C₁₋₄)- ou NR⁷R⁸.

4. Composé selon l'une quelconque des revendications 1 à 3 dans lequel ;
Z représente NH ;
Y représente -(alkyle en C₃₋₉)-, -(alkyle en C₁₋₅)-NR¹³-(alkyle en C₁₋₅)-, -(alkyle en C₁₋₅)-NR¹⁴-CO- (alkyle en C₁₋₅₎-, -(alkyle en C₁₋₂)-NR²¹ -CH₂-CO-NH- (alkyle en C₁₋₃)-ou -(alkyle en C₁₋₂)-NR²³-CO-CR¹⁶R-NH- ;
X¹ représente O ou -O-(alkyle en C₁₋₂)- ;
X² représente une liaison directe, alkyle en C₁₋₂, -CO-(alkyle en C₁₋₂) ou NR¹²- (alkyle en C₁₋₂) ;
R¹ représente hydrogène ou halogéno ; en particulier R¹ représente hydrogène ;
R² représente halogéno, acétylène ou Het¹ ; en particulier R² représente halogéno ou Het¹ ;
R³ représente hydrogène ;
R⁴ représente Ar⁴-(alkyloxy en C₁₋₄)-, (alkyloxy en C₁₋₄)-ou alkyloxy en C₁₋₄ substitué par un ou, si possible, deux substituants ou plus choisis parmi Het², NR⁷R⁸, hydroxy et (alkyloxy en C₁₋₄)-(alkyloxy en C₁₋₄)- ;
R⁷ représente hydrogène ou alkyle en C₁₋₄ ;
R⁸ représente alkyle en C₁₋₄ substitué par NR²⁵R²⁶ ou (alkyle en C₁₋₄)sulfonyle ;
R¹² représente hydrogène ou (alkyle en C₁₋₄)- ;
R¹³ représente Ar⁶-sulfonyle ou (alkyloxy en C₁₋₆)carbonyle facultativement substitué par phényle ;
R¹⁶ et R¹⁷ représentent hydrogène, alkyle en C₁₋₄ ou R¹⁶ et R¹⁷, conjointement avec l'atome de carbone auquel ils sont liés, forment cycloalkyle en C₃₋₆ ;
R²³ représente hydrogène ou alkyle en C₁₋₄ ;
R²⁵ et R²⁶ représentent chacun indépendamment hydrogène ou (alkyle en C₁₋₄)carbonyle ;
R²⁷ et R²⁸ représentent chacun indépendamment hydrogène ou (alkyle en C₁₋₄)carbonyle ;
Het¹ représente 2-bora-1,3-dioxolanyle ;
Het² représente pipéridinyle, pipérazinyle, morpholinyle, thiomorpholinyle ou 1,1-dioxothiomorpholinyle, ledit Het² étant facultativement substitué par (alkyloxy en C₁₋₄)carbonyle ou NR²⁷R²⁸-(alkyle en C₁₋₄) ;
Ar⁴ représente phényle ;
Ar⁵ représente phényle ; ou
Ar⁶ représente phényle facultativement substitué par nitro.

5. Composé selon l'une quelconque des revendications 1 à 3 dans lequel ;
Z représente NH ;
Y représente - (alkyle en C₃₋₉)-, -(alkyle en C₁₋₅)-NR¹³-(alkyle en C₁₋₅)- ou -(alkyle en C₁₋₂)-NR²³-CO-CR¹⁶R¹⁷-NH-
X¹ représente O ; X² représente une liaison directe ou NR¹²-(alkyle en C₁₋₂)- ;
R¹ représente hydrogène ; R² représente halogéno ou Het¹ ; R³ représente hydrogène ;
R⁴ représente Ar⁴- (alkyloxy en C₁₋₄)-, (alkyloxy en C₁₋₄)-ou alkyloxy en C₁₋₄ substitué par (alkyloxy en C₁₋₄)- (alkyloxy en C₁₋₄)- ;
R¹² représente hydrogène ou (alkyle en C₁₋₄)- ;
R¹³ représente Ar⁶-sulfonyle ou (alkyloxy en C₁₋₆)carbonyle facultativement substitué par phényle ;
R¹⁶ et R¹⁷, conjointement avec l'atome de carbone auquel ils sont liés, forment un cycloalkyle en C₃₋₆ ;
R²³ représente hydrogène ou alkyle en C₁₋₄ ;
Het¹ représente 2-bora-1,3-dioxolanyle ;
Ar⁴ représente phényle ; Ar⁵ représente phényle ;
Ar⁶ représente phényle facultativement substitué par nitro.

6. Composé selon la revendication 1 dans lequel
Z représente NH ;
Y représente - (alkyle en C₃₋₉)-, (alcényle en C₂₋₉)-,-(alkyle en C₁₋₅) -oxy- (alkyle en C₁₋₅)-, -(alkyle en C₁₋₅)-NR¹³- (alkyle en C₁₋₅)-, -(alkyle en C₁₋₅) -NR¹⁴-CO- (alkyle en C₁₋₅)-, -(alkyle en C₁₋₆) -NH-CO-, -NH-CO- (alkyle en C₁₋₆)-, -CO- (alkyle en C₁₋₇)-, -(alkyle en C₁₋₇)-CO-, (alkyle en C₁₋₆)-CO-(alkyle en C₁₋₆), -(alkyle en C₁₋₂)-NR²³-CO-CR¹⁶R¹⁷-NH-, -(alkyle en C₁₋₂)-CO-NH-CR¹⁸R¹⁹-CO-,-(alkyle en C₁₋₂)-CO-NR²⁰- (alkyle en C₁₋₃)-CO-, -(alkyle en C₁₋₂)-NR²¹-CH₂-CO-NH-(alkyle en C₁₋₃)-, -NR²²-CO-(alkyle en C₁₃)-NH-, -(alkyle en C₁₋₃)-NH-CO-Het²⁰-, (alkyle en C₁₋₂)-CO-Het²¹-CO-, ou -Het²²-CH₂-CO-NH- (alkyle en C₁₋₃)- ; X₁ représente O, -O-(alkyle en C₁₋₂)-, -O-N=CH-, NR¹¹ ou -NR¹¹-(alkyle en C₁₋₂)- ;
X² représente une liaison directe, alkyle en C₁₋₂, O, -O-(alkyle en C₁₋₂)-, CO, -CO-(alkyle en C₁₋₂)-, -O- N=CH-, NR¹² ou NR¹²-(alkyle en C₁₋₂)- ;
R¹ représente hydrogène, cyano, halogéno ou hydroxy ;
R² représente hydrogène, cyano, halogéno, hydroxy, hydroxycarbonyl-, (alkyloxy en C₁₋₄)carbonyl-, Het¹⁶-carbonyl-, (alkyle en C₁₋₄)-, (alcynyle en C₂₋₆)-, Ar⁵, Het¹ ou dihydroxyborane ;
R³ représente hydrogène, cyano, halogéno, hydroxy, formyle, (alcoxy en C₁₋₆)-, (alkyle en C₁₋₆)-, (alcoxy en C₁₋₆)- substitué par halogéno, ou R³ représente alkyle en C₁₋₄ substitué par un ou, si possible, deux substituants ou plus choisis parmi hydroxy ou halogéno ;
R⁴ représente Ar⁴- (alkyloxy en C₁₋₄)-, (alkyloxy en C₁₋₄)-ou R⁴ représente alkyloxy en C₁₋₄ substitué par un ou, si possible, deux substituants ou plus choisis parmi hydroxy-, halogéno, (alkyloxy en C₁₋₄)-, (alkyloxy en C₁₋₄)- (alkyloxy en C₁₋₄)-, NR³⁷R³⁸- carbonyloxy-, Het⁵-carbonyloxy-, NR⁷R⁸, NR⁹R¹⁰-carbonyl-, Het³-carbonyl-, Het¹³-oxy- ou Het²- ;
R⁷ représente hydrogène ou alkyle en C₁₋₄ ;
R⁸ représente cycloalkyle en C₃₋₆, Het⁶-carbonyl-, Het⁷-aminocarbonyl-, Het⁸, Het⁹-oxycarbonyl-, Het¹⁰-sulfonyl-, mono- ou di (alkyle en C₁₋₄)aminocarbonyl-, mono- ou di (alkyle en C₁₋₄) aminocarbonyle substitué par (alkyle en C₁₋₄)sulfonyl-, ou (alkyle en C₁₋₄)carbonyle facultativement substitué par un ou plusieurs substituants choisis parmi (alkyle en C₁₋₄)sulfonyle, hydroxy- et (alkyloxy en C₁₋₄)-, ou R⁸ représente alkyle en C₁₋₄ substitué par un ou plusieurs substituants choisis parmi (alkyle en C₁₋₄) sulfonyl-, NR²⁵R²⁶, aminocarbonyloxy-, aminocarbonyl-, (alkyloxy en C₁₋₄)-(alkyloxy en C₁₋₄)-, et Het¹¹;
R⁹ représente hydrogène ou (alkyle en C₁₋₄)- ;
R¹⁰ représente Het⁴ ou (alkyle en C₁₋₄)-substitué par (alkyle en C₁₋₄)sulfonyl-, ;
R¹¹ représente hydrogène, (alkyle en C₁₋₄)- ou (alkyle en C₁₋₄)-oxy-carbonyl- ;
R¹² représente hydrogène, (alkyle en C₁₋₄)-, (alkyloxy en C₁₋₄)carbonyl- ou (alkyloxy en C₁₋₆)carbonyl- substitué par phényle ;
R¹³ représente hydrogène, Het¹⁴-(alkyle en C₁₋₄), (alkyloxy en C₁₋₆)carbonyle facultativement substitué par phényle ou R¹³ représente Ar⁶-sulfonyle ou Het²⁴-(alkyle en C₁₋₄)carbonyle ;
R¹⁴ et R¹⁵ sont chacun indépendamment choisi parmi hydrogène, alkyle en C₁₋₄, Het¹⁵-(alkyle en C₁₋₄)- ou (alkyloxy en C₁₋₄)-(alkyle en C₁₋₄)- ;
R¹⁶ et R¹⁷ représentent chacun indépendamment hydrogène, alkyle en C₁₋₄ ou alkyle en C₁₋₄ substitué par hydroxy- ou phényle ; ou R¹⁶ et R¹⁷, conjointement avec l'atome de carbone auquel ils sont liés, forment un cycloalkyle en C₃₋₆ ;
R¹⁸ représente hydrogène ou alkyle en C₁₋₄ facultativement substitué par hydroxy ou phényle ;
R¹⁹ représente hydrogène ou alkyle en C₁₋₄ ;
R²⁰ représente hydrogène ou alkyle en C₁₋₄ ;
R²¹ représente hydrogène, alkyle en C₁₋₄, Het²³- (alkyle en C₁₋₄)carbonyl- ou
R²¹ représente mono-ou di (alkyle en C₁₋₄) amino- (alkyle en C₁₋₄)-carbonyl- facultativement substitué par hydroxy, pyrimidinyle, diméthylamine ou alkyloxy en C₁₋₄ ;
R²² représente hydrogène ou alkyle en C₁₋₄ facultativement substitué par hydroxy ou alkyloxy en C₁₋₄ ;
R²³ représente alkyle en C₁₋₄ facultativement substitué par hydroxy-, (alkyloxy en C₁₋₄)- ou Het²³ ; R²³ peut également représenter hydrogène lorsque R¹⁶ et R¹⁷, conjointement avec l'atome de carbone auquel ils sont liés, forment un cycloalkyle en C₃₋₆ ;
R²⁵ et R²⁶ représentent chacun indépendamment hydrogène, alkyle en C₁₋₄, (alkyle en C₁₋₄)sulfonyl-, aminocarbonyl-, mono- ou di (alkyle en C₁₋₄)aminocarbonyl-, (alkyle en C₁₋₄)carbonyl-, (alkyloxy en C₁₋₄)carbonyl- ou alkyle en C₁₋₄ substitué par un ou plusieurs substituants choisis parmi (alkyle en C₁₋₄)sulfonyl-, hydroxy- et (alkyloxy en C₁-₄)- ;
R²⁷ et R²⁸ représentent chacun indépendamment hydrogène, alkyle en C₁₋₄, (alkyle en C₁₋₄)sulfonyl-, aminocarbonyl-, mono- ou di (alkyle en C₁₋₄)aminocarbonyl-, (alkyle en C₁₋₄)carbonyl-, (alkyloxy en C₁₋₄)carbonyl- ou alkyle en C₁₋₄ substitué par un ou plusieurs substituants choisis parmi (alkyle en C₁₋₄)sulfonyl-, hydroxy- et (alkyloxy en C₁₋₄)- ;
R²⁹ et R³⁰ représentent chacun indépendamment hydrogène, aminosulfonyle, aminocarbonyle, mono- ou di(alkyle en C₁₋₄)aminocarbonyl-, mono- ou di (alkyle en C₁₋₄)aminosulfonyl-, ou
(alkyle en C₁₋₄)- facultativement substitué par un ou plusieurs substituants choisis parmi NR³¹R³², (alkyle en C₁₋₄)sulfonyle, aminocarbonyloxy-, hydroxy-, (alkyloxy en C₁₋₄)-, aminocarbonyl- et mono- ou di (alkyle en C₁₋₄)aminocarbonyl-, ou
(alkyloxy en C₁₋₄)carbonyle facultativement substitué par un ou plusieurs substituants choisis parmi hydroxy, (alkyloxy en C₁₋₄)- et (alkyle en C₁₋₄)sulfonyl-, ou (alkyle en C₁₋₄)carbonyle facultativement substitué par un ou plusieurs substituants choisis parmi hydroxy-, (alkyloxy en C₁₋₄)- et (alkyle en C₁₋₄)sulfonyl- ;
R³¹ et R³² représentent chacun indépendamment hydrogène, alkyle en C₁₋₄, (alkyle en C₁₋₄)sulfonyl-, aminocarbonyl-, mono- ou di (alkyle en C₁₋₄) aminocarbonyl-, (alkyle en C₁₋₄)carbonyl-, (alkyloxy en C₁₋₄)carbonyl- ou alkyle en C₁₋₄ substitué par un ou plusieurs substituants choisis parmi (alkyle en C₁₋₄)sulfonyl-, hydroxy- et (alkyloxy en C₁₋₄)- ;
R³³ représente hydrogène ou alkyle en C₁₋₄ ;
R³⁴ représente (alkyle en C₁₋₄)sulfonyl-, aminocarbonyl-, mono- ou di (alkyle en C₁₋₄) aminocarbonyl-, (alkyle en C₁₋₄)carbonyl-, (alkyloxy en C₁₋₄)carbonyl- ou alkyle en C₁₋₄ substitué par un ou plusieurs substituants choisis parmi (alkyle en C₁₋₄)sulfonyl-, hydroxy- et (alkyloxy en C₁₋₄)- ;
R³⁵ représente hydrogène ou alkyle en C₁₋₄ ;
R³⁶ représente (alkyle en C₁₋₄)sulfonyl-, aminocarbonyl-, mono- ou di (alkyle en C₁₋₄) aminocarbonyl-, (alkyle en C₁₋₄) carbonyl-, (alkyloxy en C₁₋₄) carbonyl- ou alkyle en C₁₋₄ substitué par un ou plusieurs substituants choisis parmi (alkyle en C₁₋₄)sulfonyl-, hydroxy- et (alkyloxy en C₁₋₄) - ;
R³⁷ et R³⁸ représentent chacun indépendamment hydrogène, alkyle en C₁₋₄, (alkyle en C₁₋₄)sulfonyl-, Het¹² ou alkyle en C₁₋₄ substitué par un ou plusieurs substituants choisis parmi (alkyle en C₁₋₄)sulfonyl-, hydroxy- et (alkyloxy en C₁₋₄)- ;
R³⁹ et R⁴⁰ représentent chacun indépendamment aminosulfonyle, aminocarbonyle, mono- ou di(alkyle en C₁₋₄)aminocarbonyl-, mono- ou di (alkyle en C₁₋₄)aminosulfonyl-, ou
(alkyle en C₁₋₄)- substitué par un ou plusieurs substituants choisis parmi NR³¹R³², (alkyle en C₁₋₄)sulfonyle, aminocarbonyloxy-, hydroxy-, (alkyloxy en C₁₋₄)-, aminocarbonyl- et mono- ou di (alkyle en C₁₋₄)aminocarbonyl-, ou (alkyloxy en C₁₋₄) carbonyle facultativement substitué par un ou plusieurs substituants choisis parmi hydroxy, (alkyloxy en C₁₋₄)-et (alkyle en C₁₋₄)sulfonyl-, ou (alkyle en C₁₋₄)carbonyle facultativement substitué par un ou plusieurs substituants choisis parmi hydroxy-, (alkyloxy en C₁₋₄)- et (alkyle en C₁₋₄)sulfonyl- ;
Het¹ représente thiazolyle ou 2-bora-1,3-dioxolanyle, ledit Het¹ étant facultativement substitué par un ou, si possible, deux, trois, quatre substituants ou plus choisis parmi amino, alkyle en C₁₋₄, hydroxy-(alkyle en C₁₋₄)-, phényle, phényl- (alkyle en C₁₋₄)-, (alkyle en C₁₋₄)-oxy-(alkyle en C₁₋₄)-, mono- ou di (alkyle en C₁₋₄)amino- ou amino-carbonyl- ;
Het² représente un hétérocycle choisi parmi tétrahydropyranyle, tétrahydrofuranyle, furanyle, 1,1-dioxothiomorpholinyle, pipérazininonyle, tétrahydro-1,1-dioxydo-2H-thiopyranyle, pipéridinonyle, azétidinyle ou 2-azétidinonyle, ledit Het² étant facultativement substitué par un ou, si possible, deux substituants ou plus choisis parmi hydroxy, amino, NR²⁹R³⁰, aminocarbonyle, mono- ou di (alkyle en C₁₋₄)aminocarbonyle, (alkyle en C₁₋₄)sulfonyle ou
(alkyle en C₁₋₄)- facultativement substitué par un ou plusieurs substituants choisis parmi NR²⁷R²⁸, (alkyle en C₁₋₄)sulfonyle, aminocarbonyloxy-, aminocarbonyl- et mono- ou di (alkyle en C₁₋₄)aminocarbonyl-, ou
(alkyloxy en C₁₋₄)- facultativement substitué par (alkyloxy en C₁₋₄)-, ou
(alkyloxy en C₁₋₄)carbonyle facultativement substitué par un ou plusieurs substituants choisis parmi hydroxy, (alkyloxy en C₁₋₄)- et (alkyle en C₁₋₄)sulfonyl-, ou (alkyle en C₁₋₄)carbonyle facultativement substitué par un ou plusieurs substituants choisis parmi hydroxy-, (alkyloxy en C₁₋₄)- et (alkyle en C₁₋₄)sulfonyl- ; ou
Het² représente un hétérocycle choisi parmi morpholinyle, pipérazinyle, pipéridinyle ou pyrrolidinyle, lesdits morpholinyle, pipérazinyle, pipéridinyle ou pyrrolidinyle étant facultativement substitués par un ou, si possible, deux substituants ou plus choisis parmi
(alkyle en C₁₋₄)- facultativement substitué par un ou plusieurs substituants choisis parmi NR²⁷R²⁸, (alkyle en C₁₋₄)sulfonyle, aminocarbonyloxy-, aminocarbonyl- et mono- ou di (alkyle en C₁₋₄)aminocarbonyl-, ou
(alkyloxy en C₁₋₄)- facultativement substitué par (alkyloxy en C₁₋₄)-, ou
(alkyloxy en C₁₋₄)carbonyle facultativement substitué par un ou plusieurs substituants choisis parmi hydroxy, (alkyloxy en C₁₋₄)- et (alkyle en C₁₋₄)sulfonyl-, ou (alkyle en C₁₋₄)carbonyle facultativement substitué par un ou plusieurs substituants choisis parmi hydroxy-, (alkyloxy en C₁₋₄)- et (alkyle en C₁₋₄)sulfonyl- ;
Het³ représente un hétérocycle choisi parmi tétrahydropyranyle, tétrahydrofuranyle, furanyle, 1,1-dioxothiomorpholinyle, pipérazininonyle, tétrahydro-1,1-dioxydo-2H-thiopyranyle, pipéridinonyle, azétidinyle ou 2-azétidinonyle, ledit Het³ étant facultativement substitué par un ou, si possible, deux substituants ou plus hydroxy-, amino, (alkyle en C₁₋₄)-. (cycloalkyle en C₃₋₆)-(alkyle en C₁₋₄)-, aminosulfonyl-, mono- ou di (alkyle en C₁₋₄)aminosulfonyl-, amino-(alkyle en C₁₋₄)-, mono- ou di (alkyle en C₁₋₄)amino-(alkyle en C₁₋₄), NR³⁵R³⁶, (alkyle en C₁₋₄)-sulfonyl- (alkyle en C₁₋₄)-ou (alkyloxy en C₁₋₄)- facultativement substitué par (alkyloxy en C₁₋₄)- ou hydroxy ; ou
Het³ représente un hétérocycle choisi parmi morpholinyle, pipérazinyle, pipéridinyle ou pyrrolidinyle, ledit Het³ étant facultativement substitué par un ou, si possible, deux substituants ou plus choisis parmi NR³⁵R³⁶, (alkyle en C₁₋₄)-sulfonyl-(alkyle en C₁₋₄)- ou (alkyloxy en C₁₋₄)- facultativement substitué par (alkyloxy en C₁₋₄)- ou hydroxy ;
Het⁴ représente un hétérocycle choisi parmi morpholinyle, pipérazinyle, pipéridinyle, furanyle, pyrazolyle, dioxolanyle, thiazolyle, oxazolyle, imidazolyle, isoxazolyle, oxadiazolyle, pyridinyle ou pyrrolidinyle, ledit Het⁴ étant substitué par un ou, si possible, deux substituants ou plus choisis parmi (alkyle en C₁₋₄)-sulfonyl- (alkyle en C₁₋₄)-, (alkyloxy en C₁₋₄)- facultativement substitué par (alkyloxy en C₁₋₄)- ou hydroxy ;
Het⁵ représente un hétérocycle choisi parmi furanyle, pipérazinyle, 1,1-dioxothiomorpholinyle, pipérazininonyle, pipéridinyle, tétrahydro-1,1-dioxydo-2H-thiopyranyle, pipéridinonyle, morpholinyle ou pyrrolidinyle, ledit Het⁵ étant facultativement substitué par hydroxy, amino, mono- ou di(alkyle en C₁₋₄)-amino-, alkyle en C₁₋₄,
Het⁶ et Het⁷ représentent chacun indépendamment un hétérocycle choisi parmi pipérazinyle, pipéridinyle ou pyrrolidinyle, lesdits hétérocycles étant facultativement substitués par un ou plusieurs substituants choisis parmi hydroxy-, amino-, hydroxy-(alkyle en C₁₋₄)-, (alkyloxy en C₁₋₄)-(alkyle en C₁₋₄)- et (alkyle en C₁₋₄)- ;
Het⁸ représente un hétérocycle choisi parmi tétrahydropyranyle, tétrahydrofuranyle, 1,1-dioxothiomorpholinyle, pipérazininonyle, tétrahydro-1,1-dioxydo-2H-thiopyranyle, pipéridinonyle, azétidinyle ou 2-azétidinonyle, ledit Het⁸ étant facultativement substitué par aminosulfonyle, aminocarbonyle, mono- ou di(alkyle en C₁₋₄)aminocarbonyl-, mono- ou di (alkyle en C₁₋₄)aminosulfonyl-, ou
(alkyle en C₁₋₄)- facultativement substitué par un ou plusieurs substituants choisis parmi amino, mono- ou di (alkyle en C₁₋₄) amino-, NR³³R³⁴, (alkyle en C₁₋₄)sulfonyle, aminocarbonyloxy-, hydroxy-, (alkyloxy en C₁₋₄)-, aminocarbonyl- et mono- ou di (alkyle en C₁₋₄) aminocarbonyl-, ou
(alkyloxy en C₁₋₄)carbonyle facultativement substitué par un ou plusieurs substituants choisis parmi hydroxy-, (alkyloxy en C₁₋₄)- et (alkyle en C₁₋₄)sulfonyl-, ou (alkyle en C₁₋₄)carbonyle facultativement substitué par un ou plusieurs substituants choisis parmi hydroxy-, (alkyloxy en C₁₋₄)- et (alkyle en C₁₋₄)sulfonyl- ; ou Het⁸ représente un hétérocycle choisi parmi furanyle, pipéridinyle ou pipérazinyle, ledit Het⁸ étant substitué par aminocarbonyle, mono- ou di(alkyle en C₁₋₄)aminocarbonyl-, mono- ou di (alkyle en C₁₋₄)aminosulfonyl-, ou
(alkyle en C₁₋₄)- substitué par un ou plusieurs substituants choisis parmi NR³³R³⁴, (alkyle en C₁₋₄)sulfonyle, aminocarbonyloxy-, hydroxy-, (alkyloxy en C₁₋₄)-, aminocarbonyl- et mono- ou di (alkyle en C₁₋₄) aminocarbonyl-, ou
(alkyloxy en C₁₋₄)carbonyle facultativement substitué par un ou plusieurs substituants choisis parmi hydroxy-, (alkyloxy en C₁₋₄)- et (alkyle en C₁₋₄)sulfonyl-, ou (alkyle en C₁₋₄)carbonyle facultativement substitué par un ou plusieurs substituants choisis parmi hydroxy-, (alkyloxy en C₁₋₄)- et (alkyle en C₁₋₄)sulfonyl- ;
Het⁹ et Het¹⁰ représentent chacun indépendamment un hétérocycle choisi parmi pipérazinyle, pipéridinyle ou pyrrolidinyle, lesdits hétérocycles étant facultativement substitués par un ou plusieurs substituants choisis parmi hydroxy-, amino, hydroxy-(alkyle en C₁₋₄)-, (alkyloxy en C₁₋₄)-C₁₋₄alkyl- et (alkyle en C₁₋₄) - ;
Het¹¹ représente 2-imidazolidinonyl- ou 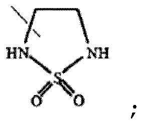 Het¹² représente un hétérocycle choisi parmi morpholinyle, pipérazinyle, pipéridinyle ou pyrrolidinyle, ledit Het¹² étant facultativement substitué par un ou, si possible, deux substituants ou plus choisis parmi hydroxy-, amino ou (alkyle en C₁₋₄)-
Het¹³ représente un hétérocycle choisi parmi furanyle, pipérazinyle, 1,1-dioxothiomorpholinyle, pipérazininonyle, pipéridinyle, tétrahydro-1,1-dioxydo-2H-thiopyranyle, pipéridinonyle, morpholinyle, pipérazinyle ou pyrrolidinyle
Het¹⁶ représente un hétérocycle choisi parmi pipéridinyle ou pyrrolidinyle ;
Het²⁰ représente pyrrolidinyle, 2-pyrrolidinonyle, pipéridinyle ou hydroxy-pyrrolidinyle, de préférence pyrrolidinyle ou hydroxy-pyrrolidinyle ;
Het²¹ représente pyrrolidinyle ou hydroxy-pyrrolidinyle ;
Het²² représente pyrrolidinyle, pipérazinyle ou pipéridinyle ;
Het²³ représente un hétérocycle choisi parmi morpholinyle, pyrrolidinyle, pipérazinyle ou pipéridinyle, ledit Het²³ étant facultativement substitué par un ou, si possible, deux substituants ou plus choisis parmi alkyle en C₁₋₄, cycloalkyle en C₃₋₆, hydroxy-(alkyle en C₁₋₄)-, (alkyloxy en C₁₋₄)-(alkyle en C₁₋₄) ou polyhydroxy-(alkyle en C₁₋₄)- ;
Ar⁴, Ar⁵ ou Ar⁶ représentent chacun indépendamment phényle facultativement substitué par nitro, cyano, (alkyle en C₁₋₄)sulfonyl-, (alkyle en C₁₋₄)sulfonylamino-, aminosulfonylamino-, hydroxy-(alkyle en C₁₋₄), aminosulfonyl-, hydroxy-, (alkyloxy en C₁₋₄)- ou alkyle en C₁₋₄, de préférence Ar⁴ ou Ar⁵ représentent chacun indépendamment phényle facultativement substitué par cyano.

7. Composé selon l'une quelconque des revendications 1 à 6 dans lequel le substituant X² est à la position 2', le substituant R¹ représente hydrogène ou halogéno et est à la position 4', le substituant R² représente halogéno et est à la position 5', le substituant R³ est à la position 2 et le substituant R⁴ à la position 7 de la structure de formule (I).

8. Composé selon l'une quelconque des revendications 1 à 4 ou 6 à 7 dans lequel R⁴ représente alkyloxy en C₁₋₄ substitué par hydroxy et un substituant choisi parmi NR⁷R⁸ ou He^{t2}-.

9. Inhibiteur de kinase de formule (I) tel que défini dans la revendication 1.

10. Composé selon l'une quelconque des revendications 1 à 8 pour utilisation en tant que médicament.

11. Utilisation d'un composé selon l'une quelconque des revendications 1 à 8 dans la fabrication d'un médicament pour traiter des troubles prolifératifs cellulaires tels que l'athérosclérose, la resténose et le cancer.

12. Composition pharmaceutique comprenant un véhicule pharmaceutiquement acceptable et, en tant que substance active, une quantité inhibitrice de kinase efficace d'un composé comme décrit dans l'une quelconque des revendications 1 à 8.
